# EUROPEAN PATENT SPECIFICATION

(11) **EP 3 150 579 B1**
(45) Date of publication and mention of the grant of the patent: **24.02.2021**
(21) Application number: 15798998.9
(22) Date of filing: 25.05.2015
(51) Int. Cl.: H01L 51/50, C07D 213/16, C07D 213/22, C07D 213/53, C07D 215/04, C07D 239/26, C07D 251/24, C07D 401/04, C09K 11/06

(54) **FLUORANTHENE DERIVATIVE, ELECTRONIC DEVICE CONTAINING SAME, LIGHT-EMITTING ELEMENT, AND PHOTOELECTRIC CONVERSION ELEMENT**
FLUORANTHENDERIVAT, ELEKTRONISCHE VORRICHTUNG DAMIT, LICHTEMITTIERENDES ELEMENT UND FOTOELEKTRISCHES UMWANDLUNGSELEMENT
DÉRIVÉ DE FLUORANTHÈNE, DISPOSITIF ÉLECTRONIQUE LE CONTENANT, ÉLÉMENT ÉLECTROLUMINESCENT, ET ÉLÉMENT DE CONVERSION PHOTOÉLECTRIQUE

(30) Priority: 28.05.2014 JP 2014109873
(43) Date of publication of application: 05.04.2017
(73) Proprietor: Toray Industries, Inc., Tokyo 103-8666 (JP)
(72) Inventor: ICHIHASHI, Yasunori, Otsu-shi Shiga 520-8558 (JP); TANAKA, Daisaku, Otsu-shi Shiga 520-8558 (JP)
(74) Representative: Hoefer & Partner Patentanwälte mbB
(86) International application number: PCT/JP2015/064904
(87) International publication number: WO 2015/182547

(56) References cited:
- WO-A1-2011/086935
- WO-A1-2011/086941
- WO-A1-2012/005009
- WO-A1-2012/046839
- WO-A1-2013/038944
- WO-A1-2013/187258
- WO-A1-2014/007287
- WO-A1-2014/081168
- WO-A1-2014/097711
- WO-A1-2015/008866
- WO-A1-2015/037675
- WO-A2-2009/148269
- JP-A- 2001 244 075
- JP-A- 2003 105 332
- JP-A- 2008 308 486
- JP-A- 2014 138 006
- JP-A- 2014 216 576
- KR-A- 20130 135 178
- KR-A- 20130 135 179
- KR-A- 20150 030 294
- US-A1- 2009 149 649
- US-A1- 2012 286 249
- WU, DIFENG ET AL.: 'Superaromatic terpyridines based on corannulene responsive to metal ions' DALTON TRANSACTIONS vol. 43, no. 4, 2014, pages 1753 - 1761, XP055240852

## Description

### TECHNICAL FIELD

The present invention relates to a light emitting device, a photoelectric conversion element, a lithium ion battery, a fuel cell, an electronic device such as transistor, and a material used therefor. The present invention can be used in the fields of display device, flat panel display, backlight, lighting, interior, label, signboard, electronic camera, light signal generator, etc.

### BACKGROUND ART

In recent years, studies are being aggressively made on an organic thin-film light emitting device that emits light when an electron injected from a cathode and a hole injected from an anode are recombined in an organic phosphor sandwiched between both electrodes. This light emitting device is characterized by being thin and capable of emitting high-luminance light at a low driving voltage and emitting multicolor light by selecting the phosphor material and is attracting attention.

As to these studies, since C. W. Tang, et al. of Kodak Co., Ltd. reported that an organic thin-film device emits light with high luminance, many discussions on practical utilization are ongoing, and an organic thin-film light emitting device is making steady progress toward practical utilization, for example, is employed in a main display, etc. of a mobile phone. However, many technical problems still remain to be solved and, among others, achieving both high efficiency and prolonged life of a device is one of the major problems.

The organic thin-film light emitting device must realize enhancement of luminance efficiency, reduction of driving voltage, and improvement of durability. Above all, it is a major problem to satisfy both luminance efficiency and durable life. For example, in order to enhance the luminance efficiency and the durable life, materials having a fluoranthene skeleton have been developed (see, for example, Patent Documents 1 to 3).
Patent Document 4 relates to heterocyclic compounds and an organic electronic device using the same. The heterocyclic compounds greatly improve efficiency and lifetime. Also, the heterocyclic compounds improve a driving voltage of the organic electronic device.

### BACKGROUND ART DOCUMENT

### PATENT DOCUMENT

Patent Document 1: WO 2013/182046
Patent Document 2: WO 2012/017680
Patent Document 3: WO 2012/030145
Patent Document 4: KR 2013-0135178 A

### SUMMARY OF THE INVENTION

### PROBLEMS THAT THE INVENTION IS TO SOLVE

However, in conventional techniques, it is difficult to sufficiently reduce the driving voltage of a device, and even if the driving voltage could be reduced, the luminance efficiency and durable life of the device are insufficient. In this way, a technique capable of satisfying all of high luminance efficiency, low driving voltage and durable life has not yet been found.

An object of the present invention is to solve those problems of the conventional techniques and provide an organic thin-film light emitting device in which all of luminance efficiency, driving voltage and durable life are improved.

### MEANS FOR SOLVING THE PROBLEMS

The present invention, which is defined in the appended claims, relates to a fluoranthene derivative represented by the following formula (3): 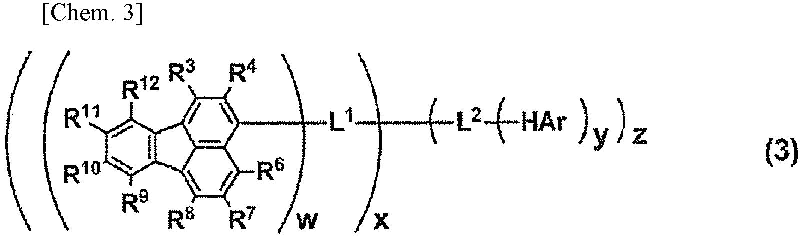 wherein R³, R⁴ and R⁶ to R¹² may be the same as or different from one another and each is selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, an amino group, a carbonyl group, a carboxyl group, an oxycarbonyl group and a carbamoyl group; L¹ is a substituted or unsubstituted phenylene group and wherein when L¹ is substituted, the substituent is an aryl group; L² is a single bond, a substituted or unsubstituted arylene group, or a substituted or unsubstituted heteroarylene group; HAr is a substituted or unsubstituted aromatic heterocyclic group containing an electron-accepting nitrogen selected from the group consisting of the following groups: 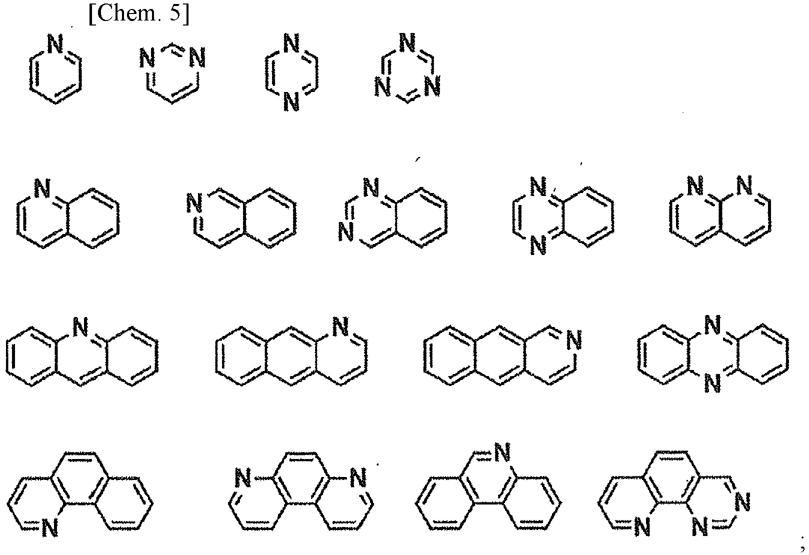 when each of the groups above is substituted, the substituent is selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, an amino group, a carbonyl group, a carboxyl group, an oxycarbonyl group, a carbamoyl group and -P(=O)R¹R² in which R¹ and R² are an aryl group or a heteroaryl group, and R¹ and R² may be fused to form a ring; w, x, y and z are an integer of 1 to 3; provided that the arylene group is neither an anthracene-containing group nor a pyrene-containing group and that each of L² and HAr is not a group containing electron-donating nitrogen; when w=x=1, L² is a substituted or unsubstituted arylene group or a substituted or unsubstituted heteroarylene group; when w=1 and x=2, HAr is not pyrimidine; when w=2 and x=1, the substituent substituting on L1 is not phenanthrene; and when w, x, y and z are 2 or 3, a plurality of fluoranthene groups, L¹, L² or HAr may be the same as or different from one another, and it is not allowed that both x and z are 2 or more.

### ADVANTAGE OF THE INVENTION

According to the present invention, an organic thin-film light emitting device satisfying all of luminance efficiency, driving voltage and durable life can be provided.

### MODE FOR CARRYING OUT THE INVENTION

A fluoranthene derivative not falling under the scope of the present invention is represented by formula (1) and is described in detail.

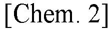

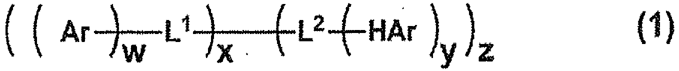

In the formula, Ar represents a group containing a fluoranthene skeleton; L¹ is a substituted or unsubstituted arylene group; L² is a single bond, a substituted or unsubstituted arylene group, or a substituted or unsubstituted heteroarylene group; HAr is a substituted or unsubstituted aromatic heterocyclic group containing an electron-accepting nitrogen; when each of the groups above is substituted, the substituent is selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, an amino group, a carbonyl group, a carboxyl group, an oxycarbonyl group, a carbamoyl group and -P(=O)R¹R² in which R¹ and R² are an aryl group or a heteroaryl group, and R¹ and R² may be fused to form a ring; w, x, y and z are each independently an integer of 1 to 3; provided that the arylene group is neither an anthracene-containing group nor a pyrene-containing group and that each of L² and HAr is not a group containing electron-donating nitrogen; when w=x=1, L² is a substituted or unsubstituted arylene group or a substituted or unsubstituted heteroarylene group; when w=1 and x=2, HAr is not pyrimidine; when w=2 and x=1, the substituent substituting on L¹ is not phenanthrene; when w, x, y or z is 2 or 3, a plurality of Ar, L¹, L² or HAr may be the same as or different from one another.

In all of the groups above, hydrogen may be heavy hydrogen. In the following description, for example, the number of carbons in the substituted or unsubstituted aryl group having a carbon number of 6 to 40 is from 6 to 40 including the number of carbons contained in the substituent substituted on the arylene group, and the same applies to other substituents in which the carbon number is specified.

As the substituent in the case of "substituted or unsubstituted", the above-described alkyl group, cycloalkyl group, heterocyclic group, alkenyl group, cycloalkenyl group, alkynyl group, alkoxy group, alkylthio group, aryl ether group, aryl thioether group, aryl group, heteroaryl group, halogen, cyano group, amino group, carbonyl group, carboxyl group, oxycarbonyl group and carbamoyl group are preferred, and preferable substituents specifically recited in the description of each substituent are more preferred. In addition, such a substituent may be further substituted with the above-described substituent.

The "unsubstituted" in the case of "substituted or unsubstituted" means that a hydrogen atom is substituted on the group.

The same applies to "substituted or unsubstituted" in the compound described below or a partial structure thereof.

The alkyl group indicates, for example, a saturated aliphatic hydrocarbon group, such as methyl group, ethyl group, n-propyl group, isopropyl group, n-butyl group, sec-butyl group and tert-butyl group, and may or may not have a substituent. When substituted, the additional substituent is not particularly limited and includes, for example, an alkyl group, an aryl group, and a heteroaryl group, and in regard to this point, the same holds true also for the following description. The carbon number of the alkyl group is not particularly limited but is preferably from 1 to 20, more preferably from 1 to 8, from the viewpoint of easy availability and cost.

The cycloalkyl group indicates, for example, a saturated alicyclic hydrocarbon group, such as cyclopropyl group, cyclohexyl group, norbornyl group and adamantyl group, and may or may not have a substituent. The carbon number of the alkyl group moiety is not particularly limited but is preferably from 3 to 20.

The heterocyclic group indicates, for example, an aliphatic ring having, in the ring, an atom other than carbon, such as pyran ring, piperidine ring and cyclic amide, and may or may not have a substituent. The carbon number of the heterocyclic group is not particularly limited but is preferably from 2 to 20.

The alkenyl group indicates, for example, a double bond-containing unsaturated aliphatic hydrocarbon group, such as vinyl group, allyl group and butadienyl group, and may or may not have a substituent. The carbon number of the alkenyl group is not particularly limited but is preferably from 2 to 20.

The cycloalkenyl group indicates, for example, a double bond-containing unsaturated alicyclic hydrocarbon group, such as cyclopentenyl group, cyclopentadienyl group and cyclohexenyl group, and may or may not have a substituent.

The alkynyl group indicates, for example, a triple bond-containing unsaturated aliphatic hydrocarbon group, such as ethynyl group, and may or may not have a substituent. The carbon number of the alkynyl group is not particularly limited but is preferably from 2 to 20.

The alkoxy group indicates, for example, a functional group bonded with an aliphatic hydrocarbon group via an ether bond, such as methoxy group, ethoxy group and propoxy group, and this aliphatic hydrocarbon group may or may not have a substituent. The carbon number of the alkoxy group is not particularly limited but is preferably from 1 to 20.

The alkylthio group is a group in which an oxygen atom of an ether bond in an alkoxy group is replaced by a sulfur atom. The hydrocarbon group of the alkylthio group may or may not have a substituent. The carbon number of the alkylthio group is not particularly limited but is preferably from 1 to 20.

The aryl ether group indicates, for example, a functional group to which an aromatic hydrocarbon group is bonded via an ether bond, such as phenoxy group, and the aromatic hydrocarbon group may or may not have a substituent. The carbon number of the aryl ether group is not particularly limited but is preferably from 6 to 40.

The aryl thioether group is a group in which an oxygen atom of an ether bond in an aryl ether group is replaced by a sulfur atom. The aromatic hydrocarbon group in the aryl ether group may or may not have a substituent. The carbon number of the aryl ether group is not particularly limited but is preferably from 6 to 40.

The aryl group indicates, for example, an aromatic hydrocarbon group, such as phenyl group, naphthyl group, biphenyl group, terphenyl group, fluorenyl group, phenanthryl group, anthracenyl group, pyrenyl group and fluoranthenyl group. The aryl group may or may not have a substituent. The carbon number of the aryl group is not particularly limited but is preferably from 6 to 40, more preferably from 6 to 20. A phenyl group, a 1-naphthyl group, and a 2-naphthyl group are more preferred.

The heteroaryl group indicates a cyclic aromatic group having, in the ring thereof, one atom or a plurality of atoms other than carbon, such as furanyl group, thiophenyl group, pyridyl group, quinolinyl group, isoquinolinyl group, pyrazinyl group, pyrimidyl group, naphthyridyl group, benzofuranyl group, benzothiophenyl group, indolyl group, dibenzofuranyl group, dibenzothiophenyl group, carbazolyl group, benzocarbazolyl group, carbolinyl group, indolocarbazolyl group, benzofurocarbazolyl group, benzothienocarbazolyl group, dihydroindenocarbazolyl group, benzoquinolinyl group, acridinyl group, dibenzoacridinyl group, benzimidazolyl group, imidazopyridyl group, benzoxazolyl group, benzothiazolyl group and phenanthrolinyl group, and this may be unsubstituted or substituted. The carbon number of the heteroaryl group is not particularly limited but is preferably from 2 to 30. A pyridyl group, a quinolyl group, a carbazolyl group, a dibenzofuranyl group, and a dibenzothiophenyl group are more preferred.

The amino group is a substituted or unsubstituted amino group. When substituted, the substituent includes, for example, an aryl group, a heteroaryl group, a linear alkyl group, and a branched alkyl group. More specifically, examples of the substituent include a phenyl group, a biphenyl group, a naphthyl group, a pyridyl group, and a methyl group, and such a substituent may be further substituted. The carbon number is not particularly limited but is preferably from 6 to 40.

The halogen indicates an atom selected from fluorine, chlorine, bromine, and iodine.

The carbonyl group, carboxyl group, oxycarbonyl group, carbamoyl group and phosphine oxide group may or may not have a substituent. Here, the substituent includes, for example, an alkyl group, a cycloalkyl group, an aryl group, and a heteroaryl group, and such a substituent may be further substituted.

The arylene group indicates a divalent or higher valent group derived from an aromatic hydrocarbon group such as benzene, naphthalene, biphenyl, fluorene and phenanthrene, and may or may not have a substituent. A divalent or trivalent arylene group is preferred. The arylene group specifically includes a phenylene group, a biphenylene group, and a naphthylene group.

The heteroarylene group indicates a divalent or higher valent group derived from an aromatic group having, in the ring thereof, one atom or a plurality of atoms other than carbon, such as pyridine, quinoline, pyrimidine, pyrazine, triazine, quinoxaline, quinazoline, dibenzofuran and dibenzothiophene, and may or may not have a substituent. A divalent or trivalent heteroarylene group is preferred. The carbon number of the heteroarylene group is not particularly limited but is preferably from 2 to 30. The heteroarylene group specifically includes a pyridylene group, a pyrimidinylene group, a triazinylene group, and a dibenzofuranylene group. More specifically, these are 2,6-pyridylene group, 2,5-pyridylene group, 2,4-pyridylene group, 3,5-pyridylene group, 3,6-pyridylene group, 2,4,6-pyridylene group, 2,4-pyrimidinylene group, 2,5-pyrimidinylene group, 4,6-pyrimidinylene group, 2,4,6-pyrimidinylene group, 2,4,6-triazinylene group, 4,6-dibenzofuranylene group, 2,6-dibenzofuranylene group, 2,8-dibenzofuranylene group, and 3,7-dibenzofuranylene group. A pyridylene group, a pyrimidinylene group, a triazinylene group, etc. are preferred. Among these, a pyridylene group is preferred in view of carrier mobility.

The electron-accepting nitrogen indicates a nitrogen atom forming a multiple bond with an adjacent atom. The aromatic heterocyclic ring containing an electron-accepting nitrogen includes, for example, a pyridine ring, a pyridazine ring, a pyrimidine ring, a pyrazine ring, a triazine ring, an oxadiazole ring, a thiazole ring, a quinoline ring, an isoquinoline ring, a quinazoline ring, a quinoxaline ring, a benzoquinoline ring, a phenanthroline ring, an acridine ring, a benzothiazole ring, and a benzoxazole ring. Furthermore, the aromatic heterocyclic ring containing an electron-accepting nitrogen may have a substituent. A pyridine ring, a pyrimidine ring, a triazine ring, a quinoline ring, an isoquinoline ring, a quinazoline ring, a quinoxaline ring, and a phenanthroline ring are preferred; a pyridine ring, a quinoline ring, and a phenanthroline ring are more preferred; and a pyridine ring and a quinoline ring are still more preferred.

In the fluoranthene derivative of the present invention, w, x, y and z are an integer of 1 to 3, whereby the crystallinity is reduced or the glass transition temperature is raised and in turn, the stability of the film is enhanced.

The fluoranthene derivative of the present invention has a fluoranthene skeleton. The fluoranthene skeleton has a five-membered ring structure of 5π electron system. When one electron enters (when reduced), the five-membered ring structure of 5π electron system turns to a 6π electron system, and aromatic stabilization occurs (Huckel's rule). Accordingly, the five-membered ring structure of 5π electron system shows high electron affinity, and the fluoranthene skeleton of the present invention also has high electron affinity. Anthracene and pyrene, which are a widely recognized aromatic fused-ring skeleton, have no five-membered ring structure of 5π electron system and therefore, do not bring about an increase in the electron affinity attributable to aromatic stabilization resulting from reduction, and the above-described phenomenon is a property specific to a skeleton having a five-membered ring structure of 5π electron system.

For this reason, when the fluoranthene derivative of the present invention is used for a light emitting device, for example, when used in an electron transporting layer, a good performance of electron injection from the electrode is exhibited, and the driving voltage of the light emitting device can be reduced, as a result, the luminance efficiency of the light emitting device can be increased. This also contributes to the extension of life.

The fluoranthene skeleton allows molecules to well overlap with one another because of its high flatness and in turn, has high charge transporting property. Accordingly, when the fluoranthene derivative of the present invention is used in any one layer constituting a light emitting device, an electron generated from the cathode and a hole generated from the anode can be efficiently transported, so that the driving voltage of the device can be reduced. As a result, the luminance efficiency of the light emitting device can be increased. This also contributes to the extension of life.

The fluoranthene skeleton has high stability to a charge and makes it possible to smoothly repeatedly perform reduction by an electron or oxidation by a hole. When the fluoranthene derivative of the present invention is used for a light emitting device, the life thereof can be enhanced.

The group containing a fluoranthene skeleton is a group having a fluoranthene skeleton in a molecular structure and may or may not have a substituent. Adjacent substituents may form a ring, and the size of the ring formed by adjacent substituents is not particularly limited, but from the viewpoint of stability of the molecular structure, a five-membered ring or a six-membered ring is preferred. The ring formed may be an aliphatic ring or an aromatic ring. The ring formed by adjacent substituents may further have a substituent or may be further fused to a ring. The ring formed may contain a heteroatom other than carbon. The heteroatom other than carbon includes a nitrogen atom, etc. Especially, when the ring is composed of only carbon and hydrogen, advantageously, the electrochemical stability is increased, which contributes to improvement of durability of the device. The carbon number of the group containing a fluoranthene skeleton is not particularly limited but is preferably from 16 to 40. Specifically, for example, a fluoranthenyl group, a benzofluoranthenyl group, a benzoaceanthrylenyl group, a benzoacephenanthrenyl group, an indenofluoranthenyl group, and an acenaphthofluoranthenyl group may be mentioned.

In L²-(HAr)_{y} of the fluoranthene derivative of the present invention, HAr is an aromatic heterocyclic group containing an electron-accepting nitrogen. In the group represented by HAr, the aromatic heterocyclic group containing an electron-accepting nitrogen may be bonded directly to L² or may be substituted through a linking group. Specifically, HAr may be a pyridylphenyl group, and a benzene ring substituted with a pyridyl group may be coupled to L². Here, it is preferable for HAr not to contain a phenanthroline skeleton.

Since the nitrogen atom has high electronegativity, a multiple bond between the nitrogen atom and an adjacent atom has an electron-accepting property and therefore, L²-(HAr)_{y} having an electron-accepting nitrogen has high electron affinity. Accordingly, when the fluoranthene derivative represented by formula (1) of the present invention is used in an emissive layer or an electron transporting layer of a light emitting device, high electron mobility is exhibited, so that the driving voltage can be reduced. As a result, the luminance efficiency of the light emitting device can be increased. This also contributes to the extension of life.

In addition, the electron-accepting nitrogen has an unshared electron pair on the nitrogen atom and in turn, exhibits a strong coordinating property to a metal atom and therefore, L²-(HAr)_{y} having an electron-accepting nitrogen has a strong coordinating property to a metal. Accordingly, the fluoranthene derivative represented by formula (1) of the present invention, when used in an electron transporting layer of a light emitting device, is likely to be coordinated to a metal that is a cathode, and an interaction with the cathode is intensified to encourage electron injection property from the cathode, so that the driving voltage of the light emitting device can be reduced. In the case where the fluoranthene derivative represented by formula (1) of the present invention is used in an electron extraction layer of a photoelectric conversion element, electron extraction into the cathode is promoted, so that the conversion efficiency or on-off ratio of the photoelectric conversion element can be enhanced.

Furthermore, the fluoranthene derivative represented by formula (1) necessarily has L¹, whereby sublimability, evaporation stability, reduction in crystallinity, and film stability owing to a high glass transition temperature are enhanced. When the thin-film stability is enhanced, deterioration of a film is suppressed even if a light emitting device is driven for a long time, and in turn, the durability is improved.

Moreover, since L¹ is a substituted or unsubstituted arylene group, the conjugation is efficiently extended, and the charge transporting property is enhanced. Accordingly, when the fluoranthene derivative represented by formula (1) of the present invention is used in an emissive layer or an electron transporting layer of a light emitting device, high electron mobility is exhibited, so that the driving voltage can be reduced. As a result, luminance efficiency can be increased. Here, in the case where L¹ has a substituent other than L²-(HAr)_{y}, it is preferable for the substituent not to contain a triazine skeleton.

As understood from the above, the fluoranthene derivative has a fluoranthene skeleton, L¹ and L²-(HAr)_{y} in the molecular thereof and thereby has all of high electron injection/transporting properties, electrochemical stability, good sublimability, good evaporation stability, good film quality and a high glass transition temperature. Thanks to these properties, when the fluoranthene derivative of the present invention is used in any one layer constituting a light emitting device, an organic thin-film light emitting device satisfying all of high luminance efficiency, low driving voltage and durable life can be provided.

The arylene group is not an anthracene-containing group or a pyrene-containing group. The anthracene-containing group is a group containing an anthracene skeleton and is specifically a substituted or unsubstituted anthracene or a substituted or unsubstituted fused-ring anthracene. The pyrene-containing group is a group where anthracene in the anthracene-containing group is replaced by pyrene. The anthracene-containing group or pyrene-containing group has a long absorption wavelength and if L¹ or L² is an anthracene-containing group or a pyrene-containing group, the absorption wavelength becomes longer, and the compound intensely absorbs visible light. If this compound is used, for example, for a light emitting device, since the compound itself intensely absorbs light emitted by the emissive layer, luminance efficiency cannot be increased. For this reason, it is preferred that the arylene group is not an anthracene-containing group or a pyrene-containing group. The carbon number of the arylene group is not particularly limited but is preferably from 6 to 40, more preferably from 6 to 18. Specifically, as the arylene group, a phenylene group, a biphenylylene group, a naphthylene group, etc. are preferred. More specifically, examples of the arylene group include 1,4-phenylene group, a 1,3-phenylene group, a 1,2-phenylene group, a 4,4'-biphenylylene group, a 4,3'-biphenylylene group, a 3,3'-biphenylylene group, a 1,4-naphthylene group, a 1,5-naphthylene group, a 2,5-naphthylene group, a 2,6-naphthylene group, a 2,7-naphthylene group, and 1,3,5-phenylene group. A 1,4-phenylene group, a 1,3-phenylene group and 1,3,5-phenyl group are more preferred.

L² and HAr are not a group containing an electron-donating nitrogen. Here, the electron-donating nitrogen represents a nitrogen atom in which all bonds between respective adjacent atoms are a single bond. For example, a carbazolyl group or a benzimidazolyl group comes under the group containing an electron-donating nitrogen. The electron-donating nitrogen has low stability to an electron and therefore, in the case of not containing an electron-donating nitrogen in HAr, when the compound is used in an electron transporting material layer of a light emitting device, the life thereof can be enhanced.

When w=x=1, L² is a substituted or unsubstituted arylene group or a substituted or unsubstituted heteroarylene group. In the case where w=x=1, i.e., in the case of containing only one fluoranthene skeleton-containing group, if L² is a single bond, the extension of conjugation in the entire molecule is small, and electron mobility is reduced. If this compound is used in an electron transporting layer of a light emitting device, the driving voltage is increased. Accordingly, in the case of containing one fluoranthene skeleton-containing group, L² must be a substituted or unsubstituted arylene group or a substituted or unsubstituted heteroaryl group, whereby the extension of conjugation in the entire molecule becomes large and the electron mobility can be enhanced. When this compound is used in an electron transporting layer of a light emitting device, the driving voltage can be reduced.

When w=1 and x=2, HAr is not pyrimidine. In the case where w=1 and x=2, in view of film stability and evaporation stability, HAr is preferably a pyridyl group or a triazyl group, more preferably a pyridyl group.

When w=2 and x=1, the substituent substituting on L¹ is not phenanthrene. In the case where w=2 and x=1, if phenanthrene is substituted on L¹, the substituent around L¹ is sterically bulky to increase the intermolecular distance and when the compound is used for a light emitting device, the voltage of the light emitting device rises. Accordingly, the substituent substituted on L¹ is preferably not a sterically bulky substituent and, specifically, is preferably hydrogen, a phenyl group, a naphthyl group, or an alkyl group.

When w, x, y or z is 2 or 3, a plurality of Ar, L¹, L² or HAr may be the same as or different from one another. It is not allowed that both x and z are 2 or more.

Ar is, in one embodiment, preferably represented by the following formula (2). When Ar is represented by formula (2), the conjugation system is appropriately extended, whereby the compound is electrochemically stabilized and the charge transporting property is further enhanced.

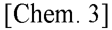

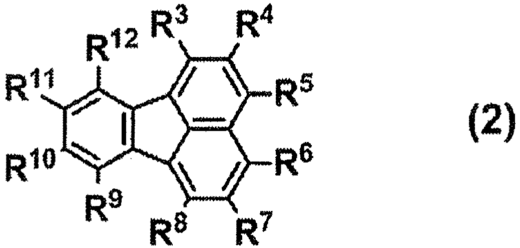

In the formula, R³ to R¹² may be the same as or different from one another and each is selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a carbonyl group, a carboxyl group, an oxycarbonyl group and a carbamoyl group. Out of R³ to R¹², adjacent substituents may form a ring. Here, Ar is linked to L¹ at any one position of R³ to R¹². Ar is preferably linked to L¹ at any one position selected from R³ to R⁹ and R¹².

In this embodiment, the fluoranthene derivative represented by formula (1) is preferably represented by the following formula (3). The fluoranthene derivative represented by formula (3) has a group represented by L¹-(L²-(HAr)_{y})_{z} at the 3-position of a fluoranthene skeleton. In the fluoranthene derivative, when an aromatic substituent is substituted on the 3-position, the electron state of the fluoranthene skeleton is greatly changed and since the conjugation is efficiently extended, the charge transporting property is enhanced. As a result, a light emitting device can be driven at a lower voltage, and the luminance efficiency can be more increased. Furthermore, extension of the conjugation leads to higher stability to a charge and in turn, when the fluoranthene derivative represented by formula (3) is used for a light emitting device, the life thereof can be further enhanced.

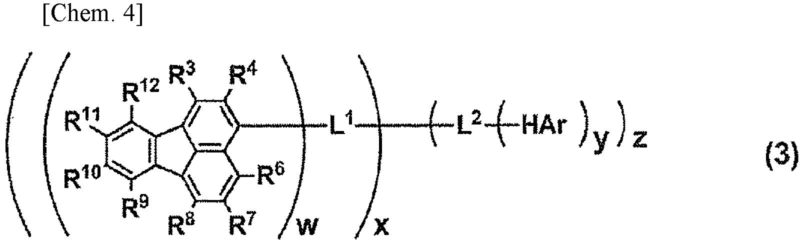

R³, R⁴ and R⁶ to R¹² in formula (3) are the same as those in formula (2). L¹, L², HAr, w, x, y and z are the same as those in formula (1).

R³ to R¹² in formulae (2) and (3) are preferably selected, among those described above, from hydrogen, an alkyl group, a cycloalkyl group, an aryl group, a heteroaryl group, and a halogen. When R³ to R¹² are hydrogen, an alkyl group, a cycloalkyl group, an aryl group, a heteroaryl group, or a halogen, the glass transition temperature rises, and the thin-film stability is enhanced. When the thin-film stability is enhanced, deterioration of a film is suppressed even if a light emitting device is driven for a long time, and in turn, the durability is improved. In addition, because of a substituent hard to decompose even at a high temperature, the heat resistance is enhanced. When heat resistance is enhanced, decomposition of a material can be suppressed at the time of manufacture of the device and therefore, the durability is improved. Furthermore, when the substituents are an aryl group or a heteroaryl group, the conjugation is extended, as a result, the compound is electrochemically stabilized and the charge transporting property is enhanced.

R⁹ and R¹² in formulae (2) and (3) are preferably hydrogen. When R⁹ and R¹² are hydrogen, the intermolecular distance in a film is decreased, and charge transfer can be efficiently performed, making it possible to drive a light emitting device at a low voltage and enhance the luminance efficiency. In addition, when the substituents are hydrogen, increase in the molecular weight can be suppressed, as a result, the sublimation temperature is reduced, and the heat resistance is more enhanced.

R⁹ and R¹² in formulae (2) and (3) are preferably a substituted or unsubstituted aryl group. When R⁹ and R¹² are a substituted or unsubstituted aryl group, overlap of π conjugate planes between molecules can be appropriately avoided. In addition, when these substituents are an aryl group, heat resistance is more improved. As a result, it becomes possible to enhance the sublimability, enhance the evaporation stability, reduce the crystallinity, and enhance the thin-film stability owing to a high glass transition temperature, without impairing high charge transporting property of the fluoranthene derivative.

R⁹ and R¹² in formulae (2) and (3) are more preferably a substituted or unsubstituted phenyl group. When R⁹ and R¹² are each a substituted or unsubstituted phenyl group, overlap of π conjugate planes between molecules can be appropriately avoided. In addition, since an appropriate molecular weight is obtained, the sublimability and evaporation stability are more enhanced.

What is common in all embodiments of the fluoranthene derivative represented by formula (1) is that HAr is preferably a group containing a structure represented by any of the following formulae (4) to (7). When HAr is a group containing a structure represented by any of the following formulae (4) to (7), high electron mobility and high electron-accepting property can be exerted to more reduce the driving voltage of a light emitting device. As a result, the luminance efficiency of the light emitting device can be more increased. This also contributes to further extension of the life of the light emitting device.

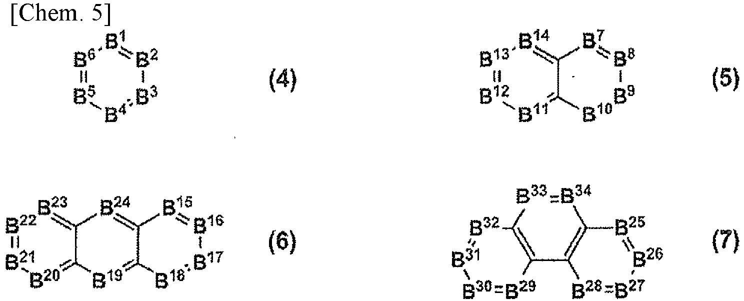

B¹ to B³⁴ represent CH, a substituted carbon atom or a nitrogen atom. In the case where B¹ to B³⁴ are substituted, the substituent is the same as those in formula (1). However, in formula (7), it is preferable to exclude a case where B²⁵ to B²⁷ are CH or a substituted carbon atom and B²⁸ and B²⁹ are a nitrogen atom.

According to the present invention, in the fluoranthene derivative represented by formula (1) HAr is selected from the group consisting of the following groups. When HAr is a structure selected from the group consisting of the following groups, high carrier mobility and high electron-accepting property are exerted. As a result, low voltage driving of a light emitting device becomes possible, and the luminance efficiency can be enhanced. In addition, sublimability, evaporation stability, reduction in crystallinity, and film stability owing to a high glass transition temperature are enhanced.

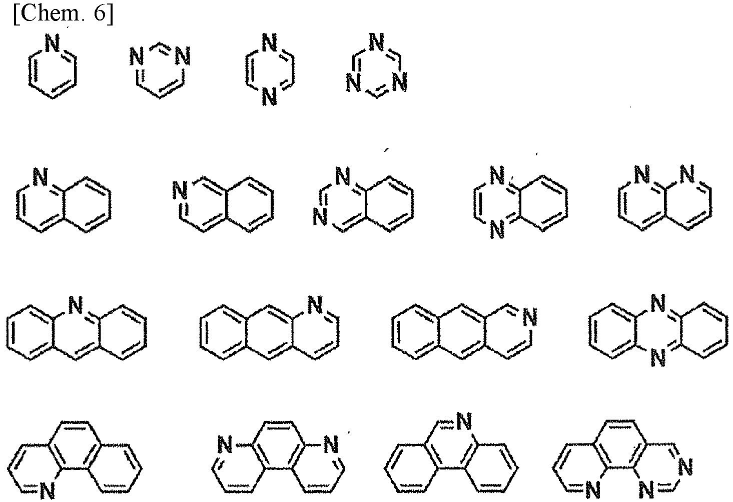

Among these, a pyridyl group, a pyrimidyl group, a triazyl group, a quinolinyl group, an isoquinolinyl group, a quinazolinylyl group, and a quinoxalinyl group are preferred. Furthermore, a pyridyl group, a pyrimidyl group and a triazyl group are preferred in view of carrier mobility, and a pyridyl group is more preferred in view of evaporation stability.

In formula (1), it is preferred that w+x=3. More specifically, two fluoranthene skeletons are preferably contained in formula (1). By containing two fluoranthene skeletons having high electron affinity, the fluoranthene derivative represented by formula (1) exerts higher carrier mobility and higher electron-accepting property. As a result, lower voltage driving of a light emitting device becomes possible, and the luminance efficiency can be more increased.

In formula (1), it is preferred that w=2 and x=1. More specifically, two fluoranthene skeleton-containing groups are preferably bonded to a substituted or unsubstituted arylene group which is L¹ in formula (1). By containing two fluoranthene skeletons with high electron affinity through an arylene group, the conjugation in the fluoranthene derivative represented by formula (1) is efficiently extended, and higher carrier mobility and higher electron-accepting property are exerted. As a result, lower voltage driving of a light emitting device becomes possible, and the luminance efficiency can be more increased. In addition, enhancement of sublimability, enhancement of evaporation stability, reduction in crystallinity, and enhancement of film stability owing to a high glass transition temperature are achieved.

In formula (1), it is also preferred that w=x=1. More specifically, it is also preferable to contain one fluoranthene skeleton in formula (1). Depending on a light emitting device, when excess electron injection into the emissive layer occurs, it is likely that the carrier balance between an electron and a hole is lost and electron attack on the hole transporting layer proceeds, resulting in degradation of the durability of the light emitting device. By containing one fluoranthene skeleton having high electron affinity, the conjugation system is appropriately extended, and moderate carrier mobility and electron-accepting property are exerted. As a result, a proper carrier balance can be produced in the light emitting device, and the durability can be more improved.

In formula (1), it is preferred that y=z=1. More specifically, one HAr is preferably contained in formula (1). By containing one HAr having high electron affinity, the conjugation system in the fluoranthene derivative represented by formula (1) is appropriately extended, and moderate carrier mobility and electron-accepting property are exerted. As a result, a proper carrier balance can be produced in the light emitting device, and the durability can be more improved.

In formula (1), it is also preferred that y+z=3. More specifically, it is also preferable to contain two HAr in formula (1). By containing two HAr having high electron affinity, the fluoranthene derivative represented by formula (1) exerts high carrier mobility and high electron-accepting property. As a result, lower voltage driving of a light emitting device becomes possible, and the luminance efficiency can be more increased. In addition, reduction in crystallinity and film stability owing to a high glass transition temperature are enhanced, and the durability of a light emitting device can be more improved.

In formula (1), it is preferred that L² is a substituted or unsubstituted heteroarylene group. At this time, the fluoranthene derivative represented by formula (1) exerts high carrier mobility and high electron-accepting property.

In formula (1), it is preferred that w+x+y+z is from 4 to 8. At this time, the fluoranthene derivative represented by formula (1) exerts high carrier mobility and high electron-accepting property. As a result, lower voltage driving of a light emitting device becomes possible, and the luminance efficiency can be more increased. In addition, reduction in crystallinity and film stability owing to a high glass transition temperature are enhanced, and the durability of the light emitting device can be more improved. More preferably, w+x+y+z is an integer of 5 to 7. When w+x+y+z is from 5 to 7, increase in the molecular weight can be suppressed while maintaining high carrier mobility and high electron-accepting property, as a result, the sublimation temperature is reduced, and the heat resistance is more enhanced.

In formula (1), it is preferred that when L¹ is substituted, the substituent does not contain a triazine skeleton. In addition, the substituents of L¹, L², A¹ and A² are preferably an aryl group. At this time, enhancement of sublimability, enhancement of evaporation stability, reduction in crystallinity, and enhancement of film stability owing to a high glass transition temperature can be achieved. The aryl group is not particularly limited but, specifically, includes a phenyl group, a naphthyl group, a biphenyl group, a phenanthryl group, a terphenyl group, a pyrenyl group, a fluoranthenyl group, and a fluorenyl group. In view of sublimability, a phenyl group is preferred.

The fluoranthene derivative represented by formula (1) is preferably any structure of the following formulae (8) to (10). In the case of such an embodiment, the fluoranthene derivative has a group represented by L¹-(L²-(HAr)_{y})_{z} at the 7-position and/or the 8-position of the fluoranthene skeleton. In the fluoranthene derivative, when an aromatic substituent is substituted on the 7-position and/or the 8-position, the electron state of the fluoranthene skeleton is greatly changed and since the conjugation is efficiently extended, the charge transporting property is more enhanced. As a result, a light emitting device can be driven at a lower voltage, and the luminance efficiency can be more increased. Furthermore, extension of the conjugation leads to higher stability to a charge and in turn, when the fluoranthene derivative represented by formulae (8) to (10) of the present invention is used for a light emitting device, the life thereof can be further enhanced. In addition, enhancement of sublimability, enhancement of evaporation stability, reduction in crystallinity, and enhancement of film stability owing to a high glass transition temperature are achieved.

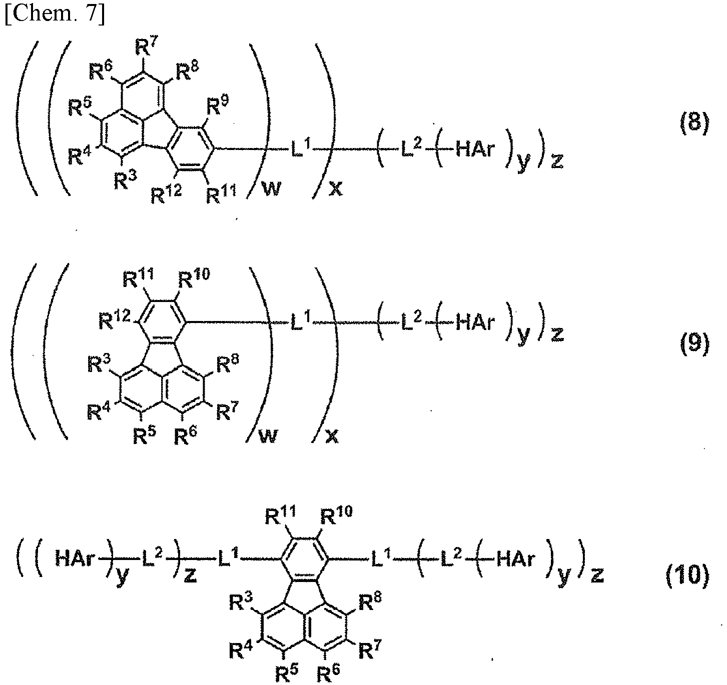

R³ to R¹² in formulae (8) to (10) are the same as those in formula (2), and L¹, L², HAr, w, x, y and z are the same as those in formula (1).

In addition, preferable groups of R³ to R¹² in formulae (8) to (10) are the same as in formulae (2) and (3).

In formulae (8) to (10), it is more preferred that w+x=3, and it is still more preferred that w=2 and x=1.

The group represented by L²-(HAr)_{y} is not particularly limited, but specific examples thereof include the followings, wherein those marked with "*" do not fall under the scope of the present invention. Each of these groups may be further substituted.

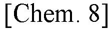

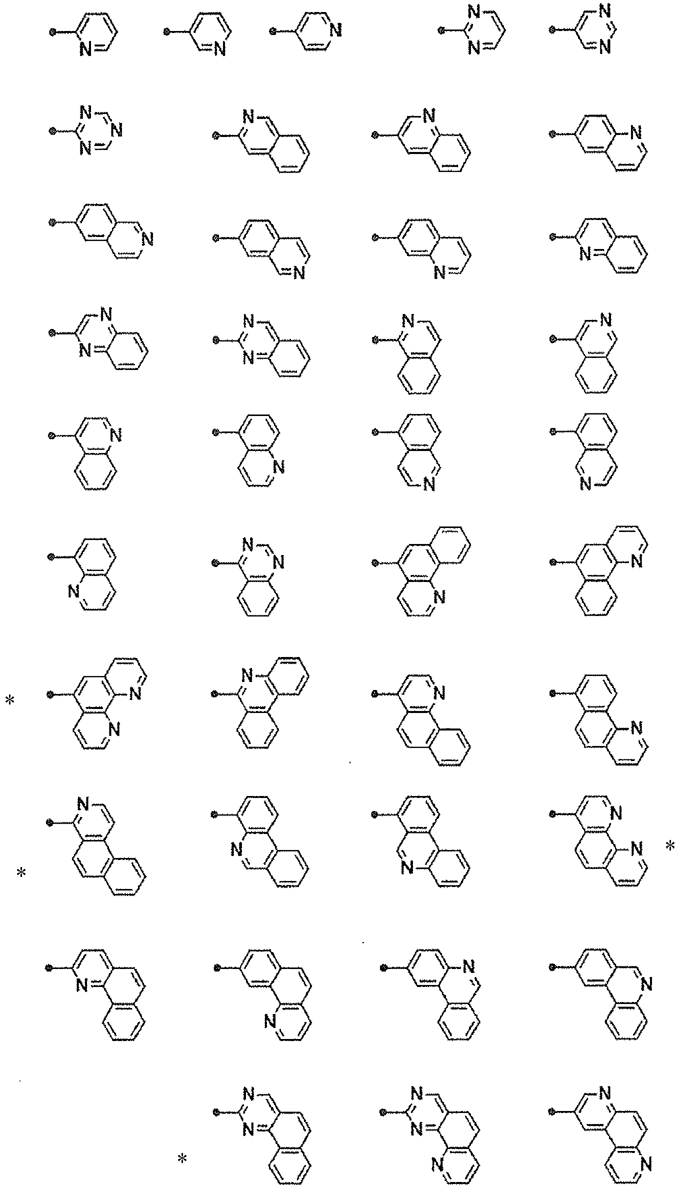

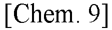

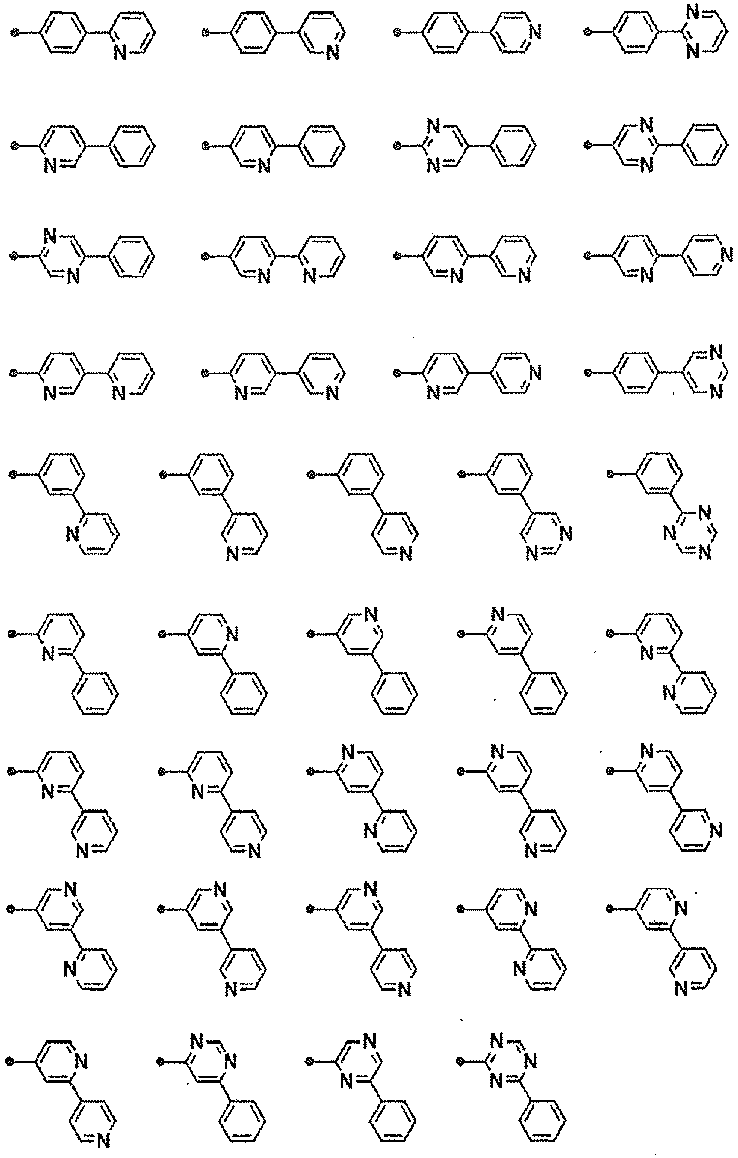

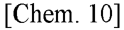

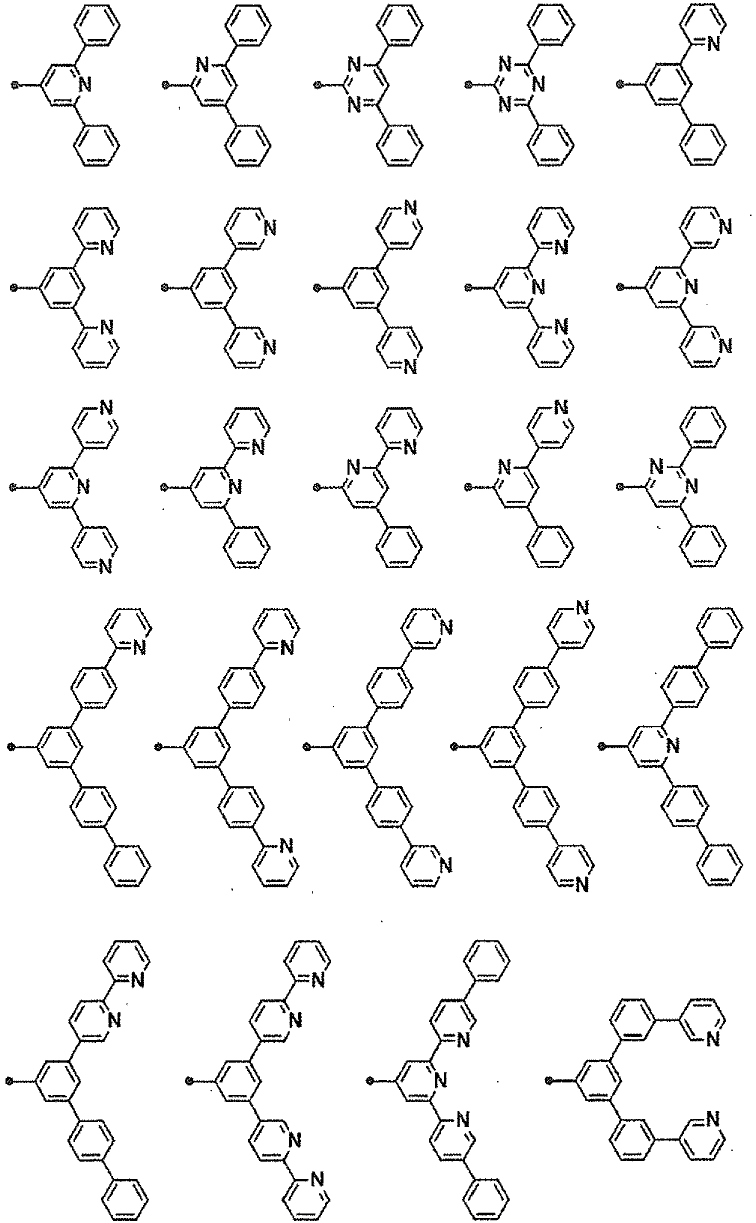

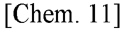

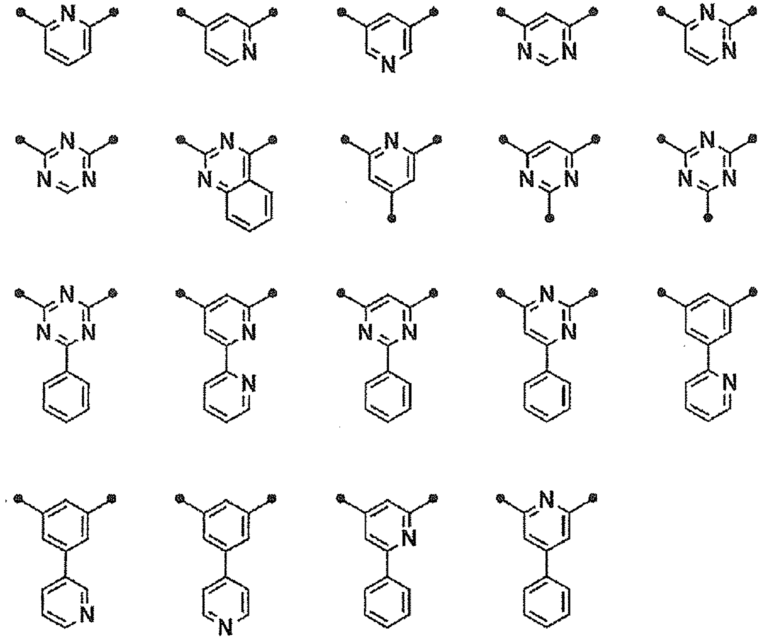

The fluoranthene derivative represented by formula (1) is more preferably either one structure, formula (9) or (10). In the case of such an embodiment, the fluoranthene derivative has a group represented by L¹-(L²-(HAr)_{y})_{z} at the 7-position of the fluoranthene skeleton. At this time, an effect of enhancing the charge transporting property to enhance the life of a light emitting device, an effect such as enhancement of sublimability, enhancement of evaporation stability and reduction in crystallinity, and an effect of enhancing the film stability owing to a high glass transition temperature are more increased.

The fluoranthene derivative represented by formula (1) is not particularly limited, but specific examples thereof include the followings, wherein those marked with "*" do not fall under the scope of the present invention.

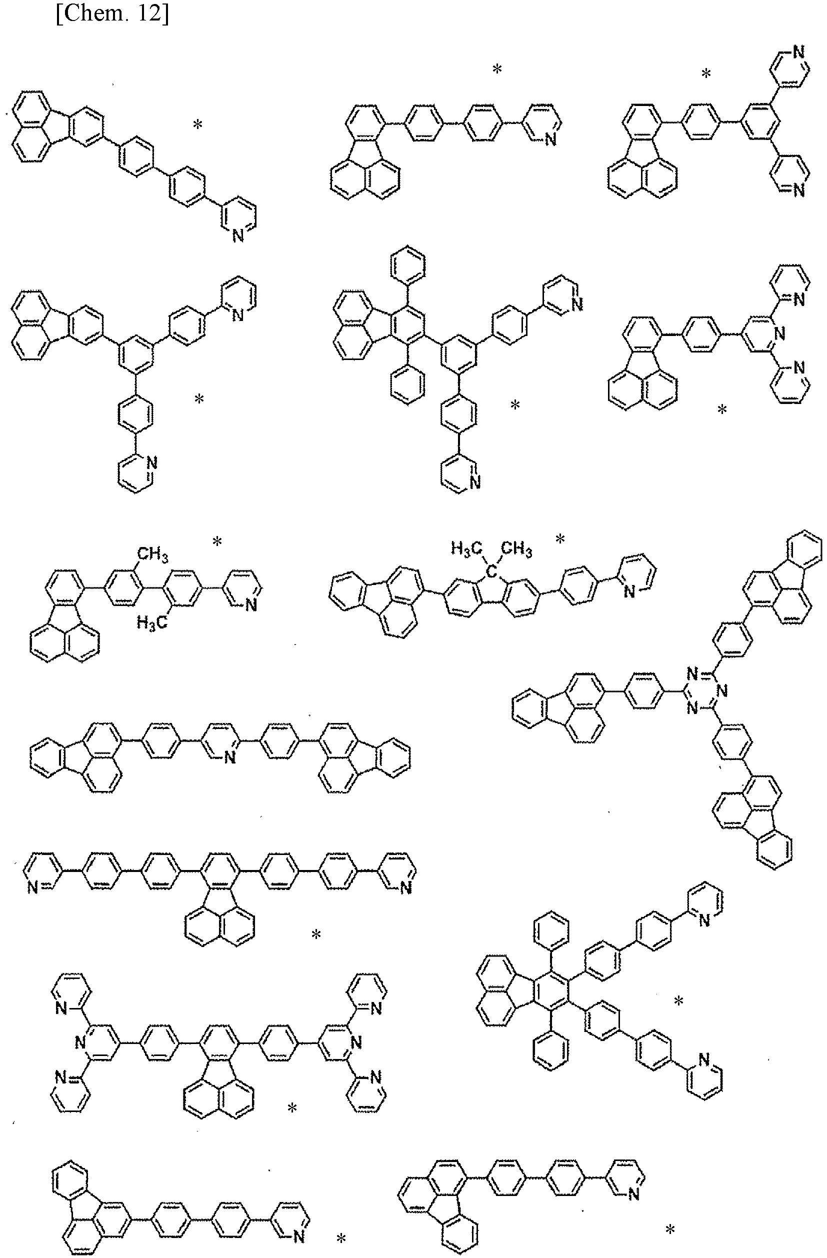

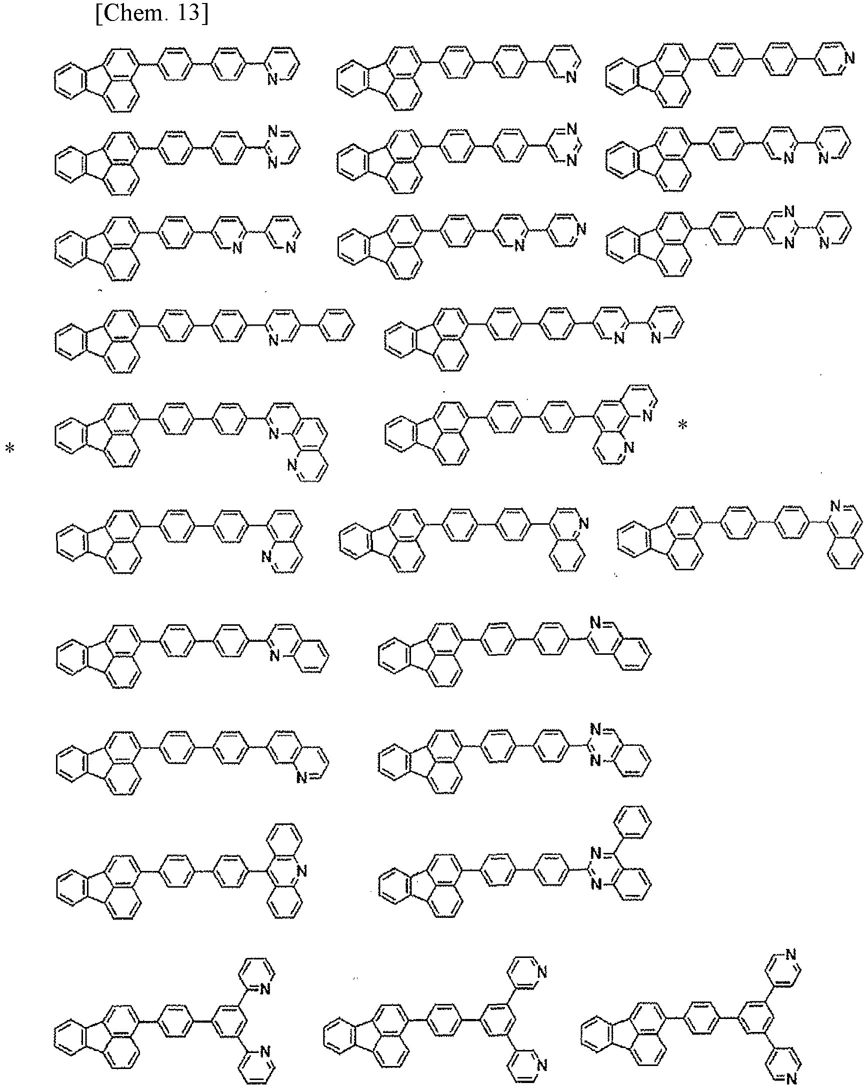

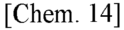

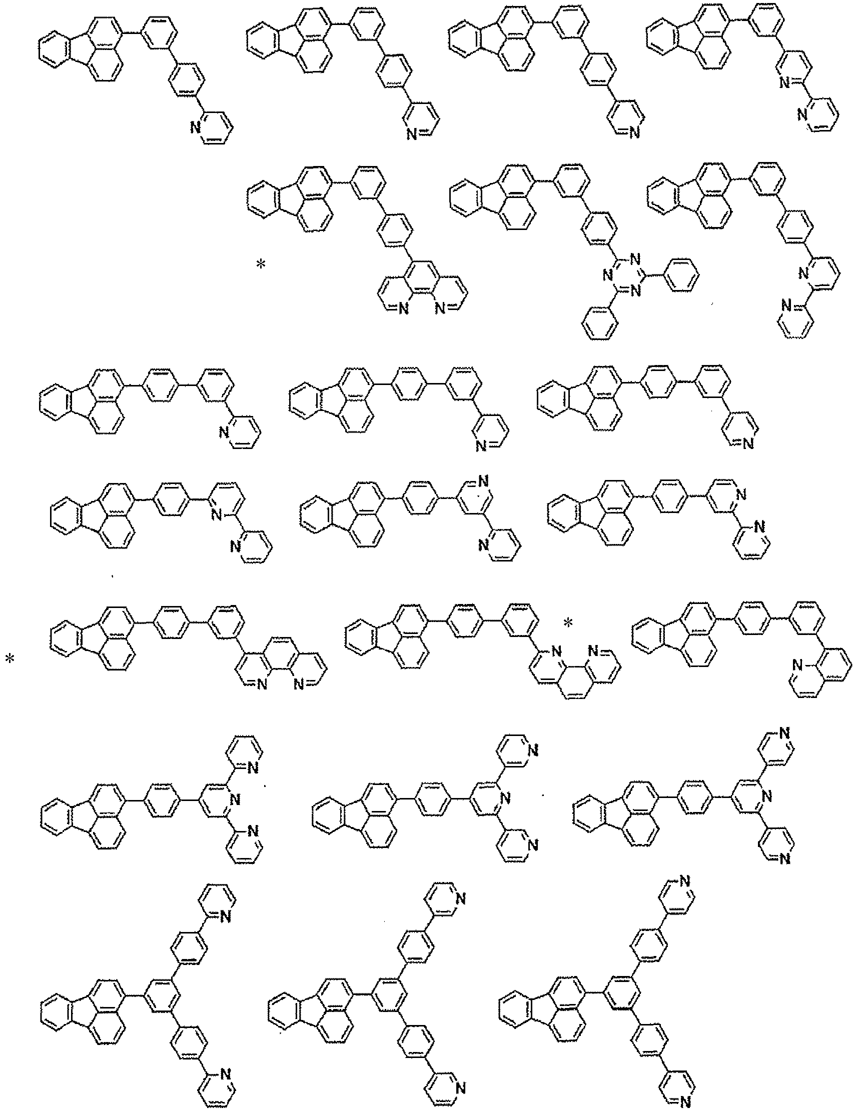

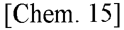

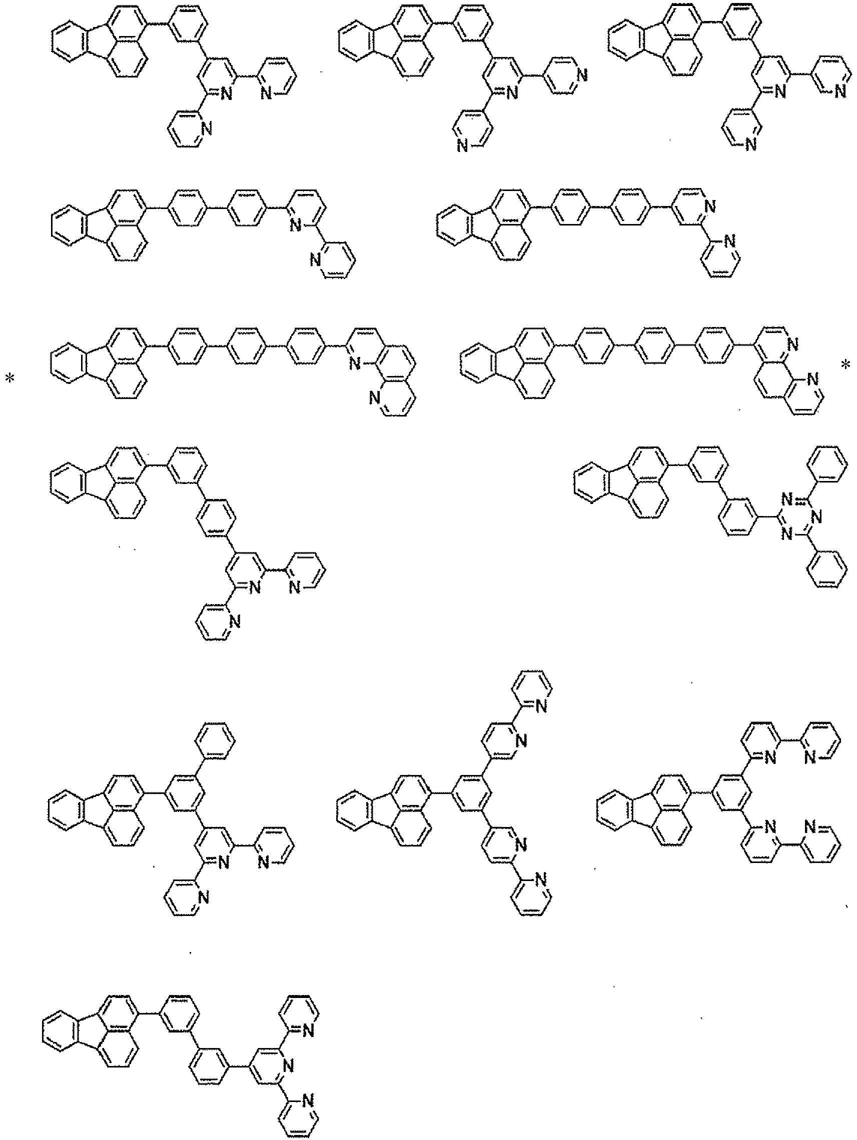

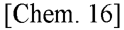

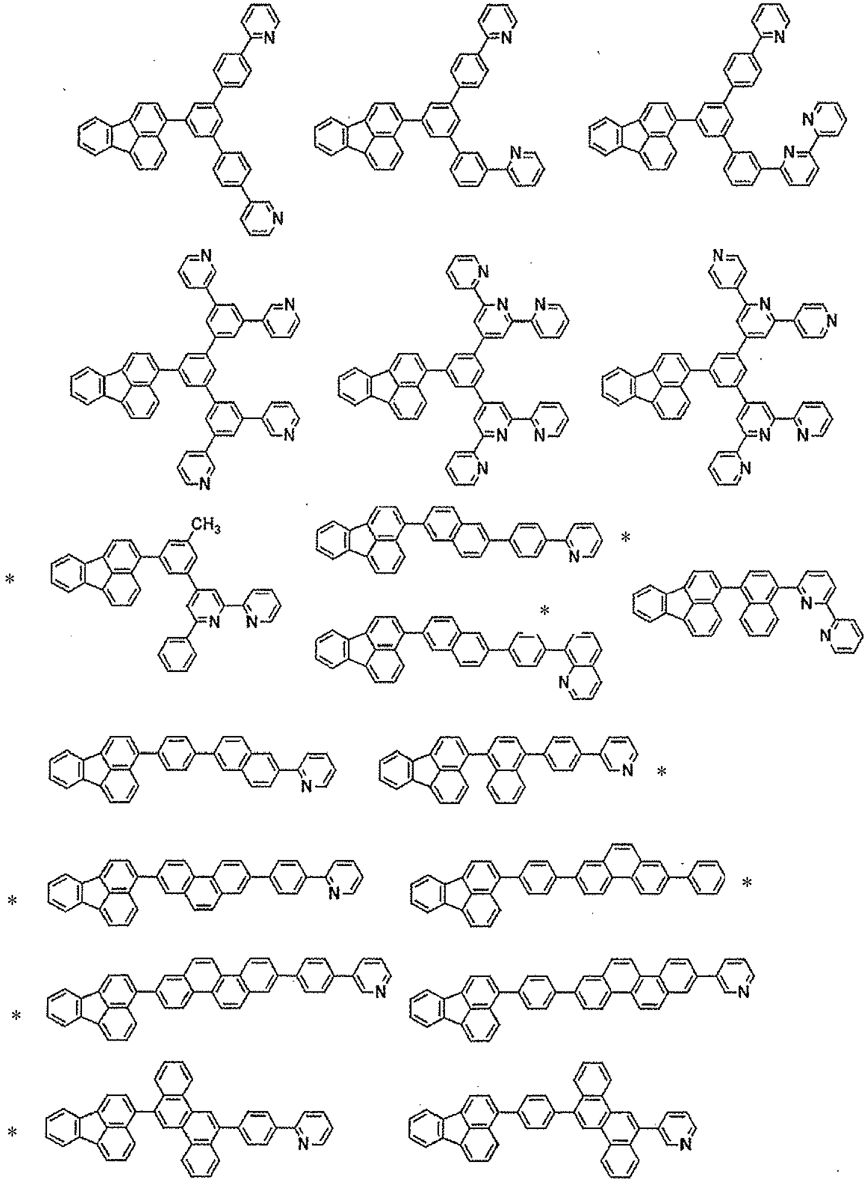

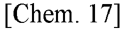

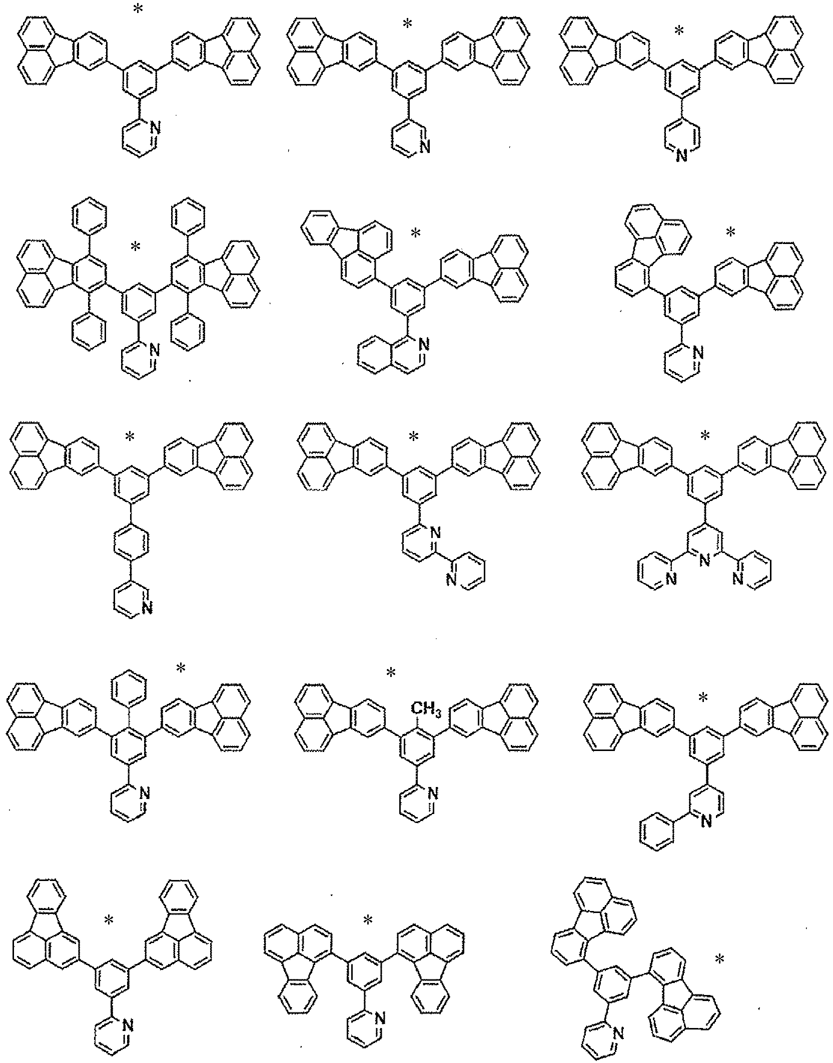

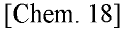

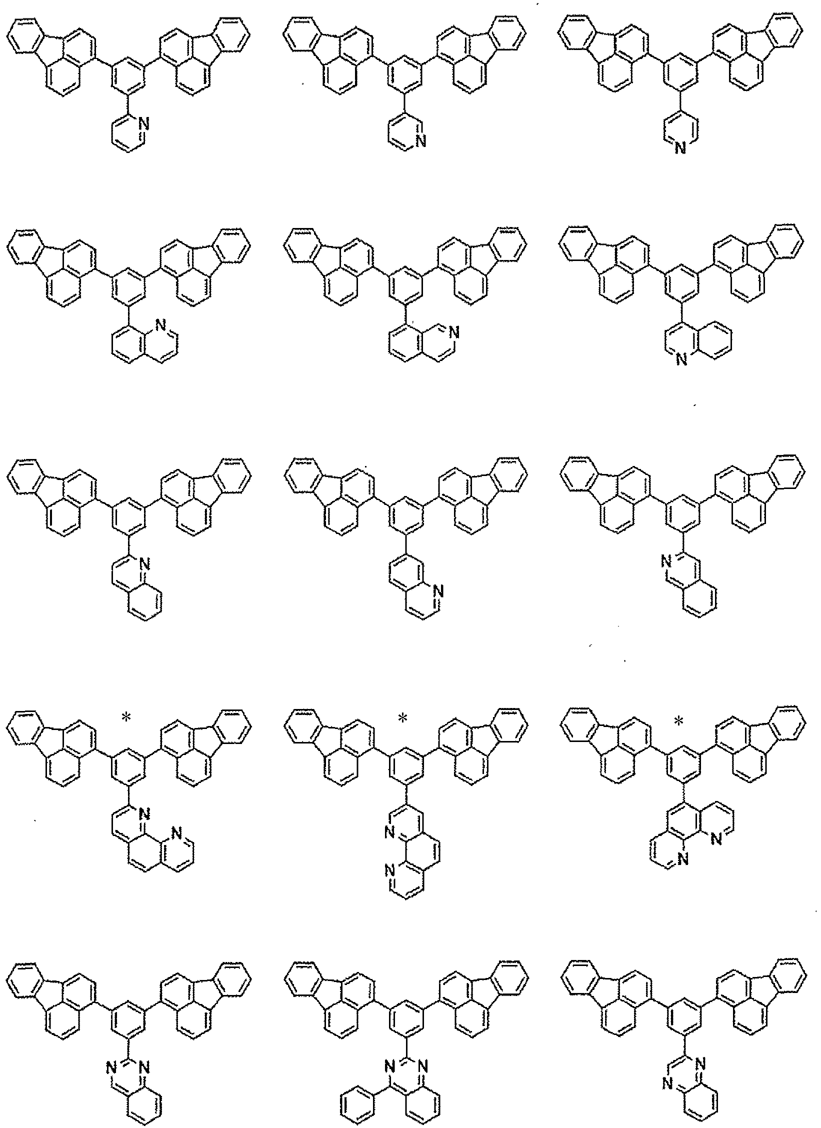

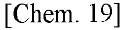

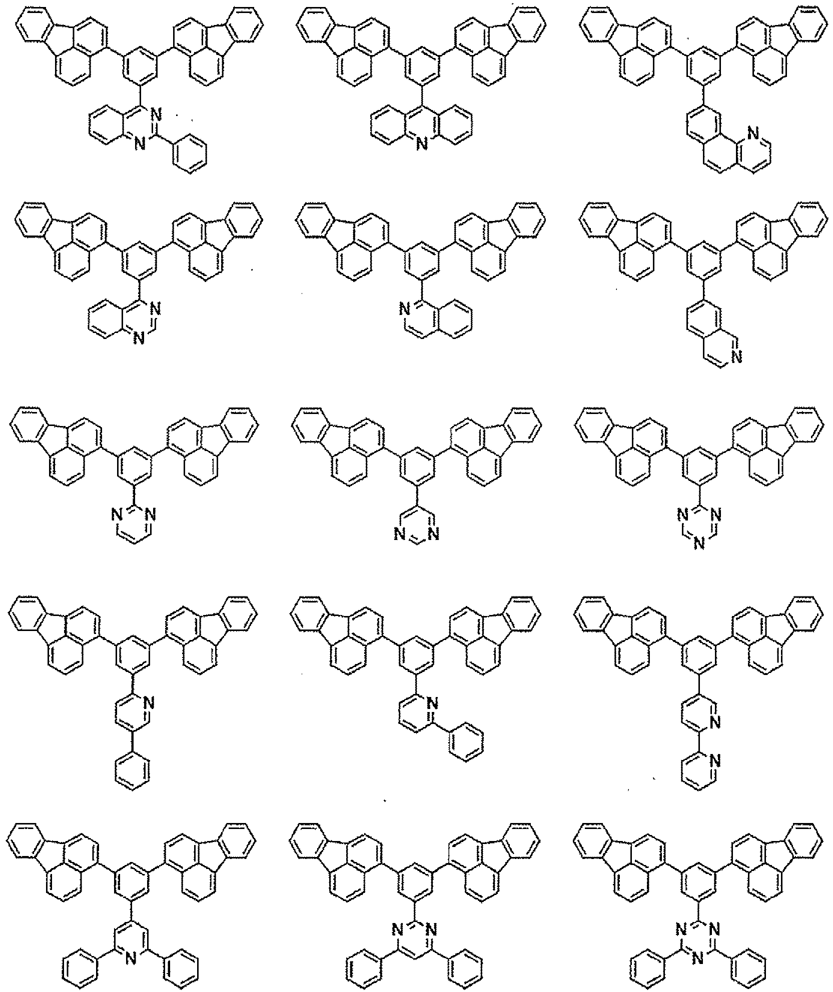

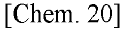

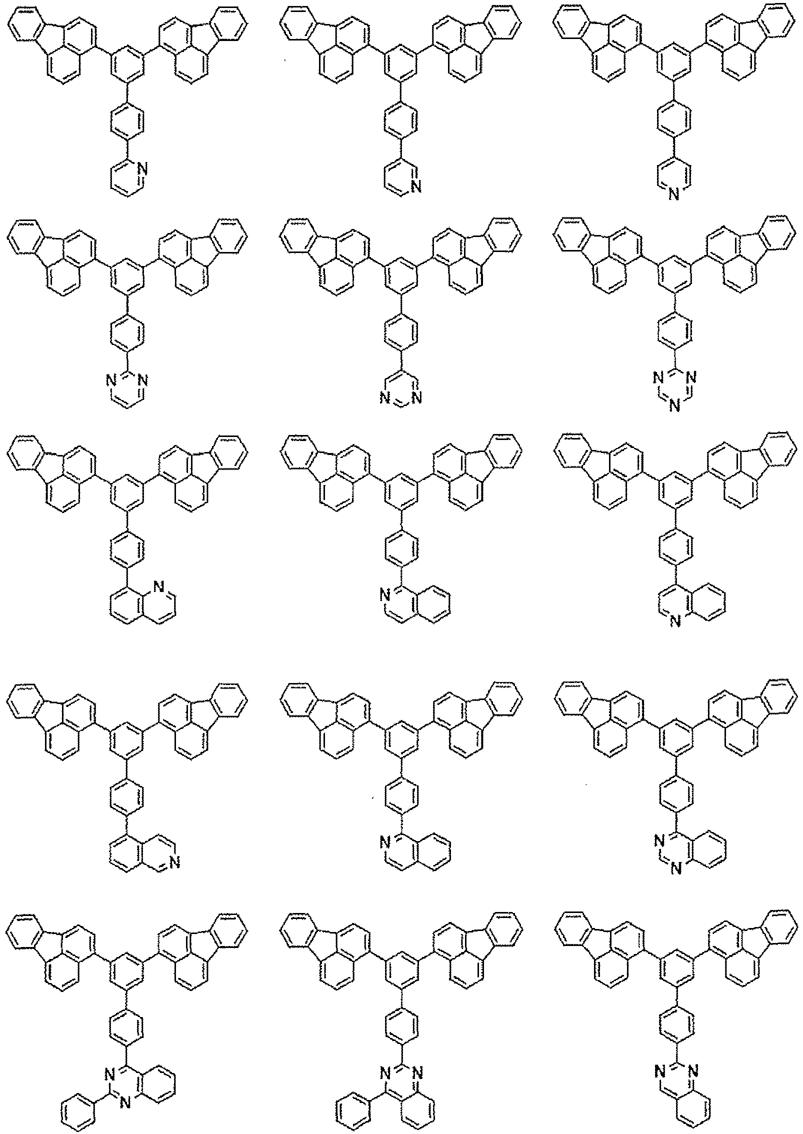

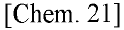

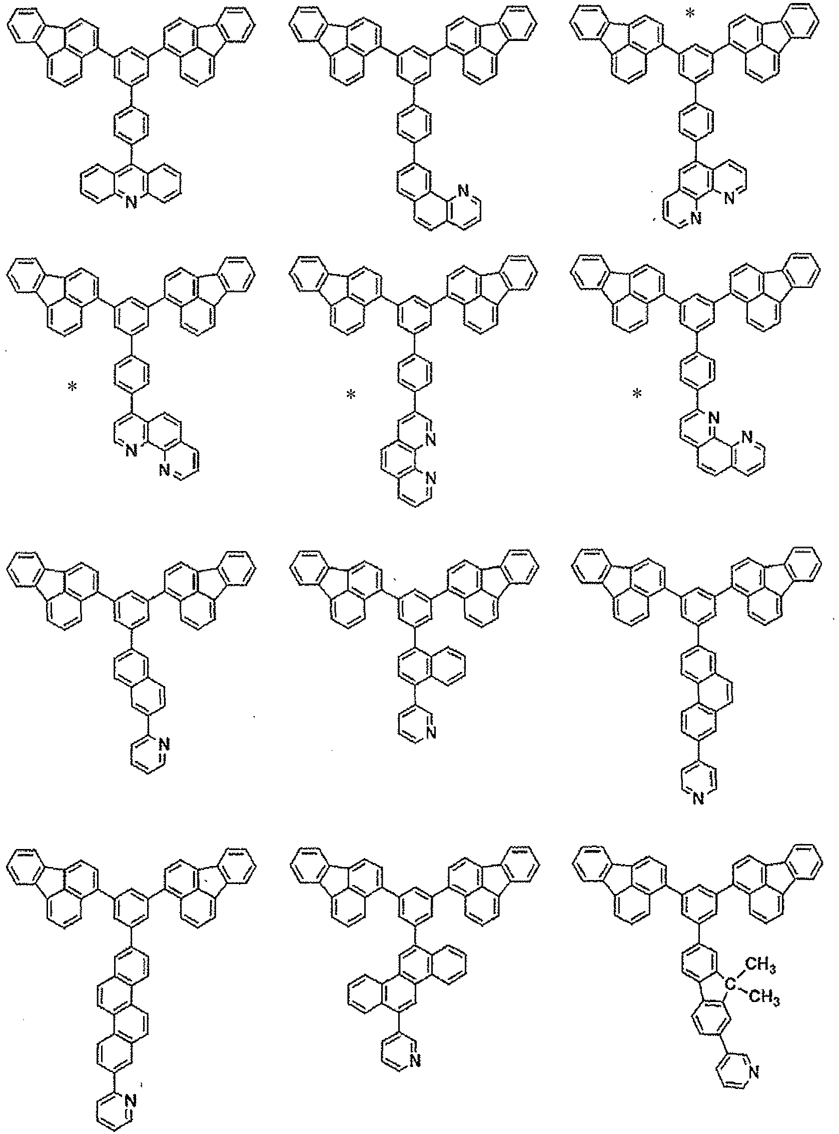

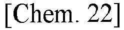

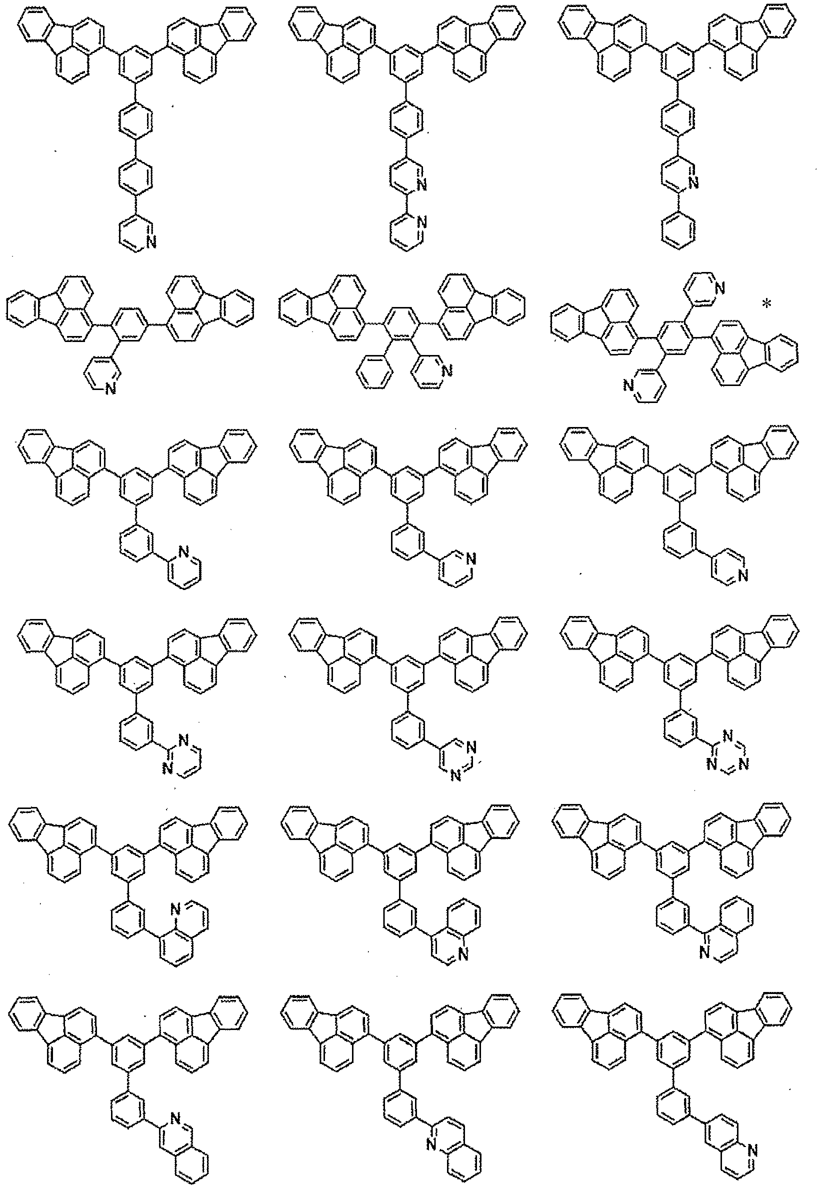

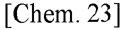

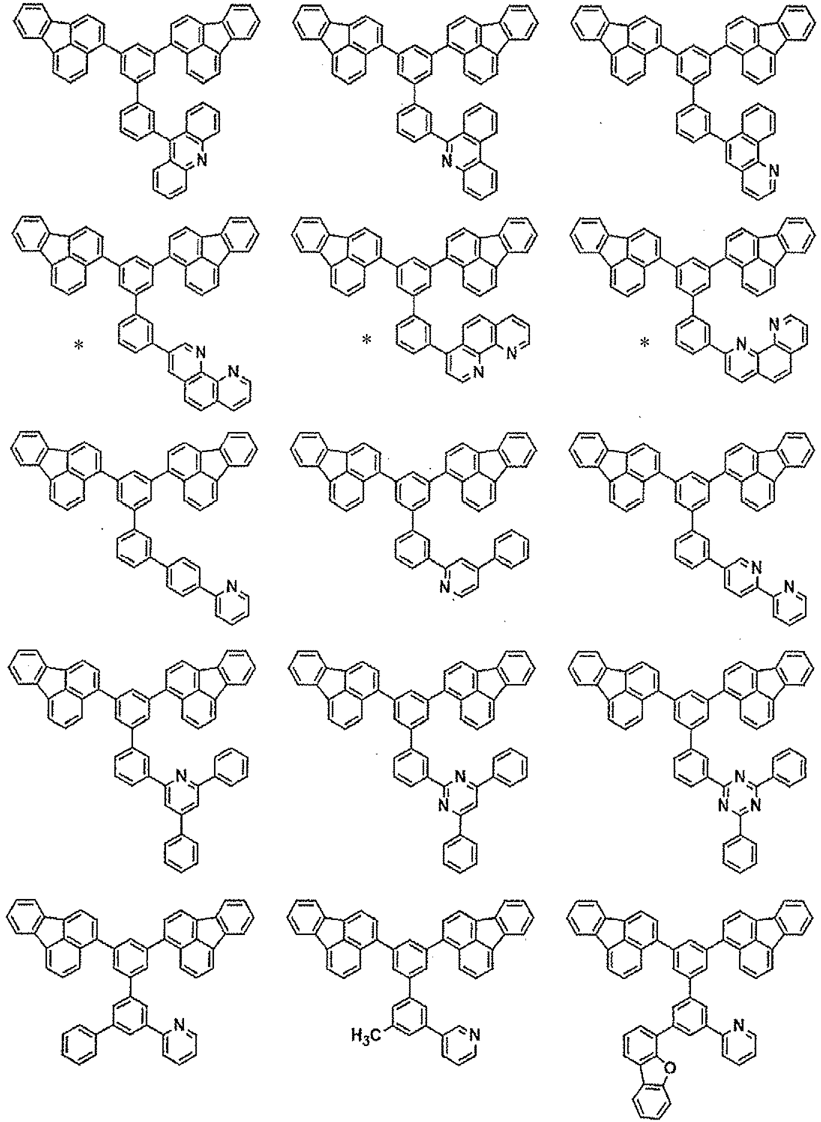

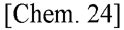

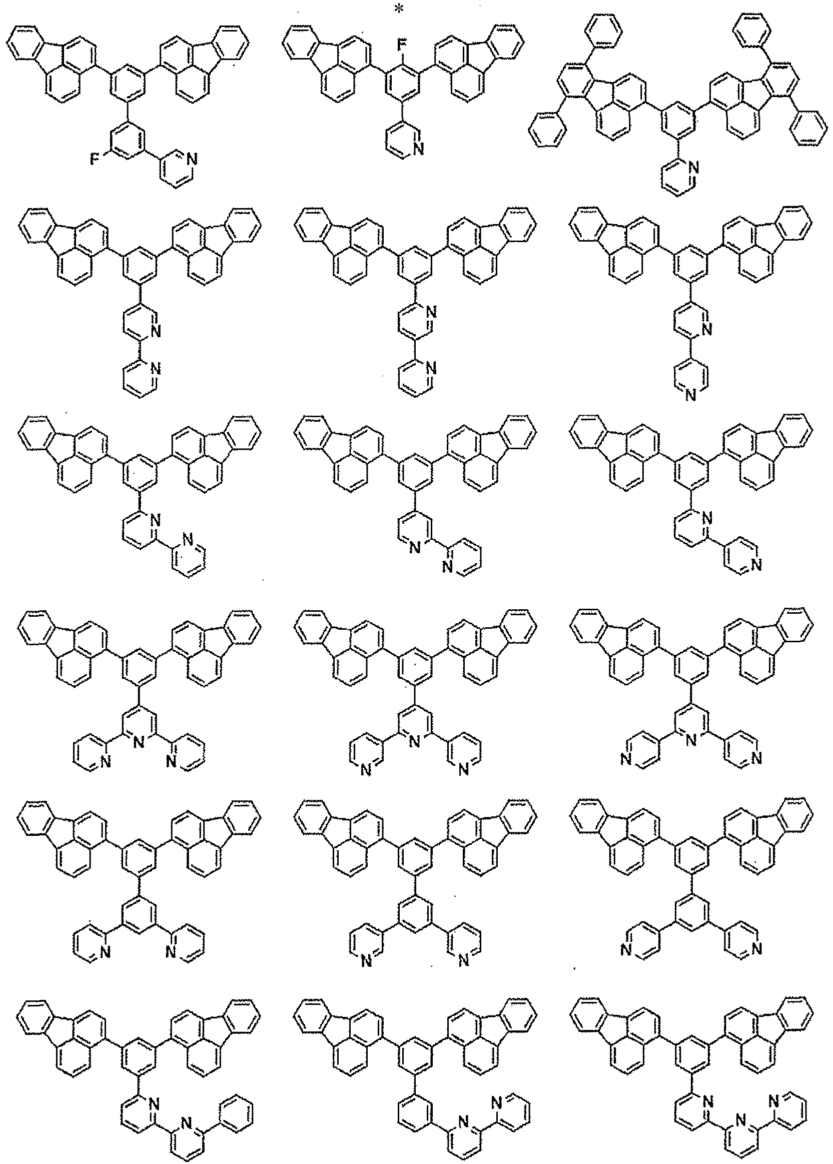

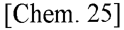

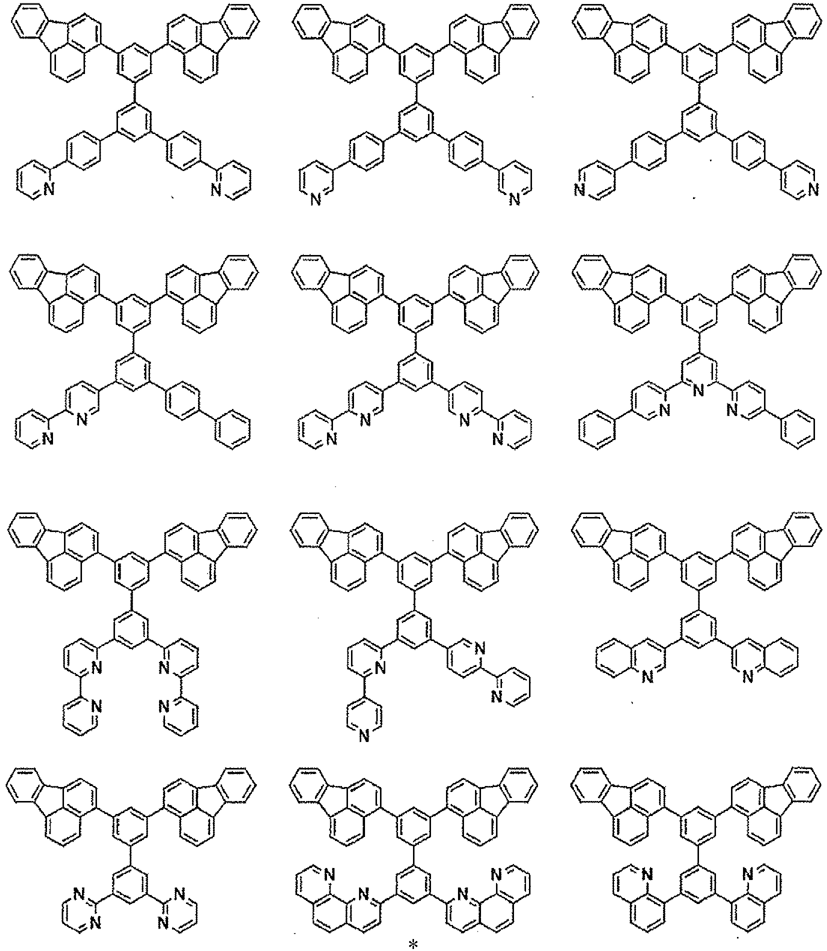

For the synthesis of the fluoranthene derivative of the present invention, a known method can be used. The method for introducing L¹-(L²-(HAr)_{y})_{z} into Ar includes, but is not limited to, for example, a method using a coupling reaction of a substituted or unsubstituted halogenated fluoranthene derivative and substituted or unsubstituted L¹-(L²-(HAr)_{y})_{z} in the presence of a palladium catalyst or a nickel catalyst, a method using a coupling reaction of a substituted or unsubstituted fluorantheneboronic acid derivative and substituted or unsubstituted L¹-(L²-(HAr)_{y})_{z} in the presence of a palladium catalyst or a nickel catalyst, and a method using a catalytic carbon-hydrogen bond activation coupling reaction of a substituted or unsubstituted fluoranthene derivative and substituted or unsubstituted L¹-(L²-(HAr)_{y})_{z} without using a halogenated fluoranthene derivative or a fluorantheneboronic acid derivative. The method for linking each of Ar, L¹, L² and HAr similarly includes, but is not limited to, the same methods using a coupling reaction. There is also a case of using not only a coupling reaction but also a cyclization reaction. Here, in the case of introducing L²-(HAr)_{y} into a fluoranthene skeleton through L¹, an arylboronic acid or heteroarylboronic acid substituted with L²-(HAr)_{y} may be used, or a fluoranthene derivative substituted with an aryl halide may be used. In addition, a boronic acid ester may be used in place of various boronic acids described above.

The fluoranthene derivative of the present invention is preferably used for a light emitting device, a photoelectric conversion element, a lithium ion battery, a fuel battery, and an electronic device such as transistor. The fluoranthene derivative of the present invention is preferably used as an electronic device material in an electronic device, and among others, is preferably used as a light emitting device material or a photoelectric conversion element material in a light emitting device or a photoelectric conversion element.

The light emitting device material indicates a material used in any layer of a light emitting device and, as described later, encompasses a material used in a protective film (cap layer) of an electrode, in addition to a material used in a layer selected from a hole transporting layer, an emissive layer and an electron transporting layer. By using the fluoranthene derivative of the present invention in any layer of a light emitting device, high luminance efficiency can be obtained, and a light emitting device having a low driving voltage and high durability can be obtained.

The photoelectric conversion element material indicates a material used in any layer of a photoelectric conversion element and, as described later, is a material used in a layer selected from a hole extraction material, a photoelectric conversion layer and an electron extraction layer. By using the compound of the present invention in any layer of a photoelectric conversion element, high conversion efficiency can be obtained.

### <Photoelectric Conversion Element>

A photoelectric conversion element has an anode, a cathode, and an organic layer interposed between the anode and the cathode, and in the organic layer, light energy is converted to an electric signal. The organic layer preferably has at least a photoelectric conversion layer, and it is more preferable for the photoelectric conversion layer to contain a p-type material and an n-type material. The p-type material is an electron-donating (donor property) material and has a shallow HOMO energy level, facilitating hole transport. The n-type material is an electron-withdrawing (acceptor property) material and has a deep LUMO energy level, facilitating electron transport. The p-type material and the n-type material may be laminated or mixed.

The organic layer includes, in addition to a configuration composed of only a photoelectric conversion layer, a laminated configuration such as 1) a hole extraction layer/a photoelectric conversion layer, 2) a photoelectric conversion layer/an electron extraction layer, and 3) a hole extraction layer/a photoelectric conversion layer/electron extraction layer. The electron extraction layer is a layer provided to facilitate electron extraction from a photoelectric conversion layer to a cathode and usually, is provided between a photoelectric conversion layer and a cathode. The hole extraction layer is a layer provided to facilitate hole extraction from a photoelectric conversion layer to an anode and usually, is provided between an anode and a photoelectric conversion layer. Each of these layers may be either a single layer or a plurality of layers.

The fluoranthene derivative of the present invention may be used in any layer of the above-described photoelectric conversion layer but is preferably used in a photoelectric conversion layer, because it has high electron affinity and thin-film stability as well as strong absorption in the visible light region. Among others, the compound has excellent electron transporting ability and therefore, is preferably used as an n-type material of a photoelectric conversion layer. In addition, the fluoranthene derivative of the present invention has particularly high electron affinity and therefore, can be suitably used also in an electron extraction layer. In this case, the electron extraction efficiency from the photoelectric conversion layer to the cathode is increased, so that the conversion efficiency can be enhanced.

The photoelectric conversion element can be used for an optical sensor. The photoelectric conversion element in this embodiment can also be used for a solar cell.

### <Light Emitting Device>

The embodiment of the light emitting device of the present invention is described in detail below.

The light emitting device of the present invention has an anode, a cathode, and an organic layer interposed between the anode and the cathode, and the organic layer has at least an emissive layer and an electron transporting layer. The organic layer, particularly the emissive layer, emits light by electric energy.

The organic layer includes, in addition to a configuration composed of only an emissive layer/an electron transporting layer, a laminated configuration such as 1) a hole transporting layer/an emissive layer/an electron transporting layer, 2) a hole transporting layer/an emissive layer/an electron transporting layer/an electron injection layer, and 3) a hole injection layer/a hole transporting layer/ an emissive layer/an electron transporting layer/an electron injection layer. Each of these layers may be either a single layer or a plurality of layers. The configuration may be of a laminated type having a plurality of phosphorescence emitting layers or fluorescence emitting layers, and a fluorescence emitting layer and a phosphorescence emitting layer may be combined in the light emitting device. Furthermore, emissive layers having emission colors different from each other may be stacked.

The device configuration may also be of a tandem type having a plurality of layers stacked through an intermediate layer. Above all, at least one layer is preferably a phosphorescence emitting layer. The intermediate layer described above is generally also called an intermediate electrode, an intermediate conductive layer, a charge generation layer, an electron-withdrawing layer, a connection layer, or an intermediate insulating layer, and a known material configuration can be used. Specific examples of the tandem type include a laminated configuration containing a charge generation layer as an intermediate layer between the anode and the cathode, such as 4) a hole transporting layer/an emissive layer/an electron transporting layer/a charge generation layer/a hole transporting layer/an emissive layer/an electron transporting layer, and 5) a hole injection layer/a hole transporting layer/an emissive layer/an electron transporting layer/an electron injection layer/a charge generation layer/a hole injection layer/a hole transporting layer/an emissive layer/an electron transporting layer/an electron injection layer. As the material constituting the intermediate layer, specifically, a pyridine derivative or a phenanthroline derivative is preferably used.

In the device configuration described above, the fluoranthene derivative of the present invention may be used in any layer but is preferably used in an emissive layer, an electron transporting layer or an intermediate layer of the light emitting device, because it has high electron injection/transporting ability, fluorescence quantum yield and thin-film stability. Above all, the compound has excellent electron injection/transporting ability and therefore, is preferably used in an electron transporting layer or an intermediate layer. In the case of using it in an intermediate layer, the compound is preferably used in a charge generation layer. Among these, the fluoranthene derivative may be suitably used in an electron transporting layer.

### (Anode and Cathode)

In the light emitting device of the present invention, the anode and the cathode have a role in supplying a sufficient current for light emission of the device, and at least one electrode is preferably transparent or translucent so as to extract light. Usually, the anode formed on a substrate is assigned to a transparent electrode.

The material used for the anode is not particularly limited as long as it is a material capable of efficiently injecting a hole into an organic layer and is transparent or translucent so as to extract light, and the material may be, for example, an electrically conductive metal oxide such as tin oxide, indium oxide, indium tin oxide (ITO) and indium zinc oxide (IZO), a metal such as gold, silver and chromium, an inorganic electrically conductive substance such as copper iodide and copper sulfide, or an electrically conductive polymer such as polythiophene, polypyrrole and polyaniline, but use of ITO glass or NESA glass is particularly preferred. One of these electrode materials may be used alone, or a plurality of materials may be used as a laminate or a mixture. Since it suffices if a sufficient current for light emission of the device can be supplied, the resistance of the transparent electrode is not limited, but from the viewpoint of power consumption of the device, a low resistance is preferred. For example, an ITO substrate having a resistance of 300 Ω/square or less functions as a device electrode, but since a substrate having a resistance of about 10 Ω/square can be currently supplied, it is particularly preferable to use a substrate having a low resistance of 20 Ω/square or less. The thickness of ITO can be arbitrarily selected according to the resistance value, but a substrate usually having a thickness of 100 to 300 nm is used in many cases.

In order to maintain the mechanical strength of a light emitting device, the light emitting device is preferably formed on a substrate. As the substrate, a glass substrate such as soda glass or alkali-free glass is suitably used. Since it suffices if the thickness of the glass substrate is large enough to maintain the mechanical strength, a thickness of 0.5 mm or more is sufficient. As for the material of glass, alkali-free glass is preferred, because the amount of ions dissolved out from glass is preferably smaller. Alternatively, soda-lime glass provided with a barrier coating such as SiO₂ is commercially available and can also be used. Furthermore, the substrate need not be glass as long as the first electrode stably functions, and, for example, the anode may be formed on a plastic substrate. The method for forming an ITO film is not particularly limited and includes an electron beam method, a sputtering method, and a chemical reaction method.

The material used for the cathode is not particularly limited as long as it is a substance capable of efficiently injecting an electron into the emissive layer. In general, a metal such as platinum, gold, silver, copper, iron, tin, aluminum and indium, and an alloy or multilayer laminate of such a metal described above with a low-work-function metal such as lithium, sodium, potassium, calcium and magnesium, are preferred. Among others, as the main component, aluminum, silver and magnesium are preferred in view of electric resistance value, ease of film formation, film stability, luminance efficiency, etc. Above all, when the material is composed of magnesium and silver, advantageously, electron injection into the electron transporting layer and the electron injection layer in the present invention is facilitated, and low voltage driving becomes possible.

Furthermore, in order to protect the cathode, as a preferable example, a metal such as platinum, gold, silver, copper, iron, tin, aluminum and indium, an alloy using such a metal, an inorganic material such as silica, titania and silicon nitride, or an organic polymer compound such as polyvinyl alcohol, polyvinyl chloride and hydrocarbon-based polymer compound, is laminated as a protective film layer on the cathode. The fluoranthene derivative of the present invention can also be utilized as the protective film layer (cap layer). However, in the case of a device structure of taking out light from the cathode side (top emission structure), the protective film layer is selected from materials having light permeability in the visible light region. The method for manufacturing these electrodes is not particularly limited and includes resistance heating, electron beam, sputtering, ion plating, and coating.

### (Hole Transporting Layer)

The hole transporting layer is formed by a method of laminating or mixing one species or two or more species of the hole transporting material, or a method using a mixture of a hole transporting material and a polymer binder. The hole transporting material must allow a hole from a positive electrode to be efficiently transported between electrodes, to which an electric field is applied, and it is preferable to have high hole injection efficiency and efficiently transport the injected hole. For this purpose, the hole transporting material is required to be a substance having appropriate ionization potential, large hole mobility and excellent stability and hardly allowing an impurity working out to a trap to be generated at the time of production and during use.

The substance satisfying these conditions is not particularly limited but includes, for example, a benzidine derivative such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB) and bis(N,N'-diphenyl-4-aminophenyl)-N,N-diphenyl-4,4'-diamino-1,1'-biphenyl (TPD232); materials called starburst arylamine, such as 4,4',4"-tris(3-methylphenyl(phenyl)amino)triphenylamine (m-MTDATA) and 4,4',4"-tris(1-naphthyl(phenyl)amino)triphenylamine (1-TNATA); and a material having a carbazole skeleton.

Among others, a carbazole multimer, specifically, a derivative of a carbazole dimer such as bis(N-arylcarbazole) or bis(N-alkylcarbazole), a derivative of a carbazole trimer, and a derivative of a carbazole tetramer; triphenylene compound; pyrazoline derivative; stilbene-based compound; hydrazone-based compound; benzofuran derivative; a heterocyclic compound such as thiophene derivative, oxadiazole derivative, phthalocyanine derivative and porphyrin derivative; a fullerene derivative; and a polymer having the monomer described above on the side chain, such as polycarbonate, styrene derivative, polythiophene, polyaniline, polyfluorene, polyvinylcarbazole and polysilane, are preferred. Furthermore, an inorganic compound such as p-type Si and p-type SiC can also be used. The fluoranthene derivative of the present invention is excellent in the electrochemical stability and therefore, can be used as the hole transporting material.

The fluoranthene derivative of the present invention is excellent in the electron injection/transporting properties and therefore, when this is used in the electron transporting layer, electrons may not be recombined in the emissive layer and partially leaked to the hole transporting layer. Accordingly, a compound excellent in the electron blocking property is preferably used in the hole transporting layer. Among others, a carbazole skeleton-containing compound is preferred, because this compound is excellent in the electron blocking property and can contribute to high efficiency of the light emitting device. Furthermore, the carbazole skeleton-containing compound preferably contains a carbazole dimer, carbazole trimer or carbazole tetramer skeleton, because these have both good electron blocking property and good hole injection/transporting properties.

In the case of using a carbazole skeleton-containing compound in the hole transporting layer, it is more preferred that the emissive layer combined contains the later-described phosphorescence emitting material, because the carbazole skeleton-containing compound also has a high triplet exciton blocking function and when combined with a phosphorescence emitting material, high luminance efficiency can be achieved. In addition, when a triphenylene skeleton-containing compound excellent in terms of having high hole mobility is used in the hole transporting layer, advantageously, the carrier balance is enhanced and an effect such as increase of luminance efficiency and improvement of durable life is obtained. It is more preferable for the triphenylene skeleton-containing compound to have two or more diarylamino groups. The carbazole skeleton-containing compound and the triphenylene skeleton-containing compound each may be used alone as a hole transporting layer, or may be mixed together and used. Other materials may be mixed to an extent that the effects of the present invention are not impaired. In the case where the hole transporting layer is composed of a plurality of layers, it may be sufficient if the carbazole skeleton-containing compound or the triphenylene skeleton-containing compound is contained in any one layer.

### (Hole Injection Layer)

A hole injection layer may be provided between the anode and the hole transporting layer. By providing a hole injection layer, the driving voltage of the light emitting device is reduced, and durable life thereof is enhanced. A material having an ionization potential smaller than that of the material usually used in the hole transporting layer is preferably used in the hole injection layer. Specifically, examples of the material include a benzidine derivative such as TPD232, and starburst arylamine materials, and in addition, a phthalocyanine derivative, etc. may be used as well. It is also preferred that the hole-injection layer is composed of an acceptor compound alone or an acceptor compound is used by being doped into another hole transporting material. Examples of the acceptor compound include a metal chloride such as iron(III) chloride, aluminum chloride, gallium chloride, indium chloride and antimony chloride, a metal oxide such as molybdenum oxide, vanadium oxide, tungsten oxide and ruthenium oxide, and a charge transfer complex such as tris(4-bromophenyl)-aminium hexachloroantimonate (TBPAH). An organic compound having a nitro group, a cyano group, a halogen or a trifluoromethyl group in the molecule thereof, a quinone-based compound, an acid anhydride-based compound, fullerene, etc. are also suitably used. Specific examples of these compounds include hexacyanobutadiene, hexacyanobenzene, tetracyanoethylene, tetracyanoquinodimethane (TCNQ), tetrafluorotetracyanoquinodimethane (F₄-TCNQ), 2,3,6,7,10,11-hexacyano-1,4,5,8,9,12-hexaazatriphenylene (HAT-CN₆), p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C₆₀, and C₇₀.

Among these, a metal oxide and a cyano group-containing compound are preferred, because these are easy to handle and to evaporate and the above-described effects are easily obtained. Preferable examples of the metal oxide include molybdenum oxide, vanadium oxide, and ruthenium oxide. Of cyano group-containing compounds, (a) a compound having, in the molecule thereof, at least one electron-accepting nitrogen other than the nitrogen atom of the cyano group, (b) a compound having both a halogen and a cyano group in the molecule thereof, (c) a compound having both a carbonyl group and a cyano group in the molecule thereof, or (d) a compound having, in the molecule thereof, both a halogen and a cyano group and further, at least one electron-accepting nitrogen atom other than the nitrogen atom of the cyano group, is more preferred, because the compound works out to a strong electron acceptor. Specifically, such a compound includes the following compounds.

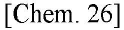

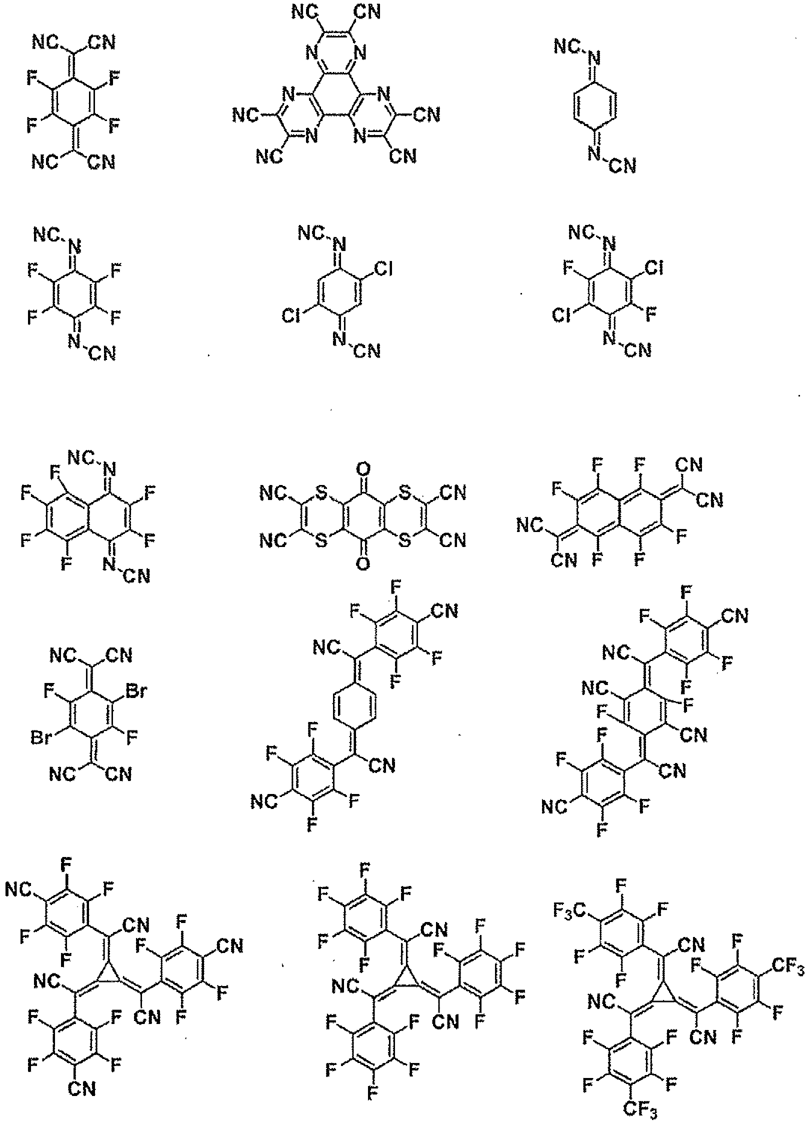

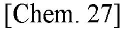

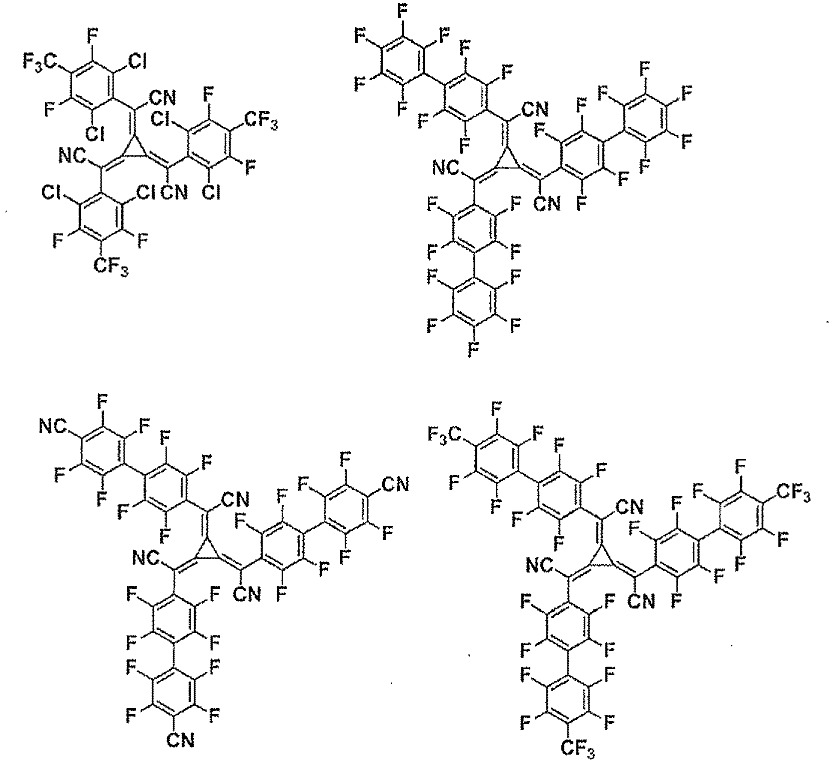

In both of the case where the hole injection layer is composed of an acceptor compound alone and the case where an acceptor compound is doped into the hole injection layer, the hole injection layer may be a single layer or may be formed by stacking a plurality of layers. In the case where an acceptor compound is doped into the hole injection layer, from the viewpoint that a barrier to hole injection into the hole transporting layer can be mitigated, the hole injection material used in combination is preferably the same compound as the compound used in the hole transporting layer.

### (Emissive Layer)

The emissive layer may be composed of a single layer or a plurality of layers, and in either case, the layer is formed of an emissive material (host material, dopant material). The emissive material may be a mixture of a host material and a dopant material or may be a host material alone. More specifically, in the light emitting device of the present invention, only a host material or a dopant material may emit light, or both a host material and a dopant material may emit light, in each emissive layer. From the viewpoint of efficiently utilizing electric energy and obtaining light emission with high color purity, the emissive layer preferably includes a mixture of a host material and a dopant material. Each of the host material and the dopant material may be one kind of a material or a combination of a plurality of kinds of materials. The dopant material may be contained in the whole or a part of the host material. The dopant material may be laminated or may be dispersed. The dopant material can control the emission color. If the amount of the dopant material is too large, concentration quenching occurs, and therefore, the amount thereof used is preferably 20 wt% or less, more preferably 10 wt% or less, relative to the host material. As for the doping method, the layer may be formed by co-evaporation with the host material, but the dopant material may be previously mixed with the host material and then evaporated simultaneously.

The emissive material that can be used is not particularly limited but includes, specifically, a fused ring derivative such as anthracene and pyrene, a metal chelated oxinoid compound such as tris(8-quinolinolato)aluminum, a bisstyryl derivative such as bisstyrylanthracene derivative and distyrylbenzene derivative, a tetraphenylbutadiene derivative, an indene derivative, a coumarin derivative, an oxadiazole derivative, a pyrrolopyridine derivative, a perinone derivative, a cyclopentadiene derivative, an oxadiazole derivative, a thiadiazolopyridine derivative, a dibenzofuran derivative, a carbazole derivative, an indolocarbazole derivative, and as a polymer, a polyphenylenevinylene derivative, a polyparaphenylene derivative, a polythiophene derivative, etc., which have been conventionally known as a luminescent material.

The host material contained in the emissive material is not particularly limited, but includes a compound having a fused aryl ring, such as naphthalene, anthracene, phenanthrene, pyrene, chrysene, naphthacene, triphenylene, perylene, fluoranthene, fluorene and indene, a derivative thereof, an aromatic amine derivative such as N,N'-dinaphthyl-N,N'-diphenyl-4,4'-diphenyl-1,r-diamine, a metal chelated oxinoid compound such as tris(8-quinolinato)aluminum(III), a bisstyryl derivative such as distyrylbenzene derivative, a tetraphenylbutadiene derivative, an indene derivative, a coumarin derivative, an oxadiazole derivative, a pyrrolopyridine derivative, a perinone derivative, a cyclopentadiene derivative, a pyrrolopyrrole derivative, a thiadiazolopyridine derivative, a dibenzofuran derivative, a carbazole derivative, an indolocarbazole derivative, a triazine derivative, and as a polymer, a polyphenylenevinylene derivative, a polyparaphenylene derivative, a polyfluorene derivative, polyvinylcarbazole derivative, a polythiophene derivative, etc. The dopant material that can be used is not particularly limited but includes a compound having a fused aryl ring, such as naphthalene, anthracene, phenanthrene, pyrene, chrysene, triphenylene, perylene, fluoranthene, fluorene and indene, and a derivative thereof (e.g., 2-(benzothiazol-2-yl)-9,10-diphenylanthracene, 5,6,11,12-tetraphenylnaphthacene); a compound having a heteroaryl ring, such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyridine, pyrazine, naphthyridine, quinoxaline, pyrrolopyridine and thioxanthene, and a derivative thereof; a borane derivative; a distyrylbenzene derivative; an aminostyryl derivative such as 4,4'-bis(2-(4-diphenylaminophenyl)ethenyl)biphenyl and 4,4'-bis(N-(stilben-4-yl)-N-phenylamino)stilbene; an aromatic acetylene derivative; a tetraphenylbutadiene derivative; a stilbene derivative; an aldazine derivative; a pyrromethene derivative; a diketopyrrolo[3,4-c]-pyrrole derivative; a coumarin derivative such as 2,3,5,6-1H,4H-tetrahydro-9-(2'-benzothiazolyl)quinolizino[9,9a,1-gh] coumarin; an azole derivative such as imidazole, thiazole, thiadiazole, carbazole, oxazole, oxadiazole and triazole, and a metal complex thereof; an aromatic amine derivative typified by N,N'-diphenyl-N,N'-di(3-methylphenyl)-4,4'-diphenyl-1,1'-diamine; etc.

A phosphorescence emitting material may be contained in the emissive layer. The phosphorescence emitting material is a material that exhibits phosphorescence emission even at room temperature. In the case of using a phosphorescence emitting material as a dopant, basically, phosphorescence emission must be obtained also at room temperature, and the phosphorescence emitting material, which is not particularly limited, is preferably an organic metal complex compound containing at least one metal selected from the group consisting of iridium (Ir), ruthenium (Ru), rhodium (Rh), palladium (Pd), platinum (Pt), osmium (Os) and rhenium (Re). Among others, an organic metal complex having iridium or platinum is preferred from the viewpoint of providing a high phosphorescence emission yield even at room temperature. As the host used in combination with a phosphorescence emitting dopant, indole derivative; carbazole derivative; indolocarbazole derivative; nitrogen-containing aromatic compound derivative having a pyridine, pyrimidine or triazine skeleton; polyarylbenzene derivative; spirofluorene derivative; truxene derivative; an aromatic hydrocarbon compound derivative such as triphenylene derivative; a chalcogen element-containing compound such as dibenzofuran derivative and dibenzothiophene derivative; an organic metal complex such as beryllium quinolinol complex; etc. are suitably used, but basically, as long as the triplet energy is higher than that of the dopant used and an electron and a hole are smoothly injected or transported from respective transporting layers, the host is not limited thereto. Two or more triplet emissive dopants may be contained, and two or more host materials may be contained. Furthermore, one or more triplet emissive dopants and one or more fluorescence emitting dopants may be contained.

The preferable phosphorescence emitting host or dopant is not particularly limited, but specific examples thereof include the followings.

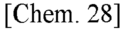

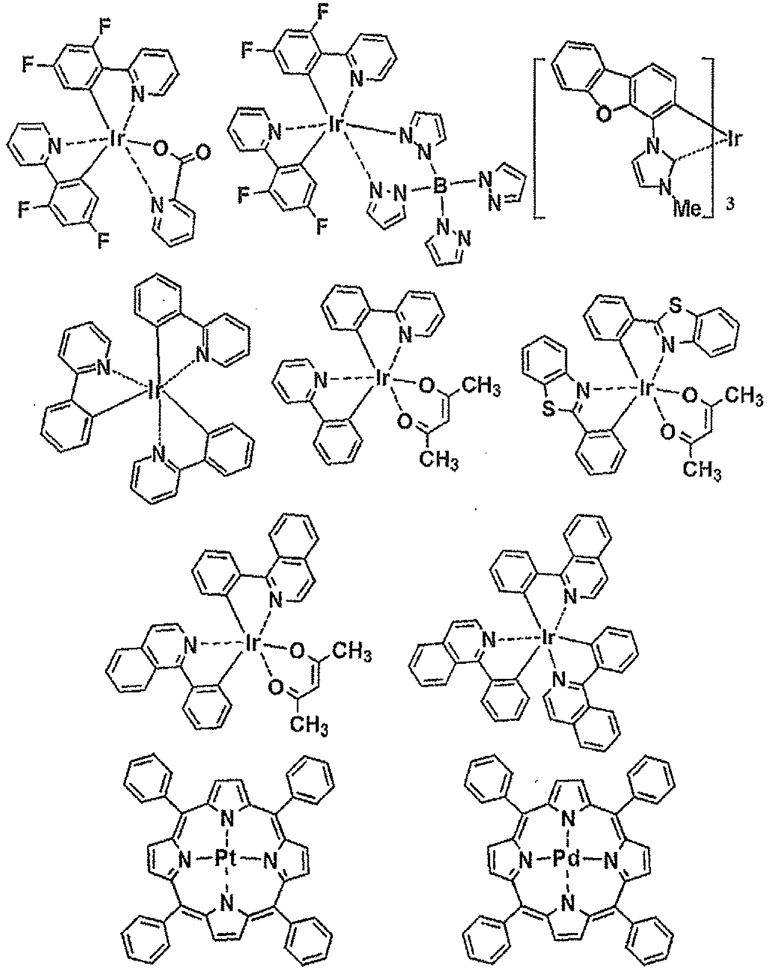

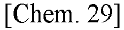

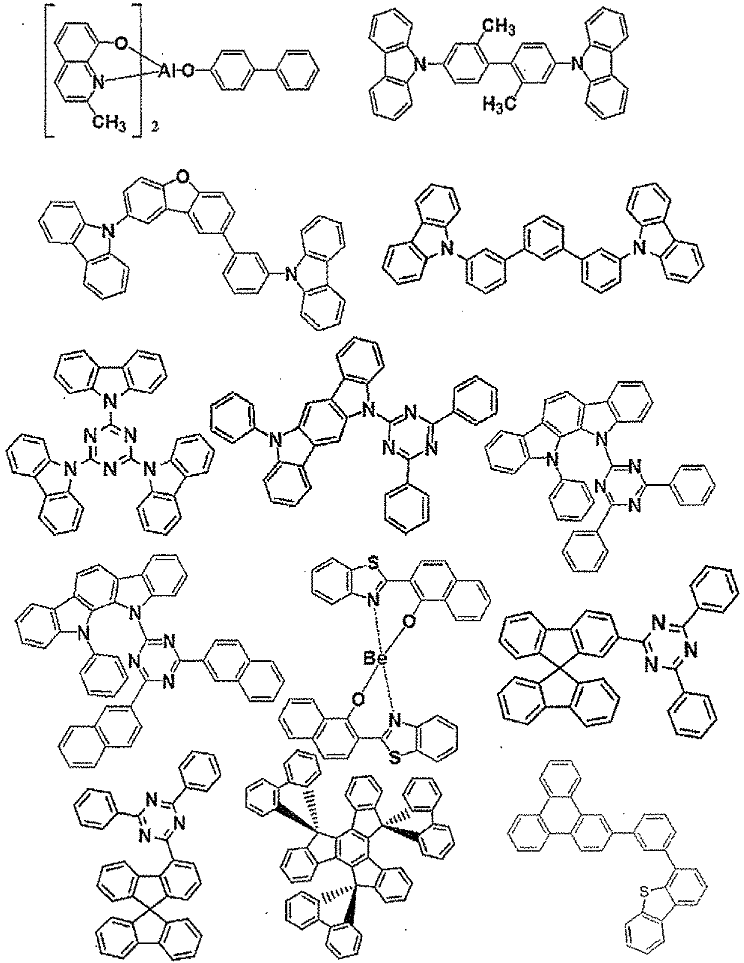

In the emissive layer, a thermally activated delayed fluorescent material may be contained. The thermally activated delayed fluorescent material is generally called a TADF material and is a material that reduces the energy gap between the energy level in a singlet excited state and the energy level in a triplet excited state to thereby promote reverse intersystem crossing from a triplet excited state to a singlet excited state and enhance the singlet exciton production probability. The thermally activated delayed fluorescent material may be a material that exhibits thermally activated delayed fluorescence by a single material or may be a material that exhibits thermally activated delayed fluorescence by a plurality of materials. The thermally activated delayed fluorescent material may be a single material or a plurality of materials, and a known material can be used. Specifically, the material includes, for example, a benzonitrile derivative, a triazine derivative, a disulfoxide derivative, a carbazole derivative, an indolocarbazole derivative, a dihydrophenazine derivative, a thiazole derivative, and an oxadiazole derivative.

The fluoranthene derivative of the present invention also has high light emitting performance and therefore, can be used as an emissive material. The fluoranthene derivative of the present invention exhibits intense luminescence in the blue to green region (region of 400 to 600 nm) and therefore, can be suitably used as blue and green emissive materials. The fluoranthene derivative of the present invention has a high fluorescence quantum yield and therefore, is suitably used as a fluorescent dopant material. In addition, since the fluoranthene skeleton has a high triplet energy level, the fluoranthene derivative can be suitably used also as a phosphorescent host and in particular, can be suitably used as a green phosphorescent host or a red phosphorescent host.

### (Electron Transporting Layer)

In the present invention, the electron transporting layer is a layer present between the cathode and the emissive layer. The electron transporting layer may be a single layer or a plurality of layers and may or may not be in contact with the cathode or the emissive layer. It is desired for the electron transporting layer, for example, to have high electron injection efficiency from the cathode, to efficiently transport the injected electron, and to provide high electron injection efficiency for light emission. Accordingly, the electron transporting layer preferably includes a substance having large electron affinity, high electron mobility and excellent stability and hardly allowing an impurity working out to a trap to be generated at the time of production and during use. However, considering the transportation balance between a hole and an electron, as long as the electron transporting layer fulfills mainly the role of efficiently blocking a hole from the anode from flowing to the cathode side without undergoing recombination, even if the electron transporting layer is constituted by a material having not so high electron transporting ability, the effect of enhancing the luminance efficiency becomes equivalent to that when the layer is constituted by a material having high electron transporting ability. For this reason, the electron transporting layer in the present invention encompasses, as having the same meaning, also a hole blocking layer capable of efficiently blocking the transfer of a hole.

Examples of the electron transporting material used in the electron transporting layer include a fused polycyclic aromatic derivative such as naphthalene and anthracene, a styryl-based aromatic ring derivative typified by 4,4'-bis(diphenylethenyl)biphenyl, a quinone derivative such as anthraquinone and diphenoquinone, a phosphorus oxide derivative, and various metal complexes, e.g., a quinolinol complex such as tris(8-quinolinolato)aluminum(III), a benzoquinolinol complex, a hydroxyazole complex, an azomethine complex, a tropolone metal complex, and a flavonol metal complex. For the reason that the driving voltage is reduced and a highly efficient luminescence is obtained, it is preferable to use a compound including an element selected from carbon, hydrogen, nitrogen, oxygen, silicon and phosphorus and having an aromatic heterocyclic structure containing an electron-accepting nitrogen.

The compound having an aromatic heterocyclic structure containing an electron-accepting nitrogen includes, for example, a pyridine ring, a pyrazine ring, a pyrimidine ring, a quinoline ring, a quinoxaline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

As the compound having an aromatic heterocyclic ring containing an electron-accepting nitrogen, preferable compounds include, for example, a benzimidazole derivative, a benzoxazole derivative, a benzothiazole derivative, an oxadiazole derivative, a thiadiazole derivative, a triazole derivative, a pyrazine derivative, a phenanthroline derivative, a quinoxaline derivative, a quinoline derivative, a benzoquinoline derivative, an oligopyridine derivative such as bipyridine and terpyridine, a quinoxaline derivative, and a naphthyridine derivative. Among these, an imidazole derivative such as tris(N-phenylbenzimidazol-2-yl)benzene, an oxadiazole derivative such as 1,3-bis[(4-tert-butylphenyl)1,3,4-oxadiazolyl]phenylene, a triazole derivative such as N-naphthyl-2,5-diphenyl-1,3,4-triazole, a phenanthroline derivative such as bathocuproine and 1,3-bis(1,10-phenanthrolin-9-yl)benzene, a benzoquinoline derivative such as 2,2'-bis(benzo[h]quinolin-2-yl)-9,9'-spirobifluorene, a bipyridine derivative such as 2,5-bis(6'-(2',2"-bipyridyl))-1,1-dimethyl-3,4-diphenylsilole, a terpyridine derivative such as 1,3-bis(4'-(2,2':6'2"-terpyridinyl))benzene, and a naphthyridine derivative such as bis(1-naphthyl)-4-(1,8-naphthyridin-2-yl)phenylphosphine oxide are preferably used in view of electron transporting ability. It is more preferred that such a derivative has a fused polycyclic aromatic skeleton, because not only the glass transition temperature is enhanced but also the electron mobility is increased to provide a great effect of reducing the voltage of the light emitting device. Furthermore, in consideration of enhanced durable life of the device, facilitated synthesis and easy availability of raw material, the fused polycyclic aromatic skeleton is preferably an anthracene skeleton, a pyrene skeleton or a phenanthroline skeleton, among others. The electron transporting material described above may be used alone, but a mixture of two or more kinds of electron transporting materials may be used, or a mixture of one or more kinds of other electron transporting materials with the above-described electron transporting material may be used.

Preferable electron transporting materials are not particularly limited but, specifically, include the followings.

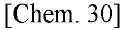

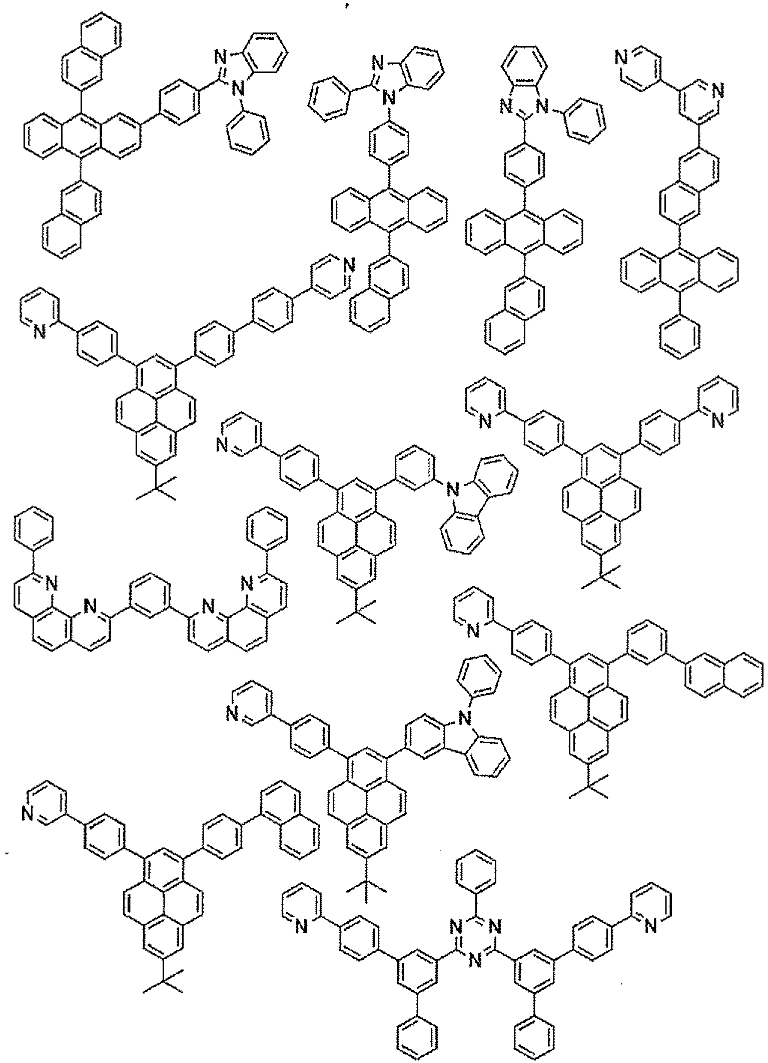

Other than these compounds, the electron transporting materials disclosed in WO 2004/63159, WO 2003/60956, Appl. Phys. Lett., 74, 865 (1999), Org. Electron., 4, 113 (2003), WO 2010/113743, and WO 2010/1817, etc. can also be used.

The fluoranthene derivative of the present invention also has high electron injection/transporting ability and therefore, is suitably used as an electron transporting material.

In the case where the fluoranthene derivative of the present invention is used as the electron transporting material, each of the fluoranthene derivatives need not be limited to use only one derivative, and a mixture of a plurality of the fluoranthene derivatives of the present invention may be used, or a mixture of one or more kinds of other electron transporting materials with the fluoranthene compound of the present invention may used as long as the effects of the present invention are not impaired. The electron transporting material that can be mixed is not particularly limited but includes a compound having a fused aryl ring, such as naphthalene, anthracene and pyrene, and a derivative thereof; a styryl-based aromatic ring derivative typified by 4,4'-bis(diphenylethenyl)biphenyl; a perylene derivative; a perinone derivative; a coumarin derivative; a naphthalimide derivative; a quinone derivative such as anthraquinone and diphenoquinone; a phosphorus oxide derivative; a carbazole derivative; a indole derivative; a quinolinol complex such as lithium quinolinol and tris(8-quinolinolato)aluminum(III); a hydroxyazole complex such as hydroxyphenyloxazole complex; an azomethine complex; a tropolone metal complex; and a flavonol metal complex.

Each of these electron transporting materials may be used alone, but a mixture of two or more of the electron transporting materials may be used, or a mixture of one or more of other electron transporting materials with the electron transporting material may be used. A donor material may also be contained. The donor material as used herein indicates a compound capable of improving the electron injection barrier, thereby facilitating electron injection into the electron transporting layer from the cathode or the electron injection layer, and furthermore, enhancing the electric conductivity of the electron transporting layer.

Preferable examples of the donor material for use in the present invention include an alkali metal, an inorganic salt containing an alkali metal, a complex of an alkali metal and an organic substance, an alkaline earth metal, an inorganic salt containing an alkaline earth metal, or a complex of an alkaline earth metal and an organic substance. Preferable kinds of the alkali metal and the alkaline earth metal include an alkali metal such as lithium, sodium and cesium, and an alkaline earth metal such as magnesium and calcium, each having a low work function and providing a great effect of enhancing the electron transporting ability.

In addition, for the reason that evaporation in vacuum is easy and handling is excellent, the donor material is preferably in the state of an inorganic salt or a complex with an organic substance, rather than a metal simple substance. Furthermore, in view of facilitated handling in the atmosphere and ease of control of the addition concentration, the donor material is more preferably in the state of a complex with an organic substance. Examples of the inorganic salt include an oxide such as LiO and Li₂O, a nitride, a fluoride such as LiF, NaF and KF, and a carbonate such as Li₂CO₃, Na₂CO₃, K₂CO₃, Rb₂CO₃ and Cs₂CO₃. Preferable examples of the alkali metal or alkaline earth metal include lithium, from the viewpoint that the raw material is inexpensive and the synthesis is easy. Preferable examples of the organic substance in the complex with an organic substance include quinolinol, benzoquinolinol, flavonol, hydroxyimidazopyridine, hydroxybenzazole, and hydroxytriazole. Among others, a complex of an alkali metal and an organic substance is preferred, a complex of lithium and an organic substance is more preferred, and lithium quinolinol is still more preferred. A mixture of two or more of these donor materials may be used.

The preferable doping concentration varies depending on the material or the film thickness in the doping region but, for example, when the donor material is an inorganic material such as alkali metal or alkaline earth metal, an electron transporting material and the donor material are preferably co-evaporated to form an electron transporting layer such that the evaporation rate ratio becomes from 10,000:1 to 2:1. The evaporation rate ratio is more preferably from 100:1 to 5:1, still more preferably from 100:1 to 10:1. In the case where the donor material is a complex of a metal and an organic substance, an electron transporting material and the donor material are preferably co-evaporated to form an electron transporting layer such that the evaporation rate ratio becomes from 100:1 to 1:100. The evaporation rate ratio is more preferably from 10:1 to 1:10, still more preferably from 7:3 to 3:7.

The electron transporting layer containing the fluoranthene derivative of the present invention doped with a donor material as described above may be used as a charge generation layer in a tandem structure-type device in which a plurality of light emitting devices are coupled. In particular, when an alkali metal or an alkaline earth metal is doped as a donor material, the layer can be suitably used as a charge generation layer.

The method of enhancing the electron transporting ability by doping a donor material into the electron transporting layer exerts its effect particularly when the thickness of a thin-film layer is large. The method is preferably used in particular when the total film thickness of the electron transporting layer and the emissive layer is 50 nm or more. The method includes, for example, a method of utilizing an interference effect for enhancing the luminance efficiency, where light extraction efficiency is enhanced by matching the phases of light emitted directly from the emissive layer and light reflected at the cathode. The optimum conditions thereof vary depending on the light emission wavelength, but the total film thickness of the electron transporting layer and the emissive layer is 50 nm or more and may be a large film thickness close to 100 nm in the case of long-wavelength emission such as red light.

The film thickness of the electron transporting layer, which is doped, may be a part or the whole of the electron transporting layer. In the case where a part of the electron transporting layer is doped, a doping region is preferably provided at least at the electron transporting layer/cathode interface, and only by doping in the vicinity of the cathode interface, the effect of reducing the voltage can be obtained. On the other hand, when the donor material is in direct contact with the emissive layer, an adverse effect of reducing the luminance efficiency may be produced, and in this case, it is preferable to provide a non-doping region at the emissive layer/electron transporting layer interface.

### (Electron Injection Layer)

In the present invention, an electron injection layer may be provided between the cathode and the electron transporting layer. The electron injection layer is generally inserted for the purpose of assisting injection of an electron from the cathode into the electron transporting layer, and in the case of inserting an electron injection layer, a compound having a heteroaryl ring structure containing an electron-accepting nitrogen may be used, or a layer containing the above-described donor material may be used. The fluoranthene derivative of the present invention may be contained in the electron injection layer.

An inorganic substance such as insulator or semiconductor may also be used in the electron injection layer. When such a material is used, advantageously, a short-circuit of the light emitting device can be effectively prevented and the electron injection property can be enhanced.

As the insulator, at least one metal compound selected from the group consisting of an alkali metal chalcogenide, an alkaline earth metal chalcogenide, a halide of an alkali metal, and a halide of an alkaline earth metal is preferably used. From the viewpoint that the electron injection property can be further enhanced, the electron injection layer is more preferably constituted by the alkali metal chalcogenide, etc.

Specifically, preferable examples of the alkali metal chalcogenide include Li₂O, Na₂S and Na₂Se; preferable examples of the alkaline earth metal chalcogenide include CaO, BaO, SrO, BeO, BaS and CaSe; preferable examples of the halide of an alkali metal include LiF, NaF, KF, LiCl, KCl and NaCl; and preferable examples of the halide of an alkaline earth metal include a fluoride such as CaF₂, BaF₂, SrF₂, MgF₂ and BeF₂, and a halide other than fluoride.

A complex of an organic substance and a metal is also suitably used. It is more preferable to use a complex of an organic substance and a metal in the electron injection layer, because the film thickness adjustment is facilitated. As for examples of this organic metal complex, preferable examples of the organic substance in the complex with an organic substance include quinolinol, benzoquinolinol, pyridylphenol, flavonol, hydroxyimidazopyridine, hydroxybenzazole, and hydroxytriazole. Among others, a complex of an alkali metal and an organic substance is preferred, a complex of lithium and an organic substance is more preferred, and lithium quinolinol is still more preferred.

### (Charge Generation Layer)

In the present invention, the charge generation layer is an intermediate layer present between the anode and the cathode in the above-described tandem structure-type device and is a layer for generating a hole and an electron by charge separation. The charge generation layer is generally formed of a p-type layer on the cathode side and an n-type layer on the anode side. These layers are desired to allow for effective charge separation and effective transport of the carriers generated.

In the p-type charge generation layer, a material used in the above-described hole injection layer or hole transporting layer can be used. For example, HAT-CN6, a benzidine derivative such as NPD and TBDB, materials called starburst arylamine, such as m-MTDATA and 1-TNATA, and a material having a skeleton represented by formula (11) or (12) may be suitably used.

In the n-type charge generation layer, a material used in the above-described electron injection layer or electron transporting layer can be used, and a compound having a heteroaryl ring structure containing an electron-accepting nitrogen may be used, or a layer containing the donor material described above may be used. A layer in which the donor material described above is doped into the fluoranthene derivative of the present invention may also be suitably used.

The method for forming each of the layers constituting the light emitting device includes a resistance heating evaporation method, an electron beam evaporation method, sputtering, a molecular stacking method, a coating method, etc. and is not particularly limited, but usually, in view of device characteristics, a resistance heating evaporation method or an electron beam evaporation method is preferred.

The thickness of the organic layer depends on the resistance value of the emissive substance and cannot be limited but is preferably from 1 to 1,000 nm. The film thickness of each of the emissive layer, the electron transporting layer and the hole transporting layer is preferably from 1 to 200 nm, more preferably from 5 nm to 100 nm.

The light emitting device of the present invention has a function capable of converting electric energy into light. Here, a DC current is mainly used as the electric energy, but a pulse current or an AC current can also be used. The current value and the voltage value are not particularly limited but considering the power consumption or life of the device, the values thereof should be selected so that a maximum luminance can be obtained at as low energy as possible.

The light emitting device of the present invention is suitably used, for example, as a display for effecting display by a matrix and/or segment system.

In the matrix system, display pixels are two-dimensionally arranged, e.g., in a lattice or mosaic pattern, and a character or an image is displayed by a set of pixels. The shape and size of the pixel are determined according to the intended use. For example, usually, in displaying an image and a character on a personal computer, a monitor or a television, a quadrangular pixel of 300 µm or less on a side is used, and in the case of a large-size display such as display panel, a pixel in the mm order on a side is used. In the case of a black and white display, it may be sufficient if pixels of the same color are arrayed, but in the case of a color display, pixels of red, green and blue are arrayed and displayed. In this case, typically, there are a delta type and a stripe type. The method for driving the matrix may be either a passive matrix driving method or an active matrix. The passive matrix driving is simple in structure, but considering the operation characteristics, the active matrix is sometimes more excellent, and therefore, it is necessary to properly use the method according to the intended use.

The segment system as used in the present invention is a system in which a pattern is formed to display predetermined information and a region determined by the arrangement of this pattern is caused to emit light. The system includes, for example, a time or temperature display in a digital watch or a thermometer, an operating state display in an audio device, an electromagnetic cooker, etc., and a panel display of an automobile. The matrix display and the segment display may be present together in the same panel.

The light emitting device of the present invention is also preferably used as a backlight of various instruments. The backlight is used mainly for the purpose of improving the visibility of a non-self-luminous display device and is used in a liquid crystal display device, a clock, an audio device, an automobile panel, a display board, a sign, etc. In particular, the light emitting device of the present invention is preferably used as a backlight for a liquid crystal display device, among others, for a personal computer of which thickness reduction is being studied, and can provide a backlight more reduced in the thickness and weight than conventional products.

### EXAMPLES

The present invention is described below by referring to Examples, but the present invention is not limited to these Examples.

### Synthesis Example 1:

### Synthesis of Compound [1]:

26.0 g of bromofluoranthene, 35.2 g of bis(pinacolato)diboron, 27.2 g of potassium acetate and 462 mL of dimethylformamide were mixed and purged with nitrogen, and to this mixed solution, 0.75 g of a [1,1'-bis(diphenylphosphino)ferrocene]palladium(II) dichloride-dichloromethane complex was added. The resulting mixture was heated at 100°C and after 1 hour, cooled to room temperature, and 250 mL of ethyl acetate, 250 mL of toluene and 250 mL of water were then added, followed by liquid separation. The aqueous layer was extracted with 200 mL of ethyl acetate and 200 mL of toluene, then combined with the organic layer, and washed with 500 mL of water three times. The organic layer was dried over magnesium sulfate, and the solvent was removed by distillation. The residue was purified by silica gel column chromatography, and the eluate was evaporated and vacuum-dried to obtain 16.4 g of Intermediate A.

Thereafter, 10.7 g of Intermediate A, 7.0 g of 1-bromo-3,5-chlorobenzene, 155 mL of dimethoxyethane and 45 ml of an aqueous 1.5 M sodium carbonate solution were mixed and purged with nitrogen, and to this mixed solution, 218 mg of bis(triphenylphosphine)palladium dichloride was added. The resulting mixture was heated under reflux and after 1 hour, cooled to room temperature, and 155 ml of water was added. The precipitate was filtrated and dried by a vacuum drier, and the filtrated product was dissolved in toluene and after adding 3.0 g of activated carbon, filtrated on a silica pad. The solvent of the filtrate was then removed by distillation, and the residue was then recrystallized from 120 mL of butyl acetate, filtrated and then vacuum-dried to obtain 8.6 g of Intermediate B as a yellowish green solid.

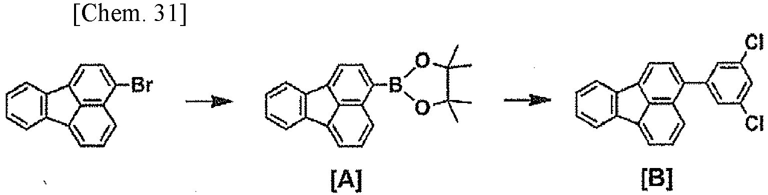

Subsequently, 3.81 g of 4-(2-pyridyl)phenylboronic acid, 2.77 g of Intermediate B, 184 mg of bis(dibenzylideneacetone)palladium(0), 235 mg of tricyclohexylphosphine-tetrafluoroborane, 8.5 g of potassium phosphate, 80 mL of dioxane, and 20 mL of water were mixed and purged with nitrogen. The resulting mixture was heated under reflux and after 12 hours, cooled to room temperature, and then the solid precipitated was filtrated. The solid was washed with 50 mL of water three times, and the filtrated product was dissolved in 100 mL of xylene and after adding 2.0 g of activated carbon and 2.5 g of "QuadraSil" (registered trademark), filtrated through Celite. The solvent of the filtrate was removed by distillation and after adding methanol, the solid precipitated was filtrated and vacuum-dried to obtain 3.1 g of Intermediate [1] as a yellowish green solid.

The results of ¹H-NMR analysis of the obtained yellowish green solid are as follows, and it was confirmed that the yellowish green solid obtained above is Compound [1].

### Compound [1]:

¹H-NMR (CDCl₃ (d=ppm)) δ 7.40-7.43 (m, 3H), 7.64-7.16 (m, 23H), 8.72 (d, 2H).

Here, Compound [1] was subjected to sublimation purification at about 320°C under a pressure of 1×10⁻³ Pa by using an oil diffusion pump and then used as a light emitting device material.

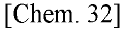

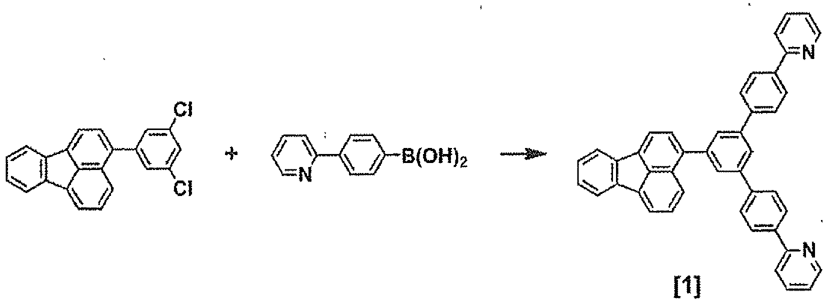

### Synthesis Example 2:

### Synthesis of Compound [2]:

Next, 3.85 g of 4-(3-pyridyl)phenylboronic acid, 2.80 g of Intermediate B, 185 mg of bis(dibenzylideneacetone)palladium(0), 143 mg of tricyclohexylphosphine-tetrafluoroborane, 8.6 g of potassium phosphate, 100 mL of dioxane, and 20 mL of water were mixed and purged with nitrogen. The resulting mixture was heated under reflux and after 1 hour, cooled to room temperature, and 20 mL of water was added. The solid precipitated was filtrated, and the obtained solid was washed with 70 mL of water three times. The filtrated product was dissolved with heating in 100 mL of pyridine, and 1.0 g of activated carbon and 1.5 g of "QuadraSil" (registered trademark) were added. The resulting mixture was stirred at 100°C for 30 minutes and then filtrated through Celite at room temperature, and 50 mL of the solvent of the filtrate was removed by distillation. The residue was cooled in a refrigerator at -20°C for 2 hours, and the solid precipitated was filtrated. The solid was recrystallized from pyridine/methanol, and the solid precipitated was filtrated. The solid was recrystallized from 40 mL of o-xylene, and the obtained solid was filtrated and vacuum-dried to obtain 4.2 g of Compound [2] as a yellowish green solid.

The results of ¹H-NMR analysis of the obtained yellowish green solid are as follows, and it was confirmed that the yellowish green solid obtained above is Compound [2].

### Compound [2]:

¹H-NMR (CDCl₃ (d=ppm)) δ 7.37-7.43 (m, 3H), 7.64-8.06 (m, 21H), 8.62 (dd, 2H), 8.93 (d, 2H).

Here, Compound [2] was subjected to sublimation purification at about 330°C under a pressure of 1×10⁻³ Pa by using an oil diffusion pump and then used as a light emitting device material.

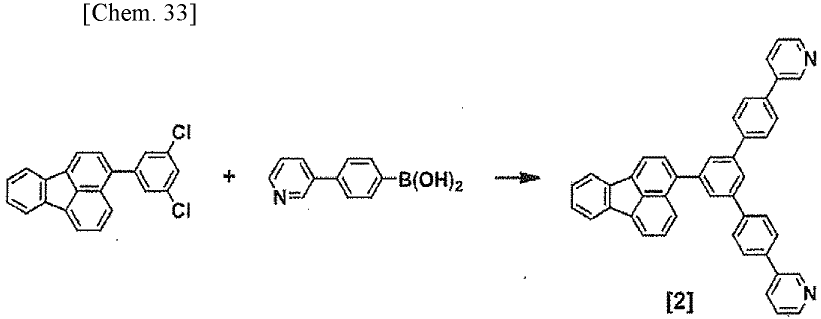

### Synthesis Example 3:

### Synthesis of Compound [3]:

Next, 6.88 g of 4-(4-pyridyl)phenylboronic acid, 5.00 g of Intermediate B, 331 mg of bis(dibenzylideneacetone)palladium(0), 255 mg of tricyclohexylphosphine-tetrafluoroborane, 15.3 g of potassium phosphate, 200 mL of dioxane, and 40 mL of water were mixed and purged with nitrogen. The resulting mixture was heated under reflux and after 5 hours, 1.50 g of 4-(4-pyridyl)phenylboronic acid was added. The mixture was further stirred with heating under reflux for 2 hours and cooled to room temperature, and 200 mL of water was added. The solid precipitated was filtrated, and the obtained solid was washed with 100 mL of water three times. The filtrated product was dissolved with heating in 200 mL of pyridine, and 5.0 g of activated carbon and 3.0 g of "QuadraSil" (registered trademark) were added. The resulting mixture was stirred at 100°C for 30 minutes and then filtrated through Celite at room temperature, and 150 mL of the solvent of the filtrate was removed by distillation. After adding 100 mL of methanol, the mixture was cooled in a refrigerator at -20°C for 18 hours, and the solid precipitated was filtrated, as a result, 7.3 g of a solid was obtained. The solid was recrystallized from 450 mL of toluene, and the solid precipitated was filtrated, as a result, 5.8 g of a solid was obtained. Furthermore, this solid was recrystallized from 150 mL of o-xylene, and the obtained solid was filtrated and vacuum-dried to obtain 5.2 g of Compound [3] as a yellowish green solid.

The results of ¹H-NMR analysis of the obtained yellowish green solid are as follows, and it was confirmed that the yellowish green solid obtained above is Compound [3].

### Compound [3]:

¹H-NMR (CDCl₃ (d=ppm)) δ 7.40-7.43 (m, 3H), 7.56-8.05 (m, 21H), 8.69 (dd, 4H).

Here, Compound [3] was subjected to sublimation purification at about 340°C under a pressure of 1×10⁻³ Pa by using an oil diffusion pump and then used as a light emitting device material.

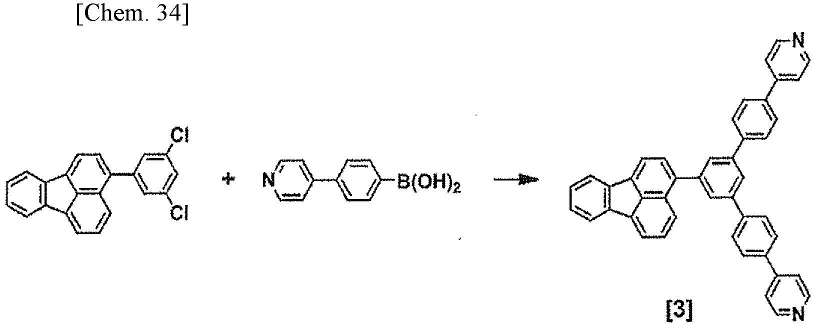

### Synthesis Example 4:

### Synthesis of Compound [4]:

Next, 10.00 g of 4-(2-pyridyl)phenylboronic acid, 10.3 g of 1-dibromo-3,5-dichlorobenzene, 251 mL of dimethoxyethane, and 67 ml of an aqueous 1.5 M sodium carbonate solution were mixed and purged with nitrogen, and to this mixed solution, 321 mg of bis(triphenylphosphine)palladium dichloride was added. The resulting mixture was heated under reflux for 3 hours and cooled to room temperature, and 400 ml of water was added. The precipitate was filtrated, dissolved with heating in 200 mL of toluene, and dried over sodium sulfate, and 2.0 g of activated carbon and 2.0 g of "QuadraSil" (registered trademark) were added. The resulting mixture was stirred and filtrated through Celite, and the solvent of the filtrate was removed by distillation. The residue was dissolved with heating in 30 mL of butyl acetate, and 200 mL of methanol was added. The solid precipitated was filtrated and vacuum-dried to obtain 9.8 g of Intermediate C as a yellowish green solid.

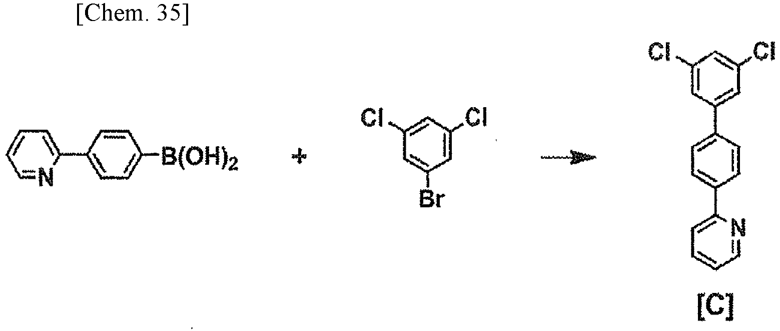

Thereafter, 2.8 g of Intermediate C, 5.0 g of 3-fluorantheneboronic acid, 92 mL of dioxane and 22 mL of an aqueous 1.27 M potassium phosphate solution were mixed and purged with nitrogen, and to this mixed solution, 106 mg of bis(dibenzylideneacetone)palladium(0) and 82 mg of tricyclohexylphosphine-tetrafluoroborane were added. The resulting mixture was heated under reflux and after 2 hours, 106 mg of bis(dibenzylideneacetone)palladium(0) and 82 mg of tricyclohexylphosphine-tetrafluoroborane were added. The resulting mixture was heated under reflux and after 1 hour, 106 mg of bis(dibenzylideneacetone)palladium(0) and 82 mg of tricyclohexylphosphine-tetrafluoroborane were further added. The resulting mixture was heated under reflux and cooled to room temperature, and the solid precipitated was filtrated and washed with water and methanol. The solid was dissolved with heating in 100 mL of o-xylene, and 1.0 g of activated carbon and 1.0 g of QuadraSil (registered trademark) were added. The resulting mixture was stirred with heating under reflux and after adding 200 mL of toluene, filtrated through Celite. The solvent of the filtrate was removed by distillation, and the residue was purified by silica gel column chromatography. The eluate was evaporated, and after adding methanol to the eluate from which the solvent was removed by distillation, the mixture was filtrated and vacuum-dried to obtain 4.7 g of Compound [4] as a yellowish green solid.

The results of ¹H-NMR analysis of the obtained yellowish green solid are as follows, and it was confirmed that the yellowish green solid obtained above is Compound [4].

### Compound [4]:

¹H-NMR (CDCl₃ (d=ppm)) δ 7.39-7.42 (m, 3H), 7.64-8.15 (m, 25H), 7.72 (d, 1H).

Here, Compound [4] was subjected to sublimation purification at about 350°C under a pressure of 1×10⁻³ Pa by using an oil diffusion pump and then used as a light emitting device material.

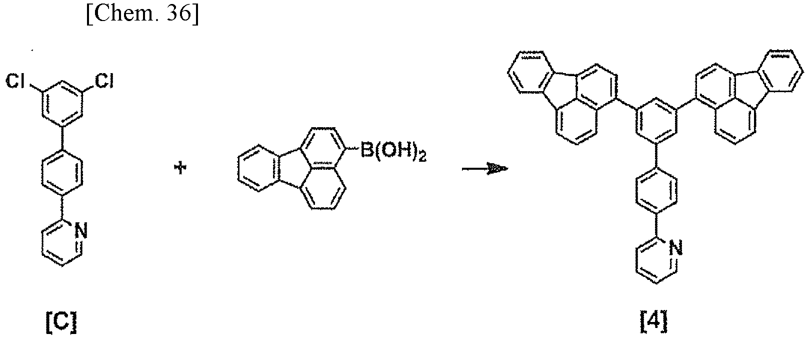

### Synthesis Example 5:

### Synthesis of Compound [5]:

Next, 30.0 g of Intermediate D, 11.8 g of 1,3-dibromo-5-chlorobenzene, 435 mL of dimethoxyethane and 87 ml of an aqueous 1.5 M sodium carbonate solution were mixed and purged with nitrogen, and to this mixed solution, 306 mg of bis(triphenylphosphine)palladium dichloride was added. The resulting mixture was heated under reflux and after 5.5 hours, cooled to room temperature, and 435 ml of water was added. The precipitate was filtrated and washed with water and methanol, and o-xylene and toluene were added. The remaining water and methanol in the system were removed by distillation, and 2.5 g of activated carbon and 2.5 g of QuadraSil (registered trademark) were added. The resulting mixture was stirred with heating under reflux for 30 minutes and filtrated on a silica pad, and the solvent of the filtrate was then removed by distillation. The residue was dissolved with heating in 200 mL of o-xylene and after adding 400 mL of butyl acetate, the solid precipitated was filtrated and vacuum-dried to obtain 19.6 g of Intermediate E as a yellowish green solid.

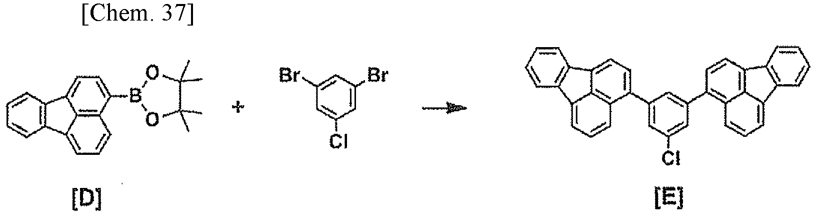

Thereafter, 4.0 g of Intermediate E, 2.7 g of 4-(8-quinolinyl)phenylboronic acid ester, 78 mL of dioxane and 10 mL of an aqueous 1.27 M potassium phosphate solution were mixed and purged with nitrogen, and to this mixed solution, 90 mg of bis(dibenzylideneacetone)palladium(0) and 69 mg of tricyclohexylphosphine-tetrafluoroborane were added. The resulting mixture was heated under reflux, and while confirming the progress of reaction, 360 mg of bis(dibenzylideneacetone)palladium(0) and 276 mg of tricyclohexylphosphine-tetrafluoroborane were added with continuous heating and stirring. The resulting solution was cooled to room temperature, and the solid precipitated was filtrated, washed with water and methanol, and dried. The solid was dissolved with heating in 49 mL of o-xylene, and 1.5 g of activated carbon and 1.5 g of QuadraSil (registered trademark) were added. The resulting mixture was stirred with heating under reflux and after adding 99 mL of o-xylene, filtrated through Celite. The solvent of the filtrate was removed by distillation and after recrystallization from o-xylene, the solid obtained was filtrated and vacuum-dried to obtain 3.8 g of Compound [5] as a yellowish green solid.

The results of ¹H-NMR analysis of the obtained yellowish green solid are as follows, and it was confirmed that the yellowish green solid obtained above is Compound [5].

### Compound [5]:

¹H-NMR (CDCl₃ (d=ppm)) δ 7.39-7.46 (m, 5H), 7.61-7.24 (m, 25H), 8.90 (s, 1H).

Here, Compound [5] was subjected to sublimation purification at about 360°C under a pressure of 1×10⁻³ Pa by using an oil diffusion pump and then used as a light emitting device material.

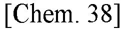

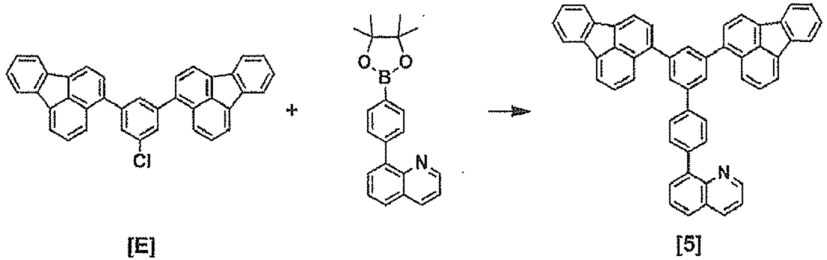

### Example 1:

A glass substrate having deposited thereon an ITO transparent conductive film of 165 nm (produced by GEOMATEC Co., Ltd., 11 Ω/square, sputtered product) was cut into 38×46 mm and subjected to etching. The obtained substrate was ultrasonically cleaned with "SEMICOCLEAN 56" (trade name, produced by Furuuchi Chemical Corporation) for 15 minutes and then washed with ultrapure water. This substrate was treated with UV-ozone for 1 hour immediately before manufacture of a device and placed in a vacuum evaporation apparatus, and the inside of the apparatus was evacuated until reaching a degree of vacuum of 5×10⁻⁴ Pa or less. By a resistance heating method, first, HAT-CN₆ as a hole injection layer and HT-1 as a hole transporting layer were evaporated to a thickness of 5 nm and 50 nm, respectively. Next, as an emissive layer, Host Material H-1 and Dopant Material D-1 were evaporated to a thickness of 20 nm such that the doping concentration became 5 wt%. Subsequently, as an electron transporting layer, Compound [1] was laminated by evaporation to a thickness of 35 nm. Thereafter, lithium fluoride was evaporated to a thickness of 0.5 nm, and aluminum was then evaporated to a thickness of 1,000 nm to form a cathode, whereby a 5×5 mm square device was manufactured. The film thickness as used herein is an indicated value of a crystal oscillation film thickness monitor. The properties of the light emitting device at 1,000 cd/m² were a driving voltage of 4.6 V and an external quantum efficiency of 4.4%. In addition, when the initial luminance was set to 1,000 cd/m² and the light emitting device was driven at a constant current, the time in which the luminance was decreased by 20% was 1,500 hours. Here, Compound [1], HAT-CN₆, HT-1, H-1 and D-1 are the compounds illustrated below.

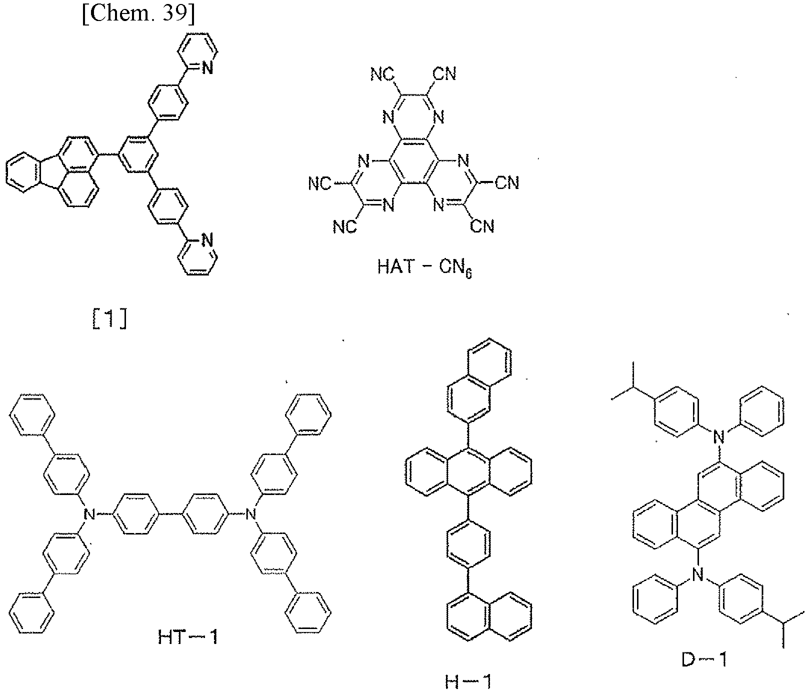

### Examples 2 to 28, and Reference Examples 29 to 31:

Light emitting devices were manufactured and evaluated in the same manner as in Example 1 except that the compound shown in Table 1 was used in the electron transporting layer. The results are shown in Table 1. Here, Compounds [6] to [31] are the compounds illustrated below, wherein Compounds [29] to [31] do not fall under the scope of the present invention.

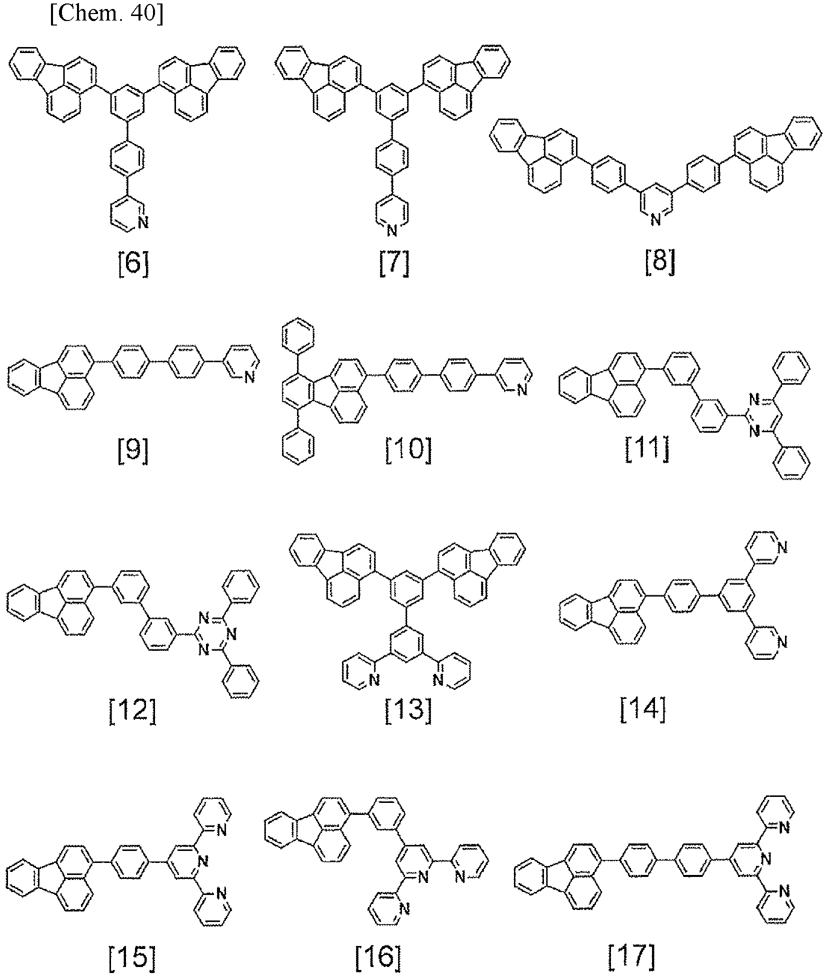

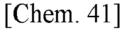

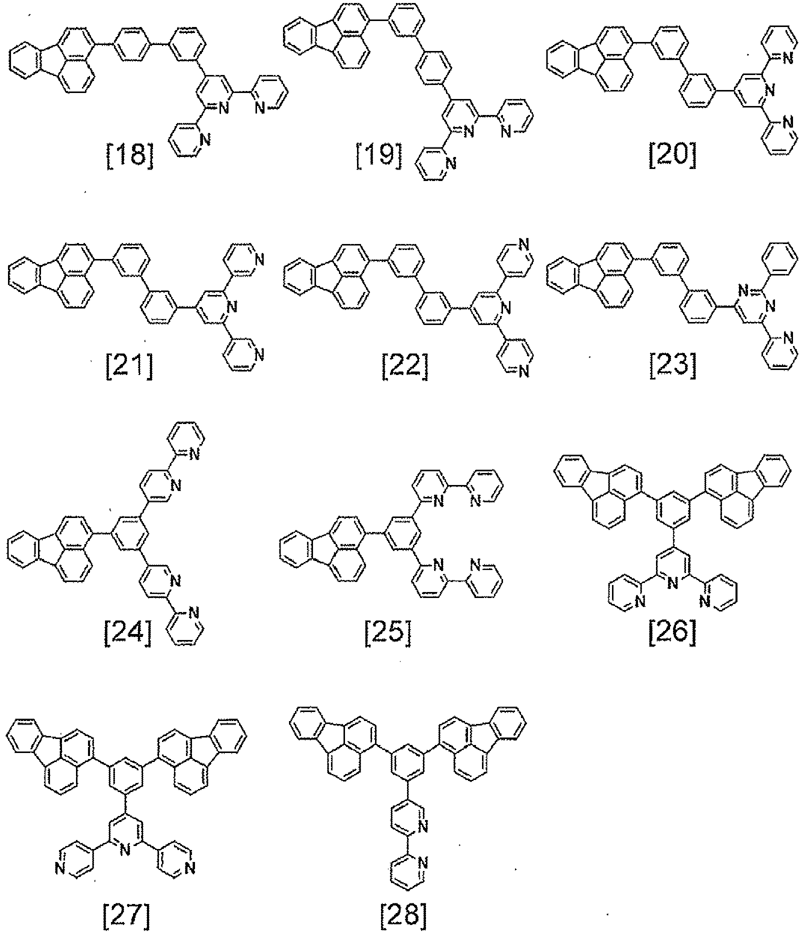

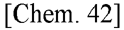

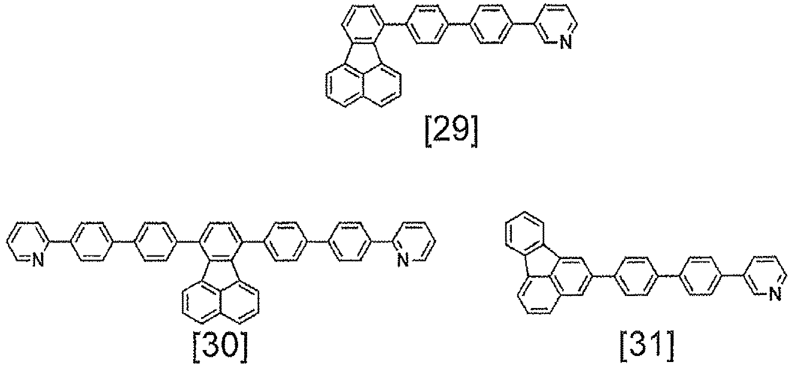

### Comparative Examples 1 to 3:

Light emitting devices were manufactured and evaluated in the same manner as in Example 1 except for using the compound shown in Table 1 in the electron transporting layer. The results are shown in Table 1. Here, E-1 to E-3 are the compounds illustrated below.

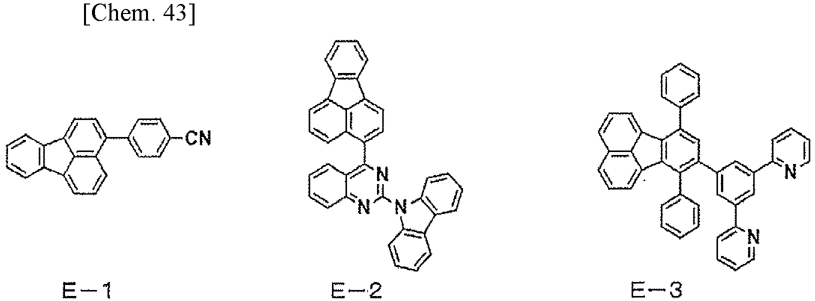

### Example 32:

A glass substrate having deposited thereon an ITO transparent conductive film of 165 nm (produced by GEOMATEC Co., Ltd., 11 Ω/square, sputtered product) was cut into 38×46 mm and subjected to etching. The obtained substrate was ultrasonically cleaned with "SEMICOCLEAN 56" (trade name, produced by Furuuchi Chemical Corporation) for 15 minutes and then washed with ultrapure water. This substrate was treated with UV-ozone for 1 hour immediately before manufacture of a device and placed in a vacuum evaporation apparatus, and the inside of the apparatus was evacuated until reaching a degree of vacuum of 5×10⁻⁴ Pa or less. By a resistance heating method, first, HAT-CN₆ as a hole injection layer and HT-1 as a hole transporting layer were evaporated to a thickness of 5 nm and 50 nm, respectively. Next, as an emissive layer, Host Material H-1 and Dopant Material D-1 were evaporated to a thickness of 20 nm such that the doping concentration became 5 wt%. Subsequently, as a first electron transporting layer, compound [1] was laminated by evaporation to a thickness of 25 nm. Furthermore, as a second electron transporting layer, Compound [1] used as an electron transporting material and lithium used as a donor material were laminated to a thickness of 10 nm such that the evaporation rate ratio between Compound [1] and lithium became 20:1. Thereafter, lithium fluoride was evaporated to a thickness of 0.5 nm, and aluminum was then evaporated to a thickness of 1,000 nm to form a cathode, whereby a 5×5 mm square device was manufactured. The properties of the light emitting device at 1,000 cd/m² were a driving voltage of 3.8 V and an external quantum efficiency of 5.3%. In addition, when the initial luminance was set to 1,000 cd/m² and the light emitting device was driven at a constant current, the time in which the luminance was decreased by 20% was 1,500 hours.

### Examples 33 to 39:

Light emitting devices were manufactured and evaluated in the same manner as in Example 32 except that the compound shown in Table 2 was used in the electron transporting layer. The results are shown in Table 2.

### Comparative Examples 4 to 6:

Light emitting devices were manufactured and evaluated in the same manner as in Example 32 except that the compound shown in Table 2 was used in the electron transporting layer. The results are shown in Table 2.

### Example 40:

A glass substrate having deposited thereon an ITO transparent conductive film of 165 nm (produced by GEOMATEC Co., Ltd., 11 Ω/square, sputtered product) was cut into 38×46 mm and subjected to etching. The obtained substrate was ultrasonically cleaned with "SEMICOCLEAN 56" (trade name, produced by Furuuchi Chemical Corporation) for 15 minutes and then washed with ultrapure water. This substrate was treated with UV-ozone for 1 hour immediately before manufacture of a device and placed in a vacuum evaporation apparatus, and the inside of the apparatus was evacuated until reaching a degree of vacuum of 5×10⁻⁴ Pa or less. By a resistance heating method, first, HAT-CN₆ as a hole injection layer and HT-1 as a hole transporting layer were evaporated to a thickness of 5 nm and 50 nm, respectively. Next, as an emissive layer, Host Material H-1 and Dopant Material D-1 were evaporated to a thickness of 20 nm such that the doping concentration became 5 wt%. Furthermore, as an electron transporting layer, Compound [1] used as an electron transporting material and 2E-1 used as a donor material were laminated to a thickness of 35 nm such that the evaporation rate ratio between Compound [1] and 2E-1 became 1:1. This electron transporting layer is denoted by Second Electron Transporting Layer in Table 3. Thereafter, lithium fluoride was evaporated to a thickness of 0.5 nm, and magnesium and silver were then co-evaporated to a thickness of 1,000 nm to form a cathode, whereby a 5×5 mm square device was manufactured. The properties of the light emitting device at 1,000 cd/m² were a driving voltage of 4.2 V and an external quantum efficiency of 5.5%. In addition, when the initial luminance was set to 1,000 cd/m² and the light emitting device was driven at a constant current, the time in which the luminance was decreased by 20% was 3,500 hours.

### Examples 41 to 44:

Light emitting devices were manufactured and evaluated in the same manner as in Example 40 except that the compound shown in Table 3 was used as the electron transporting layer and the donor material. The results are shown in Table 3. 2E-1 is the compound illustrated below.

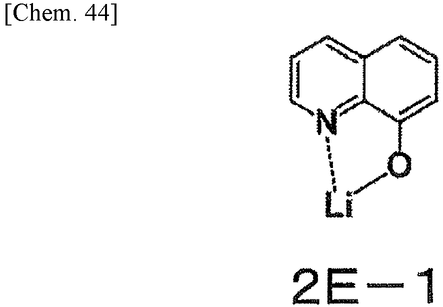

### Comparative Examples 7 to 9:

Light emitting devices were manufactured and evaluated in the same manner as in Example 40 except that the compound shown in Table 3 was used as the donor material in the electron transporting layer. The results are shown in Table 3.

### Example 45:

A glass substrate having deposited thereon an ITO transparent conductive film of 165 nm (produced by GEOMATEC Co., Ltd., 11 Ω/square, sputtered product) was cut into 38×46 mm and subjected to etching. The obtained substrate was ultrasonically cleaned with "SEMICOCLEAN 56" (trade name, produced by Furuuchi Chemical Corporation) for 15 minutes and then washed with ultrapure water. This substrate was treated with UV-ozone for 1 hour immediately before manufacture of a device and placed in a vacuum evaporation apparatus, and the inside of the apparatus was evacuated until reaching a degree of vacuum of 5×10⁻⁴ Pa or less. By a resistance heating method, first, HAT-CN₆ as a hole injection layer and HT-1 as a hole transporting layer were evaporated to a thickness of 5 nm and 50 nm, respectively. This hole transporting layer is denoted by First Hole Transporting Layer in Table 4. Next, as an emissive layer, Host Material H-2 and Dopant Material D-2 were evaporated to a thickness of 20 nm such that the doping concentration became 10 wt%. Subsequently, as an electron transporting layer, Compound [3] was laminated by evaporation to a thickness of 35 nm. Thereafter, lithium fluoride was evaporated to a thickness of 0.5 nm, and aluminum was then evaporated to a thickness of 1,000 nm to form a cathode, whereby a 5×5 mm square device was manufactured. The film thickness as used herein is an indicated value of a crystal oscillation film thickness monitor. The properties of the light emitting device at 4,000 cd/m² were a driving voltage of 3.9 V and an external quantum efficiency of 10.1%. In addition, when the initial luminance was set to 4,000 cd/m² and the light emitting device was driven at a constant current, the time in which the luminance was decreased by 20% was 1,500 hours. Here, H-2 and D-2 are the compounds illustrated below.

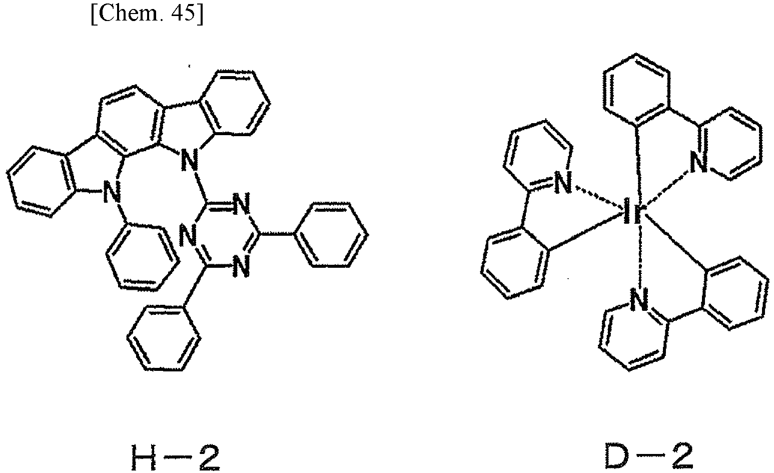

### Comparative Example 10:

A light emitting device was manufactured and evaluated in the same manner as in Example 45 except that the compound shown in Table 4 was used in the electron transporting layer. The results are shown in Table 4.

### Example 46:

A glass substrate having deposited thereon an ITO transparent conductive film of 165 nm (produced by GEOMATEC Co., Ltd., 11 Ω/square, sputtered product) was cut into 38×46 mm and subjected to etching. The obtained substrate was ultrasonically cleaned with "SEMICOCLEAN 56" (trade name, produced by Furuuchi Chemical Corporation) for 15 minutes and then washed with ultrapure water. This substrate was treated with UV-ozone for 1 hour immediately before manufacture of a device and placed in a vacuum evaporation apparatus, and the inside of the apparatus was evacuated until reaching a degree of vacuum of 5×10⁻⁴ Pa or less. By a resistance heating method, first, HAT-CN₆ as a hole injection layer and HT-1 as a first hole transporting layer were evaporated to a thickness of 5 nm and 40 nm, respectively. Furthermore, HT-2 as a second hole transporting layer was evaporated to a thickness of 10 nm. Next, as an emissive layer, Host Material H-2 and Dopant Material D-2 were evaporated to a thickness of 20 nm such that the doping concentration became 10 wt%. Subsequently, as an electron transporting layer, Compound [3] was laminated by evaporation to a thickness of 35 nm. Thereafter, lithium fluoride was evaporated to a thickness of 0.5 nm, and aluminum was then evaporated to a thickness of 1,000 nm to form a cathode, whereby a 5×5 mm square device was manufactured. The film thickness as used herein is an indicated value of a crystal oscillation film thickness monitor. The properties of the light emitting device at 4,000 cd/m² were a driving voltage of 3.9 V and an external quantum efficiency of 13.8%. In addition, when the initial luminance was set to 4,000 cd/m² and the light emitting device was driven at a constant current, the time in which the luminance was decreased by 20% was 1,900 hours. Here, HT-2 is the compound illustrated below.

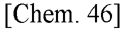

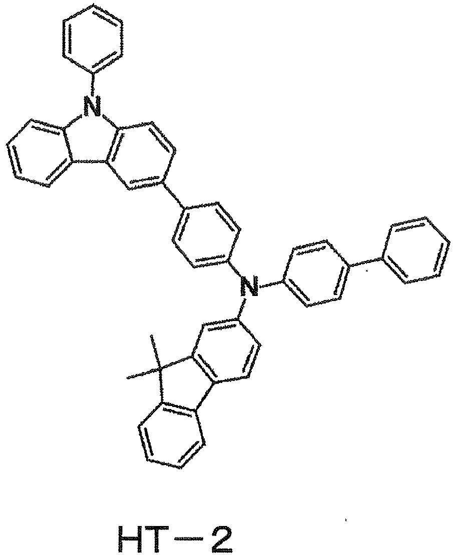

### Examples 47 and 48:

Light emitting devices were manufactured and evaluated in the same manner as in Example 46 except that the compounds shown in Table 4 were used in the second hole transporting layer and the electron transporting layer. The results are shown in Table 4. Here, HT-3 and HT-4 are the compounds illustrated below.

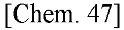

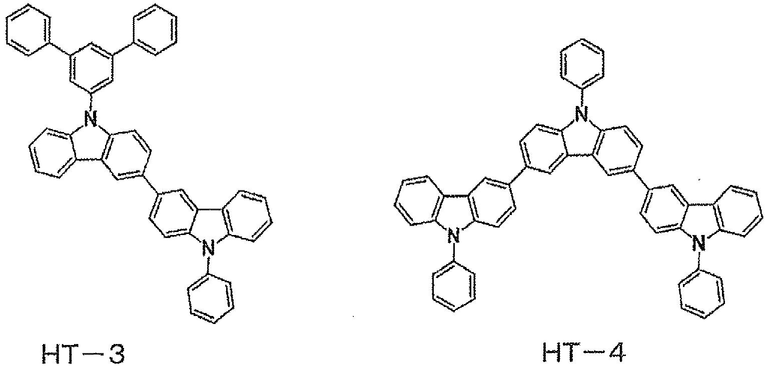

### Comparative Examples 11 to 13:

Light emitting devices were manufactured and evaluated in the same manner as in Example 46 except that the compounds shown in Table 4 were used in the second hole transporting layer and the electron transporting layer. The results are shown in Table 4.

**Table 1**

| | Emissive Material | | | Electron Transporting Layer | External Quantum Efficiency (%) | Voltage (V) | Driving Durability (h) |
|---|---|---|---|---|---|---|---|
| | Host Material | Dopant Material | Emission Color | Compound | | | |
| Example 1 | | | blue | [1] | 4.4 | 4.6 | 1500 |
| Example 2 | | | blue | [2] | 4.4 | 4.6 | 1600 |
| Example 3 | | | blue | [3] | 4.3 | 4.7 | 1700 |
| Example 4 | | | blue | [4] | 4.6 | 4.3 | 1200 |
| Example 5 | | | blue | [5] | 4.5 | 4.3 | 1100 |
| Example 6 | | | blue | [6] | 4.6 | 4.4 | 1300 |
| Example 7 | | | blue | [7] | 4.6 | 4.4 | 1200 |
| Example 8 | | | blue | [8] | 4.4 | 4.5 | 1100 |
| Example 9 | | | blue | [9] | 4.2 | 4.8 | 1400 |
| Example 10 | | | blue | [10] | 4.2 | 4.9 | 1400 |
| Example 11 | | | blue | [11] | 4.2 | 4.7 | 1400 |
| Example 12 | | | blue | [12] | 4.2 | 4.8 | 1400 |
| Example 13 | | | blue | [13] | 4.6 | 4.3 | 2000 |
| Example 14 | | | blue | [14] | 4.5 | 4.5 | 2000 |
| Example 15 | | | blue | [15] | 4.7 | 4.4 | 2200 |
| Example 16 | | | blue | [16] | 4.8 | 4.3 | 2300 |
| Example 17 | H-1 | D-1 | blue | [17] | 4.8 | 4.4 | 2200 |
| Example 18 | | | blue | [18] | 4.7 | 4.3 | 2300 |
| Example 19 | | | blue | [19] | 4.8 | 4.3 | 2300 |
| Example 20 | | | blue | [20] | 4.9 | 4.3 | 2400 |
| Example 21 | | | blue | [21] | 4.7 | 4.4 | 2200 |
| Example 22 | | | blue | [22] | 4.7 | 4.4 | 2300 |
| Example 23 | | | blue | [23] | 4.6 | 4.3 | 2200 |
| Example 24 | | | blue | [24] | 4.6 | 4.3 | 2300 |
| Example 25 | | | blue | [25] | 4.6 | 4.3 | 2200 |
| Example 26 | | | blue | [26] | 4.8 | 4.2 | 2000 |
| Example 27 | | | blue | [27] | 4.7 | 4.2 | 2000 |
| Example 28 | | | blue | [28] | 4.7 | 4.2 | 2100 |
| Reference Example 29 | | | blue | [29] | 3.5 | 5.5 | 700 |
| Reference Example 30 | | | blue | [30] | 3.6 | 5.5 | 800 |
| Reference Example 31 | | | blue | [31] | 3.7 | 5.4 | 700 |
| Comparative Example 1 | H-1 | D-1 | blue | E-1 | 1.3 | 6.1 | 300 |
| Comparative Example 2 | | | blue | E-2 | 1.5 | 6.2 | 300 |
| Comparative Example 3 | | | blue | E-3 | 1.4 | 6.1 | 300 |

**Table 2**

| | Emissive Material | | | First Electron Transporting Layer | Second Electron Transporting Layer | | External Quantum Efficiency (%) | Driving Voltage (V) | Durability (h) |
|---|---|---|---|---|---|---|---|---|---|
| | Host Material | Dopant Material | Emission Color | Compound | Compound | Donor Compound | | | |
| Example 32 | H-1 | D-1 | blue | [1] | [1] | Li | 5.3 | 3.8 | 1500 |
| Example 33 | | | blue | [2] | [2] | Li | 5.1 | 3.8 | 1600 |
| Example 34 | | | blue | [3] | [3] | Li | 5.1 | 3.9 | 1800 |
| Example 35 | | | blue | [4] | [4] | Li | 5.3 | 3.6 | 1200 |
| Example 36 | | | blue | [5] | [5] | Li | 5.2 | 3.7 | 1300 |
| Example 37 | | | blue | [17] | [17] | Li | 5.5 | 3.6 | 2100 |
| Example 38 | | | blue | [18] | [18] | Li | 5.6 | 3.6 | 2300 |
| Example 39 | | | blue | [19] | [19] | Li | 5.7 | 3.5 | 2300 |
| Comparative Example 4 | H-1 | D-1 | blue | E-1 | E-1 | Li | 2.2 | 5.8 | 400 |
| Comparative Example 5 | | | blue | E-2 | E-2 | Li | 2.3 | 6.1 | 400 |
| Comparative Example 6 | | | blue | E-3 | E-3 | Li | 2.5 | 5.8 | 500 |

**Table 3**

| | Emissive Material | | | First Electron Transporting Layer | Second Electron Transporting Layer | | External Quantum Efficiency (%) | Driving Voltage (V) | Durability (h) |
|---|---|---|---|---|---|---|---|---|---|
| | Host Material | Dopant Material | Emission Color | Compound | Compound | Donor Compound | | | |
| Example 40 | H-1 | D-1 | blue | none | [1] | 2E-1 | 5.5 | 4.2 | 3500 |
| Example 41 | | | blue | none | [2] | 2E-1 | 5.6 | 4.2 | 3800 |
| Example 42 | | | blue | none | [3] | 2E-1 | 5.5 | 4.3 | 3800 |
| Example 43 | | | blue | none | [4] | 2E-1 | 5.9 | 4.0 | 3000 |
| Example 44 | | | blue | none | [5] | 2E-1 | 5.8 | 3.9 | 3200 |
| Comparative Example 7 | H-1 | D-1 | blue | none | E-1 | 2E-1 | 2.9 | 6.3 | 800 |
| Comparative Example 8 | | | blue | none | E-2 | 2E-1 | 2.9 | 6.2 | 900 |
| Comparative Example 9 | | | blue | none | E-3 | 2E-1 | 3.0 | 5.9 | 900 |

**Table 4**

| | Hole Injection Layer | First Hole Transporting Layer | Second Hole Transporting Layer | Emissive Layer | | Electron Transporting Layer | External Efficiency (%) | Driving Voltage (V) | Durability (h) |
|---|---|---|---|---|---|---|---|---|---|
| | | | | Host Material | Dopant Material | | | | |
| Example 45 | HAT-CN6 | HT-1 | none | H-2 | D-2 | [4] | 10.1 | 3.9 | 1500 |
| Example 46 | | | HT-2 | | | | 13.8 | 3.9 | 1900 |
| Example 47 | | | HT-3 | | | | 17.2 | 4.1 | 2700 |
| Example 48 | | | HT-4 | | | | 18.1 | 4.0 | 3000 |
| Comparative Example 10 | HAT-CN6 | HT-1 | none | H-2 | D-2 | E-1 | 5.9 | 7.6 | 400 |
| Comparative Example 11 | | | HT-2 | | | | 6.2 | 7.6 | 600 |
| Comparative Example 12 | | | HT-3 | | | | 6.6 | 7.5 | 800 |
| Comparative Example 13 | | | HT-4 | | | | 6.5 | 7.6 | 800 |

## Claims

1. A fluoranthene derivative represented by the following formula (3): 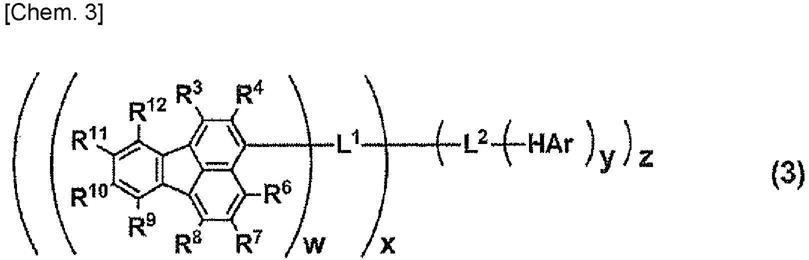 wherein R³, R⁴ and R⁶ to R¹² may be the same as or different from one another and each is selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, an amino group, a carbonyl group, a carboxyl group, an oxycarbonyl group and a carbamoyl group; L¹ is a substituted or unsubstituted phenylene group and wherein when L¹ is substituted, the substituent is an aryl group; L² is a single bond, a substituted or unsubstituted arylene group, or a substituted or unsubstituted heteroarylene group; HAr is a substituted or unsubstituted aromatic heterocyclic group containing an electron-accepting nitrogen selected from the group consisting of the following groups: 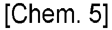 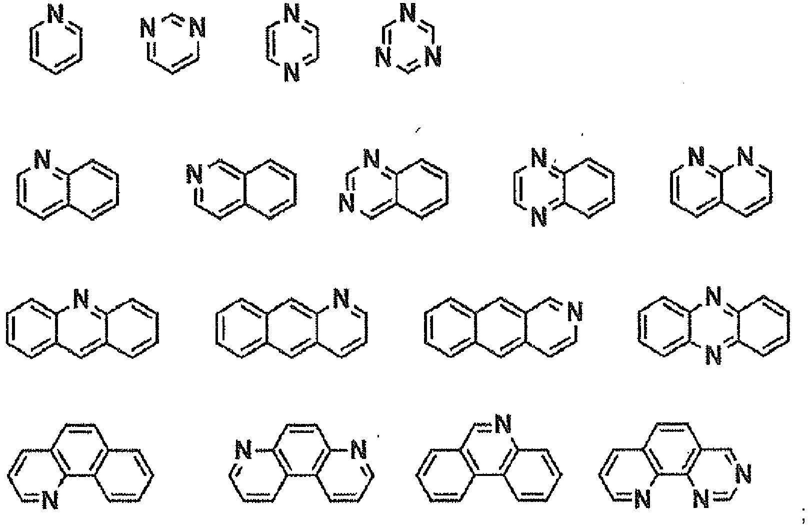 when each of the groups above is substituted, the substituent is selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, an amino group, a carbonyl group, a carboxyl group, an oxycarbonyl group, a carbamoyl group and -P(=O)R¹R² in which R¹ and R² are an aryl group or a heteroaryl group, and R¹ and R² may be fused to form a ring; w, x, y and z are an integer of 1 to 3; provided that the arylene group is neither an anthracene-containing group nor a pyrene-containing group and that each of L² and HAr is not a group containing electron-donating nitrogen; when w=x=1, L² is a substituted or unsubstituted arylene group or a substituted or unsubstituted heteroarylene group; when w=1 and x=2, HAr is not pyrimidine; when w=2 and x=1, the substituent substituting on L1 is not phenanthrene; and when w, x, y and z are 2 or 3, a plurality of fluoranthene groups, L¹, L² or HAr may be the same as or different from one another, and it is not allowed that both x and z are 2 or more.

2. The fluoranthene derivative according to claim 1, wherein R⁹ and R¹² are hydrogen.

3. The fluoranthene derivative according to claim 1, wherein R⁹ and R¹² are a substituted or unsubstituted aryl group.

4. The fluoranthene derivative according to any of claims 1 to 3, wherein HAr is a group containing a structure represented by any of the following formulae (4) to (7): 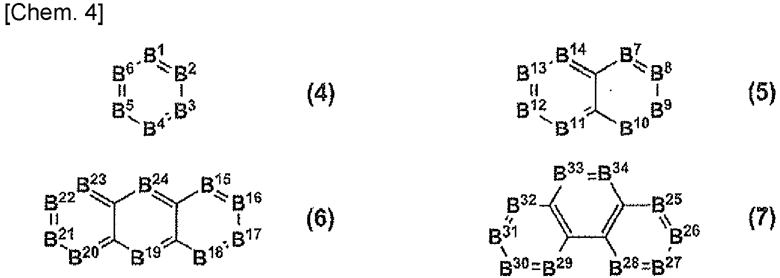 wherein B¹ to B³⁴ represent CH, a substituted carbon atom, or a nitrogen atom and the substituent when B¹ to B³⁴ are substituted is the same as those in formula (3).

5. The fluoranthene derivative according to any of claims 1 to 4, wherein w+x=3.

6. The fluoranthene derivative according to claim 5, wherein w=2 and x=1.

7. The fluoranthene derivative according to any of claims 1 to 4, wherein w=x=1.

8. The fluoranthene derivative according to any of claims 1 to 7, wherein y=z=1.

9. The fluoranthene derivative according to any of claims 1 to 7, wherein y+z=3.

10. An electronic device material containing the fluoranthene derivative according to any of claims 1 to 9.

11. A light emitting device material containing the fluoranthene derivative according to any of claims 1 to 9.

12. A photoelectric conversion element material containing the fluoranthene derivative according to any of claims 1 to 9.

13. An electronic device containing the fluoranthene derivative according to any of claims 1 to 9.

14. A light emitting device containing the fluoranthene derivative according to any of claims 1 to 9.

15. A light emitting device emitting light by electric energy, wherein an organic layer is present between an anode and a cathode, and the organic layer contains the fluoranthene derivative according to any of claims 1 to 9.

16. The light emitting device according to claim 15, wherein the organic layer has an electron transporting layer, and the electron transporting layer contains the fluoranthene derivative according to any of claims 1 to 9.

17. The light emitting device according to claim 15 or 16, wherein at least a charge generation layer is present in the organic layer, and the charge generation layer contains the fluoranthene derivative according to any of claims 1 to 9.

18. A photoelectric conversion element containing the fluoranthene derivative according to any of claims 1 to 9.

## Patentansprüche

1. Fluoranthenderivat, dargestellt durch die folgende Formel (3): 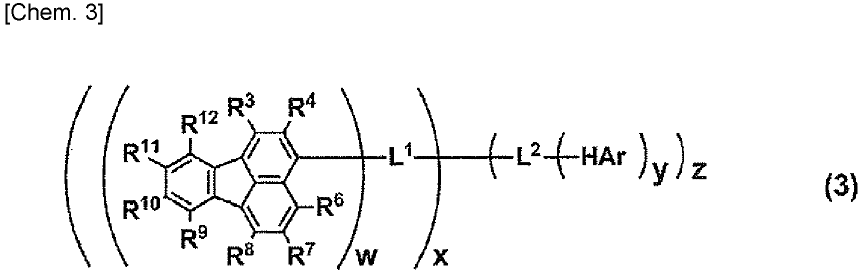 wobei R³, R⁴ und R⁶ bis R¹² gleich oder verschieden voneinander sein können und jedes ausgewählt ist aus der Gruppe bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Cyanogruppe, einer Aminogruppe, einer Carbonylgruppe, einer Carboxylgruppe, einer Oxycarbonylgruppe und einer Carbamoylgruppe; L¹ eine substituierte oder unsubstituierte Phenylengruppe ist, und wobei, wenn L¹ substituiert ist, der Substituent eine Arylgruppe ist; L² eine Einfachbindung, eine substituierte oder unsubstituierte Arylengruppe, oder eine substituierte oder unsubstituierte Heteroarylengruppe ist; HAr eine substituierte oder unsubstituierte aromatische heterocyclische Gruppe ist, die einen elektronenakzeptierenden Stickstoff enthält, ausgewählt aus der Gruppe bestehend aus den folgenden Gruppen: 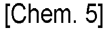 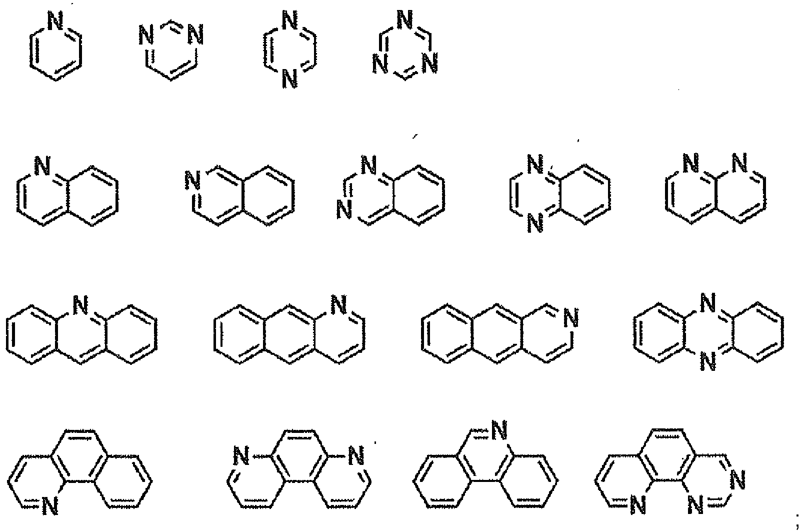 wenn jede der obigen Gruppen substituiert ist, der Substituent ausgewählt ist aus der Gruppe bestehend aus einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Cyanogruppe, einer Aminogruppe, einer Carbonylgruppe, einer Carboxylgruppe, einer Oxycarbonylgruppe, einer Carbamoylgruppe und -P(=O)R¹R², wobei R¹ und R² eine Arylgruppe oder eine Heteroarylgruppe sind, und R¹ und R² kondensiert sein können, um einen Ring zu bilden; w, x, y und z eine ganze Zahl von 1 bis 3 sind; vorausgesetzt, dass die Arylengruppe weder eine Anthracen enthaltende Gruppe noch eine Pyren enthaltende Gruppe ist und dass weder L² noch HAr eine Gruppe ist, die elektronenspendenden Stickstoff enthält; wenn w=x=1 ist, L² eine substituierte oder unsubstituierte Arylengruppe, oder eine substituierte oder unsubstituierte Heteroarylengruppe ist; wenn w=1 und x=2 ist, HAr nicht Pyrimidin ist; wenn w=2 und x=1 ist, der Substituent, der L¹ substituiert, nicht Phenanthren ist; und wenn w, x, y und z 2 oder 3 sind, mehrere Fluoranthengruppen L¹, L² oder HAr gleich oder verschieden voneinander sein können, und es nicht zulässig ist, dass sowohl x als auch z 2 oder mehr sind.

2. Fluoranthenderivat nach Anspruch 1, wobei R⁹ und R¹² Wasserstoff sind.

3. Fluoranthenderivat nach Anspruch 1, wobei R⁹ und R¹² eine substituierte oder unsubstituierte Arylgruppe sind.

4. Fluoranthenderivat nach einem der Ansprüche 1 bis 3, wobei HAr eine Gruppe ist, die eine Struktur enthält, die durch eine beliebige der folgenden Formeln (4) bis (7) dargestellt ist: 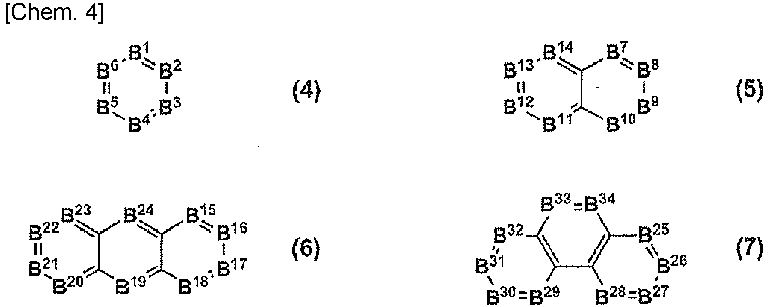 wobei B¹ bis B³⁴ CH, ein substituiertes Kohlenstoffatom oder ein Stickstoffatom darstellen, und der Substituent, wenn B¹ bis B³⁴ substituiert sind, der gleiche ist wie der in Formel (3).

5. Fluoranthenderivat nach einem der Ansprüche 1 bis 4, wobei w+x=3 ist.

6. Fluoranthenderivat nach Anspruch 5, wobei w=2 und x=1 ist.

7. Fluoranthenderivat nach einem der Ansprüche 1 bis 4, wobei w=x=1 ist.

8. Fluoranthenderivat nach einem der Ansprüche 1 bis 7, wobei y=z=1 ist.

9. Fluoranthenderivat nach einem der Ansprüche 1 bis 7, wobei y+z=3 ist.

10. Material für ein elektronisches Gerät, das das Fluoranthenderivat nach einem der Ansprüche 1 bis 9 enthält.

11. Material für eine Licht emittierende Vorrichtung, das das Fluoranthenderivat nach einem der Ansprüche 1 bis 9 enthält.

12. Material für ein photoelektrisches Umwandlungselement, das das Fluoranthenderivat nach einem der Ansprüche 1 bis 9 enthält.

13. Elektronische Vorrichtung, die das Fluoranthenderivat nach einem der Ansprüche 1 bis 9 enthält.

14. Licht emittierende Vorrichtung, die das Fluoranthenderivat nach einem der Ansprüche 1 bis 9 enthält.

15. Licht emittierende Vorrichtung, die Licht durch elektrische Energie emittiert, wobei eine organische Schicht zwischen einer Anode und einer Kathode vorhanden ist, und die organische Schicht das Fluoranthenderivat nach einem der Ansprüche 1 bis 9 enthält.

16. Licht emittierende Vorrichtung nach Anspruch 15, wobei die organische Schicht eine Elektronentransportschicht hat, und die Elektronentransportschicht das Fluoranthenderivat nach einem der Ansprüche 1 bis 9 enthält.

17. Licht emittierende Vorrichtung nach Anspruch 15 oder 16, wobei mindestens eine Ladungserzeugungsschicht in der organischen Schicht vorhanden ist, und die Ladungserzeugungsschicht das Fluoranthenderivat nach einem der Ansprüche 1 bis 9 enthält.

18. Photoelektrisches Umwandlungselement, das das Fluoranthenderivat nach einem der Ansprüche 1 bis 9 enthält.

## Revendications

1. Dérivé de fluoranthène représenté par la formule (3) suivante : 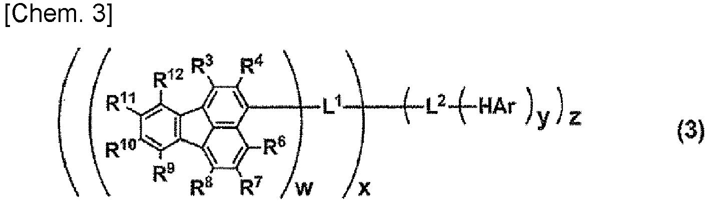 où R³, R⁴ et R⁶ à R¹² peuvent être identiques ou différents les uns des autres et chacun est choisi dans le groupe constitué d'un atome d'hydrogène, d'un groupe alkyle, d'un groupe cycloalkyle, d'un groupe hétérocyclique, d'un groupe alcényle, d'un groupe cycloalcényle, d'un groupe alcynyle, d'un groupe alcoxy, d'un groupe alkylthio, d'un groupe aryléther, d'un groupe arylthioéther, d'un groupe aryle, d'un groupe hétéroaryle, d'un halogène, d'un groupe cyano, d'un groupe amino, d'un groupe carbonyle, d'un groupe carboxyle, d'un groupe oxycarbonyle et d'un groupe carbamoyle ; L¹ représente un groupe phénylène substitué ou non substitué et où lorsque L¹ est substitué, le substituant est un groupe aryle ; L² représente une liaison simple, un groupe arylène substitué ou non substitué, ou un groupe hétéroarylène substitué ou non substitué ; HAr représente un groupe hétérocyclique aromatique substitué ou non substitué contenant un atome d'azote accepteur d'électrons choisi dans le groupe constitué des groupes suivants : 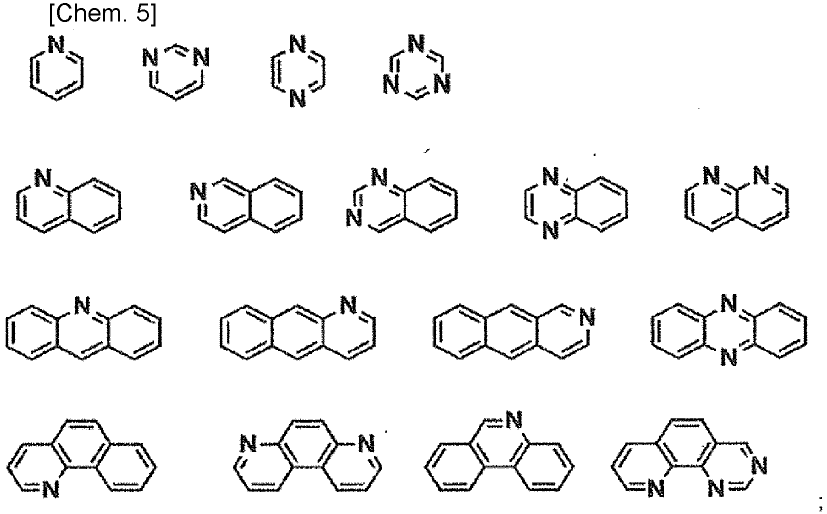 lorsque chacun des groupes ci-dessus est substitué, le substituant est choisi dans le groupe constitué d'un groupe alkyle, d'un groupe cycloalkyle, d'un groupe hétérocyclique, d'un groupe alcényle, d'un groupe cycloalcényle, d'un groupe alcynyle, d'un groupe alcoxy, d'un groupe alkylthio, d'un groupe aryléther, d'un groupe arylthioéther, d'un groupe aryle, d'un groupe hétéroaryle, d'un halogène, d'un groupe cyano, d'un groupe amino, d'un groupe carbonyle, d'un groupe carboxyle, d'un groupe oxycarbonyle, d'un groupe carbamoyle et de -P(=O)R¹R² où R¹ et R² représentent un groupe aryle ou un groupe hétéroaryle, et R¹ et R² peuvent être fusionnés pour former un cycle ; w, x, y et z sont des entiers compris entre 1 et 3 ; à condition que le groupe arylène n'est ni un groupe contenant de l'anthracène ni un groupe contenant du pyrène et que chacun de L² et de HAr n'est pas un groupe contenant un atome d'azote donneur d'électrons ; lorsque w = x = 1, L² représente un groupe arylène substitué ou non substitué ou un groupe hétéroarylène substitué ou non substitué ; lorsque w = 1 et x = 2, HAr n'est pas la pyrimidine ; lorsque w = 2 et x = 1, le substituant sur L¹ n'est pas le phénanthrène ; et lorsque w, x, y et z valent 2 ou 3, une pluralité de groupes fluoranthène, L¹, L² ou HAr peuvent être identiques ou différents les uns des autres, et il n'est pas permis que x et z soient tous deux égaux à 2 ou plus.

2. Dérivé de fluoranthène selon la revendication 1, dans lequel R⁹ et R¹² représentent un atome d'hydrogène.

3. Dérivé de fluoranthène selon la revendication 1, dans lequel R⁹ et R¹² représentent un groupe aryle substitué ou non substitué.

4. Dérivé de fluoranthène selon l'une des revendications 1 à 3, dans lequel HAr représente un groupe contenant une structure représentée par l'une des formules (4) à (7) suivantes : 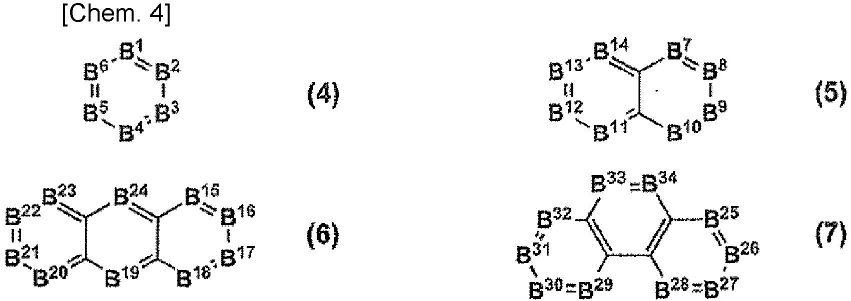 où B¹ à B³⁴ représentent CH, un atome de carbone substitué ou un atome d'azote, et le substituant lorsque B¹ à B³⁴ sont substitués est le même que ceux de la formule (3).

5. Dérivé de fluoranthène selon l'une des revendications 1 à 4, dans lequel w + x = 3.

6. Dérivé de fluoranthène selon la revendication 5, dans lequel w = 2 et x = 1.

7. Dérivé de fluoranthène selon l'une des revendications 1 à 4, dans lequel w = x = 1.

8. Dérivé de fluoranthène selon l'une des revendications 1 à 7, dans lequel y = z = 1.

9. Dérivé de fluoranthène selon l'une des revendications 1 à 7, dans lequel y + z = 3.

10. Matériau de dispositif électronique contenant le dérivé de fluoranthène selon l'une des revendications 1 à 9.

11. Matériau de dispositif émetteur de lumière contenant le dérivé de fluoranthène selon l'une des revendications 1 à 9.

12. Matériau d'élément de conversion photoélectrique contenant le dérivé de fluoranthène selon l'une des revendications 1 à 9.

13. Dispositif électronique contenant le dérivé de fluoranthène selon l'une des revendications 1 à 9.

14. Dispositif émetteur de lumière contenant le dérivé de fluoranthène selon l'une des revendications 1 à 9.

15. Dispositif émetteur de lumière émettant de la lumière par énergie électrique, dans lequel une couche organique est présente entre une anode et une cathode, et la couche organique contient le dérivé de fluoranthène selon l'une des revendications 1 à 9.

16. Dispositif émetteur de lumière selon la revendication 15, dans lequel la couche organique a une couche de transport d'électrons, et la couche de transport d'électrons contient le dérivé de fluoranthène selon l'une des revendications 1 à 9.

17. Dispositif émetteur de lumière selon la revendication 15 ou 16, dans lequel au moins une couche de génération de charge est présente dans la couche organique, et la couche de génération de charge contient le dérivé de fluoranthène selon l'une des revendications 1 à 9.

18. Élément de conversion photoélectrique contenant le dérivé de fluoranthène selon l'une des revendications 1 à 9.
